# EUROPEAN PATENT APPLICATION

(11) **EP 2 380 597 A1**
(43) Date of publication of application: **26.10.2011**
(21) Application number: 10004178.9
(22) Date of filing: 20.04.2010
(51) Int. Cl.: A61K 51/08, C07K 7/64

(54) **Cyclopeptide derivatives and uses thereof**

(71) Applicant: Technische Universität München, 80333 München (DE)
(72) Inventor: Demmer, Oliver, 81929 München (DE); Kessler, Horst, 85748 Garching (DE); Wester, Hans-Jürgen, 85304 Ilmmünster (DE); Schottelius, Margret, 80538 München (DE); Dijkgraaf, Ingrid, 47559 Kranenburg (DE); Buck, Andreas Konrad, 81247 München (DE)
(74) Representative: Wichmann, Hendrik

(57) **Abstract**

The invention relates to compounds having a structure according to formula (I), cyclo[(R¹)Xaa¹-B-(R²)Xaa²-(R³)Xaa³-(R⁴)Xaa⁴], or pharmaceutically acceptable salts thereof, wherein Xaa¹ and Xaa³ are independently of each other, a natural or unnatural amino acid, preferably with Xaa¹ and Xaa³ being, independently of each other, an amino acid comprising an aromatic moiety in its side chain, Xaa² is a natural or unnatural basic amino acid, Xaa⁴ is glycine or a D-amino acid, R¹, R², R³ and R⁴ are independently of each other H or methyl, B is an amino acid group having a structure according to formula (II), -N(-Q)-CHR⁵-C(=O)-, wherein R⁵ is selected from the group consisting of H, optionally substituted alkyl, optionally substituted aryl and optionally substituted heteroaryl, and a moiety L, and wherein Q is a group being sterically more demanding than a methyl group. The invention also relates to compositions, methods and uses related to said compounds.

## Description

The present invention is, among others, concerned with cyclopeptide derivatives, with processes for their preparation, pharmaceutical compositions comprising same and various embodiments relating to the application of said derivatives including imaging and medical applications. Specifically, the present invention relates to a compound having general the formula (I)

cyclo[(R¹)Xaa¹-B-(R²)Xaa²-(R³)Xaa³-(R⁴)Xaa⁴] (I).

The compounds of the invention are believed to be capable of binding to the seven transmembrane G-protein coupled chemokine receptor CXCR4 and are thus considered CXCR4 ligands. They may act as antagonists, agonists or inverse agonists.

The interaction between CXCR4 and its natural ligand α-chemokine stromal-derived factor (SDF-1α/ CXCL12) is a key factor in diverse bodily functions. Starting at the beginning of life with normal stem cell trafficking during embryogenesis it is also responsible for normal cardiovascular, hematopoietic and brain development as well as functions in the nervous and immune system.

The CXCR4 receptor has been found to be involved in a variety of diseases. For example, it mediates HIV-1 entry into T-cells as a co-receptor where it was first identified. Furthermore, in rheumatoid arthritis (RA) CXCR4 expressing CD4⁺ memory T cells accumulate in the inflamed synovium because of the high local CXCL12 concentration. Additionally CXCR4 is overexpressed on numerous different tumor cell types ranging from melanoma over prostate and pancreatic cancer to brain tumor cells.

Coronary heart disease has become a leading cause of death worldwide. The pathologic basis for coronary heart disease (CHD) is the growth of atherosclerotic plaques in the vascular wall over a period of many years resulting in bloss-flow-limiting stenosis or plaques disruption with acute thrombotic occlusion *(Ross, 1993; Libby, 2002; Hansson 2005).* Substantial evidence supports the concept that chronic inflammation of the vessel wall characterized by the influx of circulating immune cells is responsible for the development of atherosclerotic lesions *(Schober et al., 2008).*

In the pathogenesis of atherosclerosis, chronic inflammation of the arterial wall characterized by chemokine-mediated influx of leukocytes plays a central role. The cytokine macrophage migration inhibitory factor (MIF) is a unique pro-inflammatory regulator of many acute and chronic inflammatory diseases that contribute to lesion progression and plaque inflammation. These chemokine-like functions are mediated through interaction of MIF with the chemokine receptors CXCR2 and CXCR4, thus demonstrating the role of CXCR4 in native atherosclerosis, plaque destabilization and aneurysm formation.

Via binding of MIF, CXCR4 and other chemokine receptors, like CXCR2 play a role in atherosclerotic plaque development, vascular remodeling after injury, in atherosclerosis plaque destabilization and aneurysm formation *(Schober et al., 2008).*

Like chemokines, the interaction of MIF with the chemokine receptors CXCR2 and CXR4 as a noncanonical ligand induces recruitment of monocytes and T cells to atherosclerotic lesions, Furthermore, MIF regulates smooth muscle cell migration and proliferation, which may promote lesion growth. Increased foam-cell transformation of lesional macrophages and enhanced degradation of extracellular matrix proteins by MIF contribute to the progression into an unstable plaque phenotype. These data were largely confirmed in a study using human specimens from patients undergoing heart transplantation, carotis endarterectomy, or from autopsied individuals *(Burger-Kentischer et al, 2002).*

Accordingly, due to their potential use for medicinal applications, a variety of peptidic and non-peptidic CXCR4 antagonists have been developed. One example is the bicyclam AMD3100 (plerixafor) 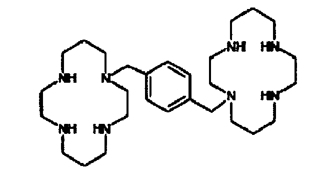 that has been approved by the FDA for the treatment of the two blood cancer types non-Hodgkin's lymphoma and multiple myeloma. Based on the structure of AMD3100 further CXCR4 antagonists such as peptidic CXCR4 antagonists have been developed. Examples include T140 and its derivatives which are side-chain cyclized peptides that contain one or two cyclization sites. Further downsizing of T140 gave the head-to-tail cyclized pentapeptide FC 131 with good antagonistic activity *(Fujii et al., 2003).* A potential advantage of CXCR4 antagonists derived from T140, like FC131, may be their suggested mechanism of action as inverse agonists in contrast to agents like AMD3100 which are partial agonists. Furthermore, a number of modifications of FC131 have been described (WO 2007/096662). Besides, certain multimeric compounds of such cyclized pentapeptides have been described (WO 2009/027706).

So far, therapeutic potential of CXCR4 ligands, such as antagonists, has been shown e.g. for the treatment of HIV infection, cancer, and rheumatoid arthritis. Other antiinflammatory uses of CXCR4 ligands have been described for asthma and multiple sclerosis. Furthermore, CXCR4 ligands can mobilize stem cells, e.g. for stem cell transplantations. Moreover, attenuation of pain has been observed (in rodents) by a specific CXCR4 ligand. In addition CXCR4 ligands are also discussed for the treatment of neurological diseases.

CXCR4 ligands comprising additional moieties may be particularly suitable in the treatment of the above diseases. Examples for the latter ligands include but are not limited to those comprising cytotoxic, (oligo)nucleotide, radioactive, and (radio)metal-chelate moieties or combinations thereof. The general concepts pursued with such moieties are known from certain other peptidic and peptidomimetic ligands.

In addition to the therapeutic potential of CXCR4 ligands, their affinity towards the receptor may be used for other applications. These include but are not limited to the imaging of CXCR4 receptors, e.g. for the diagnosis of related diseases or the visualization of CXCR4 and CXCR4 containing tissue, as well as affinity purification of CXCR4 receptors. In most of these cases, the CXCR4 ligands are modified with additional (functional) moieties and/or moieties that immobilize the CXCR4 ligands.

However, the attachment of such additional moieties to the CXCR4 ligands may result in that the ligands substantially loose their affinity to the CXCR4 receptor. Therefore, there is a need in the art to develop new CXCR4 ligands, particularly ligands having high affinity to CXCR4, more particularly such ligands that allow the introduction of additional (functional) moieties while retaining sufficient affinity to the CXCR4 receptor.

The present invention provides such new CXCR4 ligands and their uses in medicinal and scientific applications as well as such ligands comprising additional (functional) moieties and their uses in medicinal and scientific applications.

The compounds of the invention are considered capable of binding to CXCR4 and, hence, are considered CXCR4 ligands. They may be capable of functioning as CXCR4 antagonists, agonists or inverse agonists. Generally, they are based on the peptide backbone structure of FC131 and its derivatives. However, as opposed to the compounds known from the prior art, the compounds of the invention are head-to-tail cyclized (backbone cyclization from the N to the C terminus) pentapeptides wherein at least one amide proton has been substituted by a group that is sterically more demanding than a methyl group.

In preferred cases, the binding affinity of the compounds of the invention is 10 to 100 times better than that of FC 131.

### Detailed description of the invention

The present invention provides compounds, compositions, uses and methods as defined and described in the claims and hereinbelow.

In one aspect, the present invention provides a compound, or a pharmaceutically acceptable salt thereof, having a structure according to formula I

cyclo [(R¹)Xaa¹- B-(R²)Xaa² -(R ³)Xaa³ -(R ⁴)Xaa ⁴] (I)

wherein Xaa¹ and Xaa³ are independently of each other a natural or unnatural amino acid, preferably with Xaa¹ and Xaa³ being, independently of each other, an amino acid comprising an aromatic moiety in its side chain,
Xaa² is a natural or unnatural basic amino acid,
Xaa⁴ is glycine or a D-amino acid,
R¹, R², R³ and R⁴ are independently of each other H or alkyl,
B is an amino acid group having a structure according to formula II 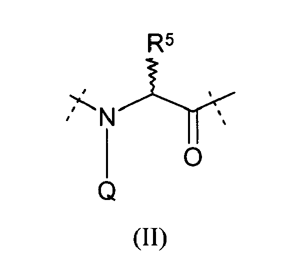 wherein R⁵ is selected from the group consisting of H, optionally substituted alkyl, optionally substituted aryl, optionally substituted heteroaryl and a moiety L,
wherein L is a linker moiety optionally being or optionally being substituted with a compound D particularly selected from detectable labels, organic complexation agents, active substances and combinations thereof, particularly wherein the active substance is selected from the group consisting of cytotoxic agents, lipids, sugars, sugar conjugates, sugar derivatives, proteins and combinations thereof,
and wherein Q is a group being sterically more demanding then a methyl group.

In the compounds of the invention having a structure according to formula I, cyclo[(R¹)Xaa¹-B-(R²)Xaa²-(R³)Xaa³-(R⁴)Xaa⁴], the carbonyl carbon atom of Xaa¹ is linked to the N^{alpha} atom of B, the carbonyl carbon atom of B is linked to the N^{alpha} atom of Xaa², the carbonyl carbon atom of Xaa² is linked to the N^{alpha} atom of Xaa³, the carbonyl carbon atom of Xaa³ is linked to the N^{alpha} atom of Xaa⁴, and the carbonyl carbon atom of Xaa⁴ is linked to the N^{alpha} atom of Xaa¹. The term "a group being sterically more demanding than a methyl group" particularly refers to any conceivable group containing atoms that, in sum, are heavier than the atoms contained in a methyl group. Preferably, a group being sterically more demanding than a methyl group is any conceivable group containing at least two atoms that are at least as heavy as a carbon atom.

According to a preferred embodiment, Q is a linker moiety comprising the group -X-R⁶ with X being selected from the group consisting of =N-, -N=, -Y-, -C(=Y)-NH-, -NH-C(=Y)-, -NH-C(=Y)-Y'-, -Y'-C(=Y)-NH-, -Y'-C(=Y)-, -C(=Y)-Y'-, -C(=Y)-, alkynylene, azide, and 1,2,3-triazole, with Y and Y' being independently of each other selected from the group consisting of NH, O and S, and with R⁶ being selected from the group consisting of H, optionally substituted alkyl, optionally substituted aryl, optionally substituted alkoxy group, optionally substituted heteroaryl, a compound D, and wherein in case R⁶ is substituted, the substituent preferably comprises a compound D. The compound D is particularly selected from detectable labels, organic complexation agents, active substances and combinations thereof, particularly wherein the active substance is selected from the group consisting of cytotoxic agents, lipids, sugars, sugar conjugates, sugar derivatives, proteins and combinations thereof.

R⁵, L, X, R⁶ and/or Xaa⁴, preferably R⁵ or L, are/is optionally attached to at least one further compound of formula (I). Accordingly, the invention also relates to multimers, particularly dimers, of compounds of formula (I). Furthermore, the present invention relates to methods for preparing the above mentioned compound. The invention also relates to compositions, methods and uses related to said compounds.

### The structural unit -(R)Xaa-:

As far as the structural units -(R¹)Xaa¹-, -(R²)Xaa²-, -(R³)Xaa³- and -(R⁴)Xaa⁴- are concerned, in the context of the present invention, the general structure -(R)Xaa- is denoted to mean a derivative of an amino acid Xaa, being substituted at the alpha nitrogen (N^{alpha}) of said amino acid with a residue R. Thus, the structural unit -(R¹)Xaa¹- represents an amino acid being substituted in N^{alpha} -position with a residue R¹. Likewise, -(R²)Xaa²- represents an amino acid being substituted in N^{alpha}-position with a residue R², -(R³)Xaa³- represents an amino acid being substituted in N^{alpha}-position with a residue R³, and -(R⁴)Xaa⁴- represents an amino acid being substituted in N^{alpha}-position with a residue R⁴.

R¹, R², R³ and R⁴ are, independently of each other, selected from H and alkyl.

The term "alkyl" as used in the context of the present invention preferably refers to a linear or branched, optionally suitably substituted, saturated aliphatic chain of preferably 1 to 12, more preferably 1 to 8, and more preferably 1 to 6 carbon atoms and includes, but is not limited to, optionally suitably substituted methyl, ethyl, propyl, isopropyl, n-butyl, sec-butyl, tert-butyl, pentyl, isopentyl, and hexyl.

The term "substituted alkyl" as used in the context of the present invention preferably refers to alkyl groups being substituted in any position by one or more substituents, preferably by 1, 2, 3, 4, 5 or 6 substituents, more preferably by 1, 2, or 3 substituents. If two or more substituents are present, each substituent may be the same or may be different from the at least one other substituent. Suitable substituents are known to the skilled person. A suitable substituent may be, for example, a halogen atom, a hydroxy, an amino group or an alkoxy group, which may be substituted.

Said term "alkoxy" preferably represents a linear or branched alkyl group, preferably having from 1 to 6 carbon atoms attached to an oxygen atom. Typical alkoxy groups include methoxy, ethoxy, propoxy, isopropoxy, butoxy, t-butoxy, pentoxy and the like, as well as polyalkoxy and the like. The term "polyalkoxy" refers to a structure unit -[(CR⁷R⁸)_{q}-O-(CR⁹R¹⁰)ᵣ]ₛ-, preferably -[(CH₂)_{q}-O-(CH₂)ᵣ]ₛ- wherein q is equal to or different from r and wherein q and r are, independently of each other from 0 to 10, with the proviso that when one of q and r is 0, the other one is not 0, preferably wherein q+r = 2; and wherein s is from 1 to 10, preferably from 1 to 5, more preferably from 1 to 2; and wherein R⁷, R⁸, R⁹ and R¹⁰ are, independently of each other, selected from H and alkyl, preferably wherein R⁷, R⁸, R⁹ and R¹⁰ are H.

The term "substituted alkoxy" preferably refers to alkoxy groups being substituted in any position by one or more substituents, preferably by 1, 2, 3, 4, 5 or 6 substituents, more preferably by 1, 2, or 3 substituents. If two or more substituents are present, each substituent may be the same or may be different from the at least one other substituent. Suitable substituents are known to the skilled person.

The term "halogen atom" as used in the context of the present invention preferably refers to a chlorine, iodine, bromine or fluorine atom. Preferred halogen atoms are fluorine and/or chlorine atoms.

Conceivable halogen substituted alkyl groups are, for example, fluoromethyl, difluoromethyl, trifluoromethyl, chloromethyl, dichloromethyl, difluoroethyl, dichloroethyl, trichloroethyl, pentafluoroethyl, difluorochloromethyl, chloroethyl, difluoropropyl, dichlorofluoroethyl, heptafluoropropyl, fluorochloroethyl, dichlorofluoromethyl.

In case any of R¹, R², R³ and R⁴ is an alkyl group, said alkyl group is preferably methyl or ethyl, in particular methyl.

Thus, according to an especially preferred embodiment of the present invention R¹, R², R³ and R⁴ are, independently of each other, selected from H and methyl.

In case the residue being substituted to the N^{alpha}-position of the amino acid Xaa is H, the structural unit can be defined as being -(H)Xaa-. Likewise, in the context of the present invention, the structural unit can equally be defined as being -Xaa-, in case the residue R is H. In other words, the group (R) can be omitted in case R is H, Thus, in the context of the present invention, the term -(H)Xaa- and the term -Xaa- are equivalent.

According to a preferred embodiment, the present invention relates to a compound having for example the structure cyclo[-Xaa¹-B-Xaa²-Xaa³-Xaa⁴-] or the structure cyclo[-(Me)Xaa¹-B-Xaa²-Xaa³-Xaa⁴-] or the structure cyclo[-Xaa¹-B-(Me)Xaa²-Xaa³-Xaa⁴-], or the structure cyclo[-Xaa¹-B-Xaa²-(Me)Xaa³-Xaa⁴-], or the structure cyclo[-Xaa¹-B-Xaa²-Xaa³-(Me)Xaa⁴-], or the structure cyclo[-(Me)Xaa¹-B-(Me)Xaa²-Xaa³-Xaa⁴-], or the structure cyclo[-(Me)Xaa¹-B-Xaa²-(Me)Xaa³-Xaa⁴-], or the structure cyclo[-(Me)Xaa¹-B-Xaa²-Xaa³-(Me)Xaa⁴-], or the structure cyclo[-Xaa¹-B-(Me)Xaa²-(Me)Xaa³-Xaa⁴-], or the structure cyclo[-Xaa¹-B-(Me)Xaa²-Xaa³-(Me)Xaa⁴-], or the structure cyclo[-Xaa¹-B-Xaa²-(Me)Xaa³-(Me)Xaa⁴-], or the structure cyclo[-(Me)Xaa¹-B-(Me)Xaa²-(Me)Xaa³-Xaa⁴-], or the structure cyclo[-Xaa¹-B-(Me)Xaa²-(Me)Xaa³-(Me)Xaa⁴-], or the structure cyclo[-(Me)Xaa¹-B-Xaa²-(Me)Xaa³-(Me)Xaa⁴-], or the structure cyclo[-(Me)Xaa¹-B-(Me)Xaa²-Xaa³-(Me)Xaa⁴-], or the structure cyclo[-(Me)Xaa¹-B-(Me)Xaa²-(Me)Xaa³-(Me)Xaa⁴-]; with (Me) being a methyl group.

Most preferably at least one, more preferably at least two, more preferably at least three and most preferably all of R¹, R², R³ and R⁴ are H.

According to a preferred embodiment, the present invention relates to a compound having a structure selected from the group consisting of cyclo[-(Me)Xaa¹-B-(Me)Xaa²-(Me)Xaa³-Xaa⁴-], cyclo[-Xaa¹-B-(Me)Xaa ²-(Me)Xaa³-(Me)Xaa⁴-], cyclo[-(Me)Xaa¹-B-Xaa² - (Me)Xaa³-(Me)Xaa⁴-], cyclo[-(Me)Xaa¹-B-(Me)Xaa²-Xaa³-(Me)Xaa⁴-], cyclo[-(Me)Xaa¹-B-Xaa²-Xaa³-Xaa⁴-], cyclo[-Xaa¹-B-(Me)Xaa²-Xaa³ -Xaa⁴-], cyclo[-Xaa¹-B-Xaa²-(Me)Xaa³-Xaa⁴-], cyclo[-Xaa¹-B-Xaa²-Xaa³-(Me)Xaa⁴-], cyclo[-(Me)Xaa¹-B-(Me)Xaa²- Xaa³-Xaa⁴-], cyclo[-(Me)Xaa¹-B-Xaa²-(Me)Xaa³-Xaa⁴-], cyclo[-(Me)Xaa¹-B-Xaa²-Xaa ³⁻(Me)Xaa⁴-], cyclo[-Xaa¹-B-(Me)Xaa²-(Me)Xaa³-Xaa⁴-], cyclo[-Xaa¹-B-(Me)Xaa²-Xaa³-(Me)Xaa⁴-], cyclo[-Xaa¹-B-Xaa²-(Me)Xaa³-(Me)Xaa⁴-], and cyclo[-Xaa¹-B-Xaa²-Xaa³-Xaa⁴-], more preferably having a structure selected from the group consisting of cyclo[-(Me)Xaa¹-B-Xaa²-Xaa³-Xaa⁴-], cyclo[-Xaa¹-B-(Me)Xaa²-Xaa³-Xaa⁴-], cyclo[-Xaa¹-B-Xaa²-(Me)Xaa ³-Xaa ⁴-], cyclo[-Xaa¹-B-Xaa²-Xaa³-(Me)Xaa⁴-], cyclo[-(Me)Xaa¹-B-(Me)Xaa²-Xaa³-Xaa⁴-], cyclo[-(Me)Xaa¹-B-Xaa²-(Me)Xaa³-Xaa⁴-], cyclo[-(Me)Xaa¹-B-Xaa²-Xaa³-(Me)Xaa⁴-], cyclo[-Xaa¹-B-(Me)Xaa²-(Me)Xaa³-Xaa⁴-], cyclo[-Xaa¹-B-(Me)Xaa²-Xaa³-(Me)Xaa⁴-], cyclo[-Xaa¹-B-Xaa²-(Me)Xaa³-(Me)Xaa⁴-], and cyclo[-Xaa¹-B-Xaa²-Xaa³-Xaa⁴-], more preferably having a structure selected from the group consisting of cyclo[-(Me)Xaa¹-B-Xaa²-Xaa³-Xaa⁴-], cyclo[-Xaa¹-B-(Me)Xaa²-Xaa³-Xaa⁴-], cyclo[-Xaa¹-B-Xaa²-(Me)Xaa³-Xaa⁴-], cyclo[-Xaa¹-B-Xaa²-Xaa ³-(Me)Xaa⁴-], and cyclo[-Xaa¹-B-Xaa²-Xaa³-Xaa⁴-], and most preferably having the structure cyclo[-Xaa¹-B-Xaa²-Xaa³-Xaa⁴-].

As regards the general amino acid abbreviations Xaa¹, Xaa², Xaa³, Xaa⁴, said abbreviations encompass the L-enantiomer as well as the respective D-enantiomers.

### The amino acid Xaa¹:

Xaa¹ is a natural or unnatural amino acid. In the context of the present invention the term "natural or unnatural amino acid" refers to residues of any naturally occurring or synthetic amino acid and their respective D and L stereoisomers if their structures allow such stereoisomeric forms.

The term "natural amino acid" refers to naturally occurring amino acids which typically occur in proteins including their stereoisomeric forms. Natural amino acids include alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan (Trp), tyrosine (Tyr) and valine (Val).

The term unnatural amino acid includes any conceivable amino acid. This term includes amino acids bearing a side chains comprising acidic, basic, neutral and/or aromatic moieties. Conceivable amino acids to be mentioned are, for example, azetidinecarboxylic acid, 2-aminoadipic acid, 3-aminoadipic acid, beta-alanine, aminopropionic acid, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, 2,4-diaminoisobutyric acid, desmosine, 2,2'-diaminopimelic acid, 2,3-diaminopropionic acid, N-ethylglycine, N-ethylasparagine, hydroxylysine, allo-hydroxylysine, 3-hydroxyproline, 4-hydroxyproline, isodesmosine, allo-isoleucine, N-methylglycine, N-methylisoleucine, N-methylvaline, norvaline, norleucine, ornithine, naphthylalanine, diaminopropionic acid, N-(fluoropropionyl)-diaminobutyric acid, N-fluorobenzoyl-diaminobutyric acid, N-fluorobenzoyldiaminopropionic acid, citrulline and pipecolic acid. Any unnatural amino acid referred to herein, particularly in context with Xaa⁴, may bear or may not bear a side chain that comprises at least one, preferably one or two, further compound(s) having a structure according to formula (I).

In the context of Xaa¹, the term residues refers to building blocks being incorporated in the cyclic pentapeptide having the structure: 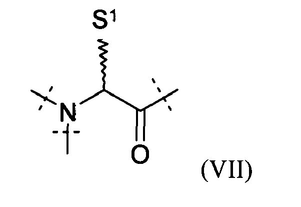 wherein S¹ is the side chain of the natural or unnatural amino acid. For example in case the amino acid is glycine S¹ is H. S¹ may form a cyclic ring with the group N, in particular in case Xaa¹ is proline or a proline derivative.

Preferably Xaa¹ is a naturally or unnaturally amino acid comprising an aromatic moiety in its side chain S¹. The term "amino acid comprising an aromatic moiety in its side chain" refers to an amino acid, the side chain of which comprises an aromatic group. Side chains comprising an aromatic moiety include side chains with the aromatic moiety being directly attached to the C^{alpha} of the amino acid, thus with S¹ being the aromatic moiety, as well as side chains being substituted in any position with at least one aromatic moiety or group, such as for example alkyl chains being substituted with an aromatic moiety.

The term aromatic moiety as used in this context of the invention, refers to an optionally substituted aryl group and/or heteroaryl group, wherein the term "aryl", in turn, refers to, but is not limited to, optionally suitably substituted 5- and 6-membered single-ring aromatic groups as well as optionally suitably substituted multicyclic groups, for example bicyclic or tricyclic aryl groups. The term "aryl" thus includes, for example, optionally substituted phenyl groups or optionally suitably substituted naphthyl groups. Aryl groups can also be fused or bridged with alicyclic or heterocycloalkyl rings which are not aromatic so as to form a polycycle, e.g., benzodioxolyl or tetraline. The term heteroaryl includes optionally suitably substituted 5- and 6-membered single-ring aromatic groups as well as substituted or unsubstituted multicyclic aryl groups, for example tricyclic or bicyclic aryl groups, comprising one or more, preferably from 1 to 4 such as 1, 2, 3 or 4, heteroatoms, wherein in case the aryl residue comprises more than 1 heteroatom, the heteroatoms may be the same or different. Such heteroaryl groups including from 1 to 4 heteroatoms are, for example, benzodioxolyl, pyrrolyl, furanyl, thiophenyl, thiazolyl, isothiaozolyl, imidazolyl, triazolyl, tetrazolyl, pyrazolyl, oxazolyl, isoxazolyl, pyridinyl, pyrazinyl, pyridazinyl, benzoxazolyl, benzodioxazolyl, benzothiazolyl, benzoimidazolyl, benzothiophenyl, methylenedioxyphenylyl, naphthridinyl, quinolinyl, isoquinolinyl, indolyl, benzofuranyl, purinyl, benzofuranyl, deazapurinyl, or indolizinyl.

The term "substituted aryl" and the term "substituted heteroaryl" as used in the context of the present invention describes moieties having substituents replacing a hydrogen on one or more atoms, e.g. C or N, of an aryl or heteroaryl moiety. There are in general no limitations as to the substituent. The substituents may be, for example, selected from the group consisting of alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxy, phosphate, phosphonato, phosphinato, amino, acylamino, including alkylcarbonylamino, arylcarbonylamino, carbamoyl and ureido, amidino, nitro, imino, sulfhydryl, alkylthio, arylthio, thiocarboxylate, sulfates, alkylsulfinyl, sulfonate, sulfamoyl, sulfonamido, trifluoromethyl, cyano, azido, heterocyclyl, alkylaryl, morpholino, piperizinyl, cyclopentanyl, cyclohexanyl, and piperidinyl.

In case S¹ comprises an aryl group or heteroaryl being substituted at at least one position, the at least one substituent is preferably selected from the group consisting of alkyl, halogen, amino and hydroxyl.

In case S¹ is an aryl group or a heteroaryl being substituted with an amino group, the term "amino" is meant to encompass, for example, groups such as -NH₂, alkylamino, dialkylamino, arylamino, diarylamino, and alkylarylamino groups. In case S¹ comprises an aryl group or a heteroaryl group being substituted at at least one position with an amino group, the amino group is preferably selected from the group consisting of methylamino, dimethylamino, ethylamino, diethylamino, benzylamino, and dibenzylamino.

In this context of the present invention, the term "halogen" or "halogen atom" preferably refers to a chlorine, iodine, bromine or fluorine atom and the respective radioactive and non-radioactive isotopes. Thus, the term halogen is for example selected from the group consisting of Cl, Br, I, ¹²⁷I, ¹²³I, ¹²⁰I, ¹²⁴I, ¹²⁵I, ¹³¹I, ⁷⁵Br , ⁷⁶Br , ⁷⁷Br, ¹⁸F and ¹⁹F and ³⁴Cl. Preferred halogen atoms are fluorine and/or iodine atoms. The term halogen also includes the respective isotopes of these atoms. Thus, in case S¹ comprises an aryl group or heteroaryl being substituted at at least one position with an iodine, the iodine may be selected from the group consisting of ¹²⁷I, ¹²³I, ¹²⁰I, ¹²⁴I, ¹²⁵I, and ¹³¹I. In case S¹ comprises an aryl group or heteroaryl being substituted at at least one position with an fluorine atom, the fluorine atom may be selected from the group consisting of ¹⁸F and ¹⁹F.

Accordingly, Xaa¹ may or may not comprise a compound D as defined herein.

According to one preferred embodiment of the invention, Xaa¹ is a tyrosine residue being substituted with an iodine or a respective radioactive or non radioactive isotope of iodine.

The term "alkyl chain" as used in this context of the invention preferably refers to alkyl chains of 1 to 20 carbon atoms, preferably of 1 to 10 carbon atoms, more preferably of 1 to 8 carbon atoms, most preferably of 1 to 6 carbon atoms, such as for example, 1, 2, 3, 4, 5 or 6 carbon atoms. The alkyl chains may be branched or unbranched, substituted or unsubstituted, and the alkyl chains may be interrupted by one or more heteroatoms, cyclic groups and/or heterocyclic groups.

The amino acid Xaa¹ may have multiple asymmetric centers. As a consequence, the resulting cyclopeptides may occur as racemates, mixtures of enantiomers and as individual enantiomers, as well as diastereomers and mixtures of diastereomers. All asymmetric forms, possible stereoisomers, single isomers and mixtures of isomers are included within the scope of the present invention. The designation " 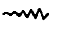 " shown in formula (VII) above refers to a bond to which the stereochemistry is not specifically designated.

Preferably, Xaa¹ is present in enantiomerically pure form.

The term "enantiomerically pure" as used in the context of the present invention refers to compounds having an enantiomeric excess of at least 95 % (i.e. minimum 97,5 % of one enantiomer and maximum 2,5% of the other enantiomer) up to an enantiomeric excess of 100% (i.e. 100% of one enantiomer and none of the other), in particular compounds having an enantiomeric excess of at least 98 %, more in particular having an enantiomeric excess of at least 99% and most in particular having an enantiomeric excess of at least 99.9 %, especially of 100 %.

According to a preferred embodiment of the present invention, S¹ comprises an aromatic group being selected from, optionally substituted, phenyl, naphthyl and indole, most preferably, S¹ is selected from the group consisting of, optionally substituted, phenyl, and, optionally further substituted, hydroxy-phenyl, an optionally further substituted group having the following structure 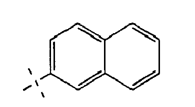 or an optionally substituted group having the following structure

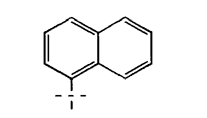

Thus, in other words, Xaa¹ is preferably selected from the group consisting of phenylalanine, tyrosine, tryptophan, phenylglycine, and naphthylalanine, i.e. Xaa¹ is most preferably selected from the group consisting of L-phenylalanine (Phe), D-phenylalanine (D-Phe), L-tyrosine (Tyr), D-tyrosine (D-Tyr), L-tryptophan (Trp), D-tryptophan (D-Trp), D-phenylglycine (D-Phg), L-phenylglycine (Phg), L-naphthylalanine (Nal) and D-naphthylalanine (D-Nal).

In case Xaa¹ is a "L-naphthylalanine", said building block preferably has a structure selected from the structures

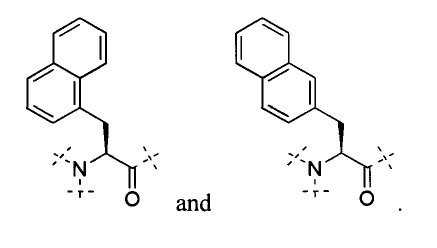

Thus, the present invention preferably refers to a compound, as described above, having a structure being selected from the group consisting of cyclo[-Phe-B-Xaa²-Xaa³-Xaa⁴], cyclo[-D-Phe-B-Xaa²-Xaa³-Xaa⁴], cyclo[-Tyr-B-Xaa²-Xaa³-Xaa⁴], cyclo[-D-Tyr-B-Xaa²-Xaa³-Xaa⁴], cyclo[-Trp-B-Xaa²-Xaa³-Xaa⁴], cyclo[-D-Trp-B-Xaa²-Xaa³-Xaa⁴], cyclo[-Phg-B-Xaa²-Xaa³-Xaa⁴], cyclo[-D-Phg-B-Xaa²-Xaa³-Xaa⁴], cyclo[-Nal-B-Xaa²-Xaa³-Xaa⁴] and cyclo[-D-Nal-B-Xaa²-Xaa³-Xaa⁴].

According to a particular preferred embodiment, Xaa¹ is D-Tyrosine. Thus, the present invention preferably relates to a compound having the structure cyclo[-D-Tyr-B-Xaa²-Xaa³-Xaa⁴].

### The amino acid Xaa²:

Xaa² is a natural or unnatural basic amino acid. In the context of the present invention the term "natural or unnatural basic amino acid" refers to residues of any naturally occurring or synthetic amino acid comprising a basic group in its side chain and their respective D and L stereoisomers if their structures allow such stereoisomeric forms.

The term "natural basic amino acid" refers to naturally occurring amino acids or residues which typically occur in proteins including their stereoisomeric forms. Natural basic amino acids include arginine (Arg), histidine (His) and lysine (Lys).

The term unnatural basic amino acid includes any conceivable basic amino acid, thus this term includes amino acids comprising at least one basic moiety in its side chain. Basic group and basic moiety are used interchangeably herein. Basic preferably means a group, which has a net positive charge at pH 6 or lower in aqueous solvents.

In this context of the invention, the term residues refers to building blocks being incorporated in the cyclic pentapeptide having the structure: 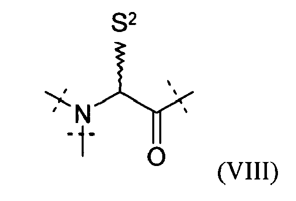 wherein S² is the side chain of the natural or unnatural basic amino acid. S² may form a cyclic ring with the group N, in particular in case Xaa² is a proline derivative.

Preferably S² comprises at least one basic group, preferably one basic group selected from the group consisting of amino groups, guanidine groups or guanidine mimics. According to one preferred embodiment, S² comprises a guanidine group.

According to a preferred embodiment of the invention S² is an alkyl chain being substituted with the at least one basic group, thus, preferably with one basic group selected from the group consisting of amino groups, guanidine groups guanidine mimics, most preferably with a guanidine group.

The amino acid Xaa² may have multiple asymmetric centers. As a consequence, the resulting cyclopeptides may occur as racemates, mixtures of enantiomers and as individual enantiomers, as well as diastereomers and mixtures of diastereomers. All asymmetric forms, possible stereoisomers, single isomers and mixtures of isomers are included within the scope of the present invention. The designation " 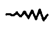 " shown in formula (VIII) above refers to a bond to which the stereochemistry is not specifically designated.

Especially preferred amino acids to be mentioned for Xaa² are, for example, ornithine (Orn or D-Orn), diaminopropionic acid (Dap or D-Dap), arginine, lysine or homolysine (homoLys or D-homoLys).

Thus, the present invention preferably refers to a compound having a formula selected from the group consisting of cyclo[-Xaa¹-B-Om-Xaa³-Xaa⁴-], cyclo[-Xaa¹-B-D-Orn-Xaa³-Xaa⁴-], cyclo[-Xaa¹-B-Dap-Xaa³-Xaa⁴-], cyclo[-Xaa¹-B-D-Dap-Xaa³-Xaa⁴-], cyclo[-Xaa¹-B-Lys-Xaa³-Xaa⁴-], cyclo[-Xaa¹-B-D-Lys-Xaa³-Xaa⁴-], cyclo[-Xaa¹-B-homoLys-Xaa³-Xaa⁴-], cyclo[-Xaa¹-B-D-homoLys-Xaa³-Xaa⁴-], [-Xaa¹-B-Arg-Xaa³-Xaa⁴] and [-Xaa¹-B-D-Arg-Xaa³-Xaa⁴].

More preferably Xaa² is arginine, most preferably L-arginine (Arg). Thus, the present invention preferably relates to a compound having the structure cyclo[-Xaa¹-B-Arg-Xaa³-Xaa⁴].

Thus, according to a particular preferred embodiment, the present invention also describes a compound according to a formula being selected from the group consisting of cyclo[-Phe-B-Arg-Xaa³-Xaa⁴], cyclo[-D-Phe-B-Arg-Xaa³-Xaa⁴], cyclo[-Tyr-B-Arg-Xaa³-Xaa⁴], cyclo[-D-Tyr-B-Arg-Xaa³-Xaa⁴], cyclo[-Trp-B-Arg-Xaa³-Xaa⁴], cyclo[-D-Trp-B-Arg-Xaa³-Xaa⁴], cyclo[-Phg-B-Arg-Xaa³-Xaa⁴], cyclo[-D-Phg-B-Arg-Xaa³-Xaa⁴], cyclo[-Nal-B-Arg-Xaa³-Xaa⁴] and cyclo[-D-Nal-B-Arg-Xaa³-Xaa⁴].

According to another preferred embodiment the present invention refers to a compound having a formula being selected from the group consisting of cyclo[-D-Tyr-B-Orn-Xaa³-Xaa⁴-], cyclo[-D-Tyr-B-D-Orn-Xaa³-Xaa⁴-], cyclo[-D-Tyr-B-Dap-Xaa³-Xaa⁴-], cyclo[-D-Tyr-B-D-Dap-Xaa³-Xaa⁴-], cyclo[-D-Tyr-B-Lys-Xaa³-Xaa⁴-], cyclo[-D-Tyr-B-D-Lys-Xaa³-Xaa⁴-], cyclo[-D-Tyr-B-homoLys-Xaa³-Xaa⁴-], cyclo[-D-Tyr-B-D-homoLys-Xaa³-Xaa⁴-], cyclo[-D-Tyr-B-Arg-Xaa³-Xaa⁴-], and cyclo[-D-Tyr-B-D-Arg-Xaa³-Xaa⁴-].

In particular, the present invention refers to a compound having a formula cyclo[-D-Tyr-B-Arg-Xaa³-Xaa⁴].

### The amino acid Xaa³:

Xaa³ is a natural or unnatural amino acid. As already described above, the term "natural or unnatural amino acid" refers to residues of any naturally occurring or synthetic amino acid and their respective D and L stereoisomers if their structures allow such stereoisomeric forms.

In this context of the invention, the term residues refers to building blocks being incorporated in the cyclic pentapeptide having the structure: 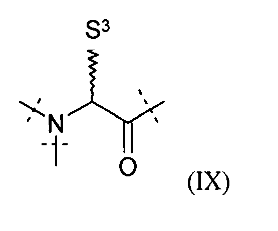 wherein S³ is the side chain of the natural or unnatural amino acid. For example in case the amino acid is glycine S³ is H. S³ may form a cyclic ring with the group N, in particular in case Xaa³ is proline or a proline derivative.

Preferably Xaa³ is a naturally or unnaturally amino acid comprising an aromatic moiety in its side chain S³. As regards, the term "amino acid comprising an aromatic moiety in its side chain" this term refers to an amino acid comprising an aromatic group as described above for Xaa¹. In this context, side chains comprising an aromatic moiety include side chains, with the aromatic moiety being directly attached to the C^{alpha} of the amino acid, thus with S³ being the aromatic moiety, as well as side chains S³ being substituted in any position with at least one aromatic moiety, such as for example alkyl chains being substituted with an aromatic moiety.

The term aromatic moiety as used in this context of the invention, refers to an optionally substituted aryl group and/or optionally substituted heteroaryl group, with the terms "aryl" and "substituted aryl", "heteroaryl" and "substituted heteroaryl" being as defined above.

The amino acid Xaa³ may have multiple asymmetric centers. As a consequence, the resulting cyclopeptides may occur as racemates, mixtures of enantiomers and as individual enantiomers, as well as diastereomers and mixtures of diastereomers. All asymmetric forms, possible stereoisomers, single isomers and mixtures of isomers are included within the scope of the present invention. The designation " 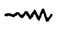 " shown in formula (IX) above refers to a bond to which the stereochemistry is not specifically designated.

Preferably, Xaa³ is present in enantiomerically pure form.

According to a preferred embodiment of the present invention, S³ comprises an aromatic group being selected from, optionally substituted, phenyl, naphthyl and indole, most preferably, S³ comprises an aromatic group being naphthyl.

According to a preferred embodiment, Xaa³ is selected from the group consisting of phenylalanine, tyrosine, tryptophan, phenylglycine, and naphthylalanine, i.e. Xaa³ is most preferably selected from the group consisting of L-phenylalanine (Phe), D-phenylalanine (D-Phe), L-tyrosine (Tyr), D-tyrosine (D-Tyr), L-tryptophan (Trp), D-tryptophan (D-Trp), D-phenylglycine (D-Phg), L-phenylglycine (Phg), L-naphthylalanine (Nal) and D-naphthylalanine (D-Nal).

According to a preferred embodiment, the present invention refers to a compound having a structure according to a formula selected from the group consisting of cyclo[-Xaa¹-B-Xaa²-Phe-Xaa⁴], cyclo[-Xaa¹-B-Xaa²-D-Phe-Xaa⁴], cyclo[-Xaa¹-B-Xaa²-Tyr-Xaa⁴], cyclo[-Xaa¹-B-Xaa²-D-Tyr-Xaa⁴], cyclo[-Xaa¹-B-Xaa²-Trp-Xaa⁴], cyclo[-Xaa¹-B-Xaa²-D-Trp-Xaa⁴], cyclo[-Xaa¹-B-Xaa²-Phg-Xaa⁴], cyclo[-Xaa¹-B-Xaa²-D-Phg-Xaa⁴], cyclo[-Xaa¹-B-Xaa²-Nal-Xaa⁴] and cyclo[-Xaa¹-B-Xaa²-D-Nal-Xaa⁴].

Further, the present invention relates to a compound, as described above, wherein Xaa¹ and Xaa³ are, independently of each other, selected from the group phenylalanine, D-phenylalanine, tyrosine, D-tyrosine, tryptophan, D-tryptophan, D-phenylglycine, phenylglycine, naphthylalanine (Nal) and D-naphthylalanine (D-Nal).

According to a particular preferred embodiment, the present invention refers to a compound having a structure according to a formula selected from the group consisting of cyclo[-Xaa¹-B-Arg-Phe-Xaa⁴], cyclo[-Xaa¹-B-Arg-D-Phe-Xaa⁴], cyclo[-Xaa¹-B-Arg-Tyr-Xaa⁴], cyclo[-Xaa¹-B-Arg-D-Tyr-Xaa⁴], cyclo[-Xaa¹-B-Arg-Trp-Xaa⁴], cyclo[-Xaa¹-B-Arg-D-Trp-Xaa⁴], cyclo[-Xaa¹-B-Arg-Phg-Xaa⁴], cyclo[-Xaa¹-B-Arg-D-Phg-Xaa⁴], cyclo[-Xaa¹-B-Arg-Nal-Xaa⁴] and cyclo[-Xaa¹-B-Arg-D-Nal-Xaa⁴].

According to another preferred embodiment, the present invention also describes a compound according to a formula being selected from the group consisting of cyclo[-D-Tyr-B-Xaa²-Phe-Xaa⁴], cyclo[-D-Tyr-B-Xaa²-D-Phe-Xaa⁴], cyclo[-D-Tyr-B-Xaa²-Tyr-Xaa⁴], cyclo[-D-Tyr-B-Xaa²-D-Tyr-Xaa⁴], cyclo[-D-Tyr-B-Xaa²-Trp-Xaa⁴], cyclo[-D-Tyr-B-Xaa²-D-Trp-Xaa⁴], cyclo[-D-Tyr-B-Xaa²-Phg-Xaa⁴], cyclo[-D-Tyr-B-Xaa²-D-Phg-Xaa⁴], cyclo[-D-Tyr-B-Xaa²-Nal-Xaa⁴] and cyclo[-D-Tyr-B-Xaa²-D-Nal-Xaa⁴].

According to a particularly preferred embodiment, the present invention refers to a compound having a structure according to a formula selected from the group consisting cyclo[-D-Tyr-B-Arg-Phe-Xaa⁴], cyclo[-D-Tyr-B-Arg-D-Phe-Xaa⁴], cyclo[-D-Tyr-B-Arg-Tyr-Xaa⁴], cyclo[-D-Tyr-B-Arg-D-Tyr-Xaa⁴], cyclo[-D-Tyr-B-Arg-Trp-Xaa⁴], cyclo[ -D- Tyr-B-Arg-D-Trp-Xaa⁴], cyclo[-D-Tyr-B-Arg-Phg-Xaa⁴], cyclo[-D-Tyr-B-Arg-D-Phg-Xaa⁴], cyclo[-D-Tyr-B- Arg-Nal-Xaa⁴] and cyclo[-D-Tyr-B-Arg-D-Nal-Xaa⁴].

According to a particular preferred embodiment, Xaa³ is L-naphthylalanine (Nal) or D-naphthylalanine (D-Nal), more preferably L-naphthylalanine.

Thus, the present invention preferably relates to a compound having the structure cyclo[-Xaa¹-B-Xaa ²-Nal-Xaa⁴] or cyclo[-Xaa¹-B-Xaa²-D-Nal-Xaa⁴], in particular cyclo[-Xaa¹-B-Xaa²-Nal-Xaa⁴], more preferably cyclo[-Xaa¹-B-Arg-Nal-Xaa⁴] or cyclo[-D-Tyr-B-Xaa²-Nal-Xaa⁴], most preferably cyclo[-D-Tyr-B-Arg-Nal-Xaa⁴].

In case, Xaa³ is Nal, e.g. the following structures are conceivable: 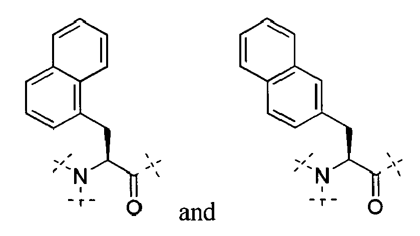 with L-2-naphthylalanine (2-Nal) being particularly preferred.

### The amino acid Xaa⁴:

Xaa⁴ is glycine or a D-amino acid of a natural or unnatural amino acid. As already described above, the term "natural or unnatural amino acid" refers to residues of any naturally occurring or synthetic amino acid.

In this context of the invention, the term residues refers to building blocks being incorporated into the cyclic pentapeptide having the structure: 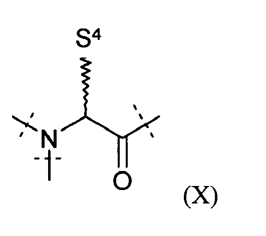 wherein S⁴ is the side chain of the natural or unnatural amino acid. Said unnatural amino acid may bear or may not bear a side chain that comprises or is linked to at least one, preferably one or two, further compound(s) having a structure according to formula (I). Accordingly, in some embodiments, S⁴ is a linker group linking the compound of formula (I), to a further compound having a structure according to formula (I), wherein the compound according to formula (I) and the further compound according to formula (I) may be the same or may be different.

According to preferred embodiments, S⁴ is the side chain of the natural or unnatural amino acid. For example, in case the amino acid is glycine, S⁴ is H. S⁴ may form a cyclic ring with the group N, in particular in case Xaa⁴ is a proline derivative. S⁴ may or may not comprise a compound D.

In case Xaa⁴ is a D-amino acid, the D-amino acid is preferably selected from the group consisting of D-diaminopropionic acid, D-diaminobutyric acid, D-ornithine, D-lysine. The amino acid Xaa⁴ may have multiple asymmetric centers. As a consequence, the resulting cyclopeptides may occur as racemates, mixtures of enantiomers and as individual enantiomers, as well as diastereomers and mixtures of diastereomers. All asymmetric forms, possible stereoisomers, single isomers and mixtures of isomers are included within the scope of the present invention, with the proviso that the stereocenter in C^{alpha} position, which is shown as " 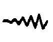 " in the formula (X) is selected in the way that, in case Xaa⁴ is not glycine, the resulting amino acid Xaa⁴ is present in D-conformation.

Preferably, Xaa⁴ is present in enantiomerically pure form.

According to a preferred embodiment, Xaa⁴ is selected from the group consisting of D-diaminopropionic acid, D-diaminobutyric acid, D-ornithine, D-lysine.

According to an alternative preferred embodiment, Xaa⁴ is glycine. Thus, according to one embodiment, the present invention also describes a compound having the following structure:
cyclo[-Xaa¹-B-Xaa²-Xaa³-Gly-].

According to a preferred embodiment, the present invention relates to a compound, as described above, wherein Xaa² is L-arginine and wherein Xaa⁴ is glycine.

Thus, according to one embodiment, the present invention also describes a compound having the following structure:
cyclo[-Xaa¹-B-Arg-Xaa³-Gly-].

The following list illustrates, but does not limit, further preferred compounds of the invention:
cyclo[-D-Tyr-B-Arg-Nal-Gly-]
cyclo[-Tyr-B-Arg-Nal-Gly-]
cyclo[-D-Phe-B-Arg-Nal-Gly-]
cyclo[-Phe-B-Arg-Nal-Gly-]
cyclo[-D-Trp-B-Arg-Nal-Gly-]
cyclo[-Trp-B-Arg-Nal-Gly-]
cyclo[-Nal-B-Arg-Nal-Gly-]
cyclo[-D-Nal-B-Arg-Nal-Gly-]
cyclo[-D-Tyr-B-Arg-D-Nal-Gly-]
cyclo[-D-Tyr-B-Arg-Phe-Gly-]
cyclo [-D-Tyr-B-Arg-D-Phe-Gly-]
cyclo [-D-Tyr-B-Arg-Tyr-Gly-]
cyclo [-D-Tyr-B-Arg-D-Tyr-Gly-]
cydo[-D-Tyr-B-Arg-Trp-Gly-]
cyclo[-D-Tyr-B-Arg-D-Trp-Gly-]

According to a particular preferred embodiment, the present invention relates to a compound, as described above, having the formula cycle [D-Tyr-B-Arg-Nal-Gly].

### The structural unit B:

As regards the structural unit B, said unit is an amino acid group having a structure according to formula (II)

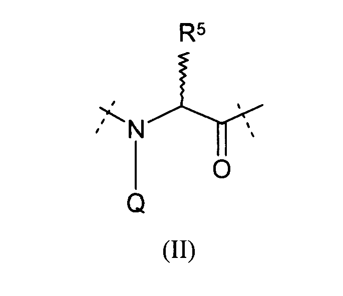

### The structural unit O:

As described above, the residue Q is a moiety being sterically more demanding than a methyl group. Preferably, Q is a linker moiety comprising the group -X-R⁶. Preferably, Q is a linker moiety comprising the group -X-R⁶ with X being selected from the group consisting of =N-, -N=, -Y-, -C(=Y)-NH-, -NH-C(=Y)-, -NH-C(=Y)-Y'-, -Y'-C(=Y)-NH-, -Y'-C(=Y)-, -C(=Y)-Y'-, -C(=Y)-, alkynylene, azide, and 1,2,3-triazoles, with Y and Y' being independently of each other selected from the group consisting of NH, O and S, and with R⁶ being selected from the group consisting of H, optionally substituted alkyl, optionally substituted aryl, optionally substituted alkoxy group, optionally substituted heteroaryl, a compound D; and wherein in case R⁶ is substituted, the substituent preferably comprises a compound D; preferably wherein the compound D is selected from the group consisting of detectable labels, organic complexation agents, active substances and combinations thereof, particularly wherein the active substance is selected from the group consisting of cytotoxic agents, lipids, sugars, sugar conjugates, sugar derivatives, proteins and combinations thereof, particularly wherein the compound D is selected from the group consisting of detectable labels, organic complexation agents, cytotoxic agents, and combinations thereof.

According to one aspect of the invention the functional group X is selected from the group consisting of -NH-, -S-, -O-, =N-, -N=, -C(=O)-NH-, -C(=S)-NH-, -C(=NH)-NH-, -NH-C(=S)-, -NH-C(=O)-, -NH-C(=NH)-, -NH-C(=O)-NH-, -NH-C(=S)-NH-, -NH-C(=NH)-NH-, -NH-C(=O)-O-, -C(=O)-NH-, -C(=O)-O-, -NH-C(=O)-, -O-C(=O)-, -C(=S)-O-, -C(=O)-, -C(=S)-, alkynylene, azide, and 1,2,3-triazoles. In preferred embodiments, the functional group X is -C(=NH)-NH-. In other preferred embodiments, the functional group X is -NH-C(=NH)-.

According to a preferred aspect of the invention the functional group X is selected from the group consisting of -NH-, -C(=O)-NH-, -NH-C(=O)-, -NH-C(=NH)-NH-, -C(=O)-O-, and -O-C(=O)-.

According to an alternative aspect of the invention the group X is a 1,2,3-triazole. In the event that X is a triazole, inter alia, the following structures are conceivable for the structural unit -X-R⁶:

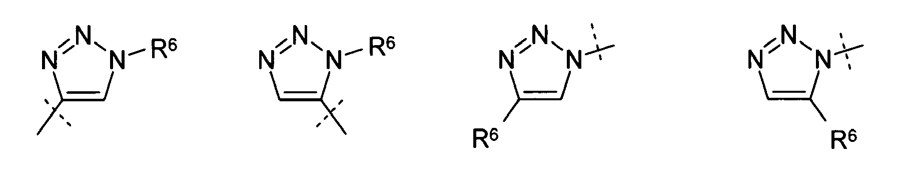

Preferably such a triazole group is formed via a 1,3-dipolar cycloaddition between an azide and a terminal or internal alkyne to give a 1,2,3-triazole.

The residue R⁶ being attached to group X is preferably selected from the group consisting of H, optionally substituted alkyl, optionally substituted aryl, optionally substituted alkoxy, and optionally substituted heteroaryl or R⁶ comprises or is a compound D, with the terms "alkyl", "alkoxy", "substituted alkoxy", "substituted alkyl", "aryl" and "substituted aryl", "heteroaryl" and "substituted heteroaryl" being as defined above.

In case R⁶ is substituted, the substituent preferably comprises or is a halogen atom or a compound D; particularly selected from the group consisting of detectable labels, organic complexation agents, active substances, and combinations thereof, particularly wherein the active substance is selected from cytotoxic agents, lipids, sugars, sugar conjugates, sugar derivatives, proteins and combinations thereof; more particularly selected from the group consisting of detectable labels, organic complexation agents, cytotoxic agents, and combinations thereof.

According to one embodiment R⁶ is H or

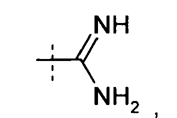

Thus, the present invention also describes a compound, as described above, wherein R⁶ is H or 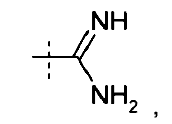 preferably wherein R⁶ is

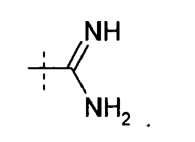

According to another preferred embodiment R⁶ comprises an organic complexation agent or a combination of an organic complexation agent and a detectable label, or R⁶ consists of an organic complexation agent or a combination of an organic complexation agent and a detectable label.

The moiety -X-R⁶ may be linked directly to the N^{alpha} atom of the amino acid. Alternatively, the moiety Q comprises a suitable spacer moiety linking the functional groups X and the group N^{alpha}. Any conceivable spacer moiety is meant to be included in the present application. Thus, said spacer moiety may comprise alkyl chains or alkoxy chains, peptidic structures, sugar moieties, aromatic groups, and the like.

Preferably, the moiety Q comprises in addition to the group -X-R⁶ at least one further linking group (linker_{Q}) linking the N^{alpha} of the amino acid building block B and the group X.

Thus the group Q is preferably a group having the general structure -linker_{Q}-X-R⁶.

Thus, B has preferably the structure:

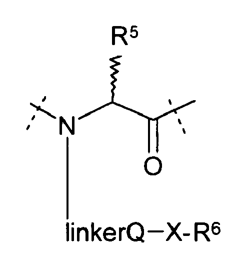

Accordingly, the compound of the present invention preferably has a structure according to

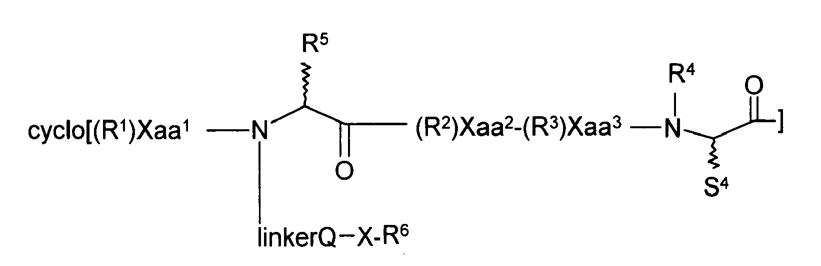

Linker_{Q} may be any suitable linking group such as alkyl groups, aryl groups, alkoxy groups, aralkyl groups and the like. Preferably linker_{Q} is an alkoxy group or an alkyl group, most preferably linker_{Q} is an alkyl chain. The at least one alkyl chain preferably contains from 1 to 25 carbon atoms. The alkyl chain may be interrupted by one or more heteroatoms.

Most preferably the group Q has a structure according to the formula -[C(R⁷R⁸)]ₙ-X-R⁶, with R⁷ and R⁸ being independently of each other selected from the group consisting of H and optionally substituted alkyl, preferably with both R⁷ and R⁸ being H.

As regards integer n, said integer is preferably in the range of from 1 to 25. It is further preferred that integer n is in the range of from 1 to 20, more preferably in the range of from 1 to 16, more preferably in the range of from 1 to 10, more preferably in the range of from 1 to 6, such as 1, 2, 3, 4, 5 or 6, most preferably in the range 3 to 6.

According to a further preferred embodiment, X is selected from the group consisting of -Y- or -C(=Y)-Y'-, - Y'-C(=Y)-, -NH-C(=Y)-Y'-, -Y'-C(=Y)-NH-, with Y and Y' being NH, wherein Q is -[C(R⁷R⁸)]ₙ-X-R⁶ with both R⁷ and R⁸ being H, and preferably with n being 1, 2, 3, 4, 5 or 6.

Thus, according to a preferred aspect of the invention, the present invention describes a compound of formula (I), as described above with Q being selected from the group consisting of -CH₂-NH-R⁶, -(CH₂)₂-NH-R⁶, -(CH₂)₃-NH-R⁶, -(CH₂)₄-NH-R⁶, -(CH₂)₅-NH-R⁶ and -(CH₂)₆-NH-R⁶, more preferably Q being selected from the group consisting of -CH₂-NH-C(=NH)-NH₂, -(CH₂)₂-NH-C(=NH)-NH₂, -(CH₂)₃-NH-C(=NH)-NH₂, -(CH₂)₄-NH-C(=NH)-NH₂, -(CH₂)₅-NH-C(=NH)-NH₂, -(CH₂)₆-NH-C(=NH)-NH₂, -CH₂-NH₂, -(CH₂)₂-NH₂, -(CH₂)₃-NH₂, -(CH₂)₄-NH₂, -(CH₂)₅-NH₂ and -(CH₂)₆-NH₂, more preferably Q being selected from the group consisting of -CH₂-NH-C(=NH)-NH₂, -(CH₂)₂-NH-C(=NH)-NH₂, -(CH₂)₃-NH-C(=NH)-NH₂, -(CH₂)₄-NH-C(=NH)-NH₂, -(CH₂)₅-NH-C(=NH)-NH₂, -(CH₂)₆-NH-C(=NH)-NH₂, most preferably Q being -(CH₂)₆-NH-C(=NH)-NH₂.

In certain embodiments, any one of X, linker_{Q}-X or R⁶ may or may not be linked to at least one, preferably one or two, further compound(s) having a structure according to formula (I). Accordingly, in some embodiments, X or R⁶ is a linker group linking the compound of formula (I), to at least one further compound having a structure according to formula (I), wherein the compound according to formula (I) and the further compound according to formula (I) may be the same or may be different. In the latter embodiments, in case R⁶ is linked to one or more further compound(s) having a structure according to formula (I), a hydrogen of R⁶ may be replaced by said further compound(s). Such a group R⁶ may also be referred to herein as R^{6,}. X or R⁶ may also be substituted by a group which is capable of being coupled to said further compound(s). Optionally, in case X or R⁶ is linked to one or more further compound(s) having a structure according to formula (I), the linkage may additionally comprise one or more bifunctional linkers described herein.

Other preferred embodiments for residue Q, linker_{Q}, X and/or R⁶ can be taken from the particular compounds disclosed in the Examples herein.

### The residue R⁵:

As regards residue R⁵, said residue R⁵ is selected from the group consisting of H, optionally substituted alkyl, optionally substituted aryl, optionally substituted heteroaryl and a moiety L. The terms "alkyl", "alkoxy", "substituted alkoxy", "substituted alkyl", "aryl" and "substituted aryl", "heteroaryl" and "substituted heteroaryl" are as defined above.

According to a preferred embodiment, L is a linker moiety, such as an alkyl group, alkoxy group, aryl group, heteroaryl group, aralkyl group, alkynyl group or the like, being optionally substituted with a compound D. Said compound D is particularly selected from the group consisting of detectable labels, organic complexation agents, active substances and combinations thereof, particularly wherein the active substance is selected from cytotoxic agents and lipids. The linker group L may comprise further suitable functional groups for attachment of compound D, known to those skilled in the art.

In case R⁵ is a substituted alkyl, R⁵ is preferably substituted with an amino group.

In preferred embodiments, R⁵ and particularly L comprises or is a compound D as defined herein. According to certain embodiments, R⁵ comprises or is an organic complexation agent or a combination of an organic complexation agent and a detectable label. An exemplary residue R⁵ can be taken from the below exemplary compound envisaged according to the invention, wherein "Ga" may be a Ga radionuclide, preferably ⁶⁸Ga:

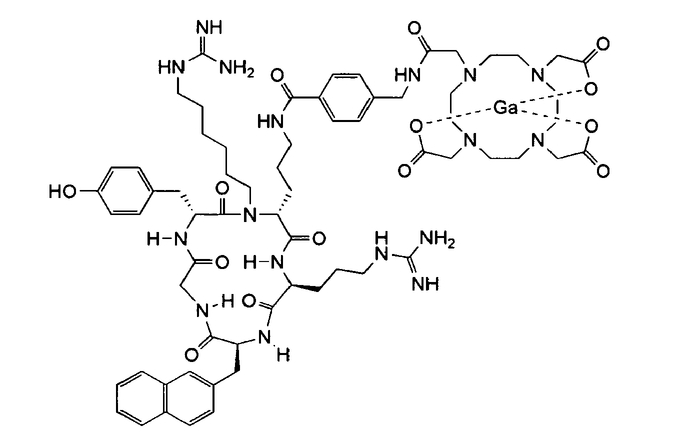

According to a preferred aspect of the invention, R⁵ is selected from the group consisting of H, methyl, aminomethyl, 1-aminoethyl, 1-aminopropyl, 1-aminopentyl, 1-aminobutyl and 1-aminohexyl, preferably wherein R⁵ is H or methyl, in particular wherein R⁵ is methyl.

The following structures are meant to illustrate, but do not limit, preferred amino acids B of the invention:

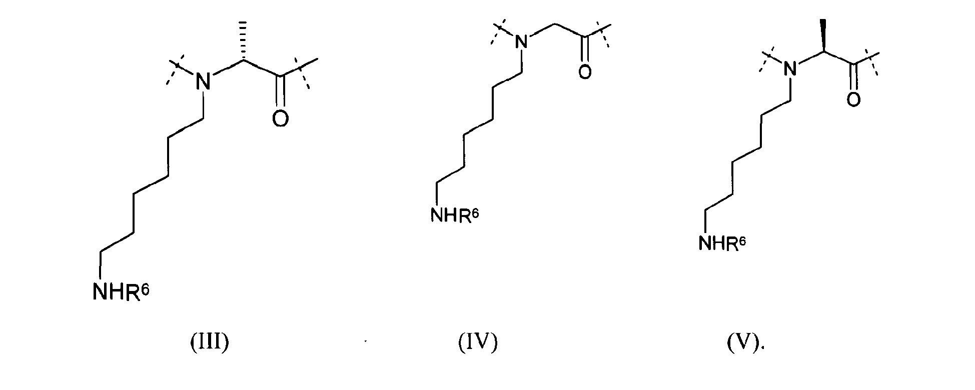

The present invention also relates to a compound as described above, preferably having a formula
cyclo[D-Tyr-B-Arg-Nal -Gly],
wherein B is an amino acid group having a structure according to formula III, IV or V

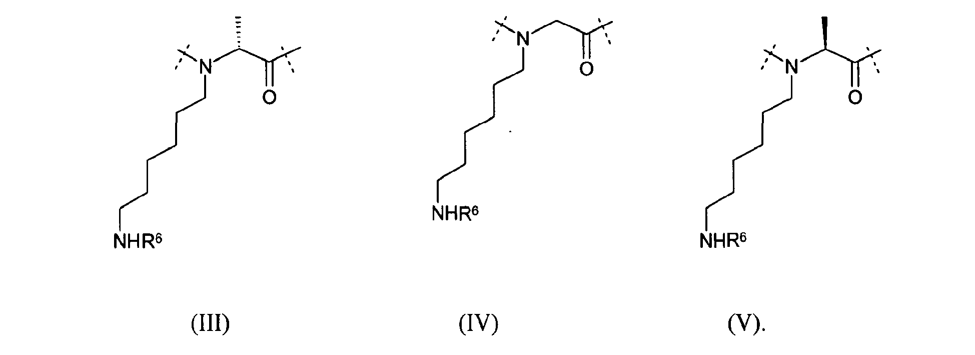

The residue B may have multiple asymmetric centers. As a consequence, the resulting cyclopeptides may occur as racemates, mixtures of enantiomers and as individual enantiomers, as well as diastereomers and mixtures of diastereomers. All asymmetric forms, possible stereoisomers, single isomers and mixtures of isomers are included within the scope of the present invention.

Preferably the stereocenter in C^{alpha} position, which is shown as " 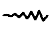 " in the formula (II) is selected in the way that the resulting amino acid is present in D-conformation. Thus, the present invention also relates to a compound, as described above, wherein the amino acid group having the structure according to formula (II) is present in D-configuration.

Preferably, B is present in enantiomerically pure form.

In certain embodiments of the invention, any one of L or R⁵ may or may not be linked to at least one, preferably one or two, further compound(s) having a structure according to formula (I). Accordingly, in some embodiments, L or R⁵ is a linker group linking the compound of formula (I), to at least one further compound having a structure according to formula (I), wherein the compound according to formula (I) and the further compound according to formula (I) may be the same or may be different. In the latter embodiments, in case L or R⁵ is linked to one or more further compound(s) having a structure according to formula (I), L or R⁵ is preferably substituted with said further compound(s). Such a group R⁵ may also be referred to herein as L' or R⁵'. L or R⁵ may also be substituted by a group which is capable of being coupled to said further compound(s). Optionally, in case L or R⁵ is linked to one or more further compound(s) having a structure according to formula (I), the linkage may additionally comprise one or more bifunctional linkers described herein.

### Multimeric structures:

The present invention also describes multimeric compounds. The term "multimeric compounds" as used in the present application refers to compounds of formula (I), as described above which are linked via a suitably linking moiety to at least one further compound according to formula (I), wherein both compounds of formula (I) may be the same or may be different. In case the compound of formula (I) is linked to one further compound of formula (I) a dimeric compound results. In case three compounds are crosslinked, a trimeric compound results. The covalent linkage of two or more high affinity ligands for a receptor system using a linker or spacer group can increase the apparent binding affinity of the entire construct when compared to the monomeric ligands.

The compound of formula (I) may be linked via group R⁵, L, X, R⁶ and/or Xaa⁴, to said at least one further compound. Preferably, it is linked via building block B, particularly via R⁵ or L to said at least one further compound. In certain preferred embodiments, the further compound is coupled via its group R⁵ and/or group R⁶ and/or amino acid Xaa⁴.

Thus, some embodiments of the invention relate to a multimeric compound wherein a compound of formula (I) is linked via i) R⁵, particularly group L; and/or ii) group Q, particularly group X or R⁶; and/or iii) amino acid Xaa⁴, particularly group S⁴; to at least one further compound according to formula (I). In this aspect, the compound according to formula (I) and the further compound according to formula (I) generally may be the same or may be different.

Accordingly, certain embodiments relate to a compound of formula (I) wherein the compound is linked via i) R⁵, particularly group L; and/or ii) group Q, particularly group X or R⁶; and/or iii) amino acid Xaa⁴, particularly group S⁴; to at least one further compound according to formula (I).

In case, the compound of formula (I) is linked via Xaa⁴ to at least one further compound, the residue S⁴ of the amino acid Xaa⁴ is preferably a linker group, as described above. In said case, the compound of formula (I) is linked via group S⁴ to a further compound having a structure according to formula (I), wherein the compound according to formula (I) and the further compound according to formula (I) may be the same or may be different.

In case the compound of formula (I) is linked via R⁶ to at least one further compound, the residue R⁶ is preferably selected from the group consisting of substituted alkyl, substituted aryl, substituted alkoxy group, substituted heteroaryl, with the at least on substituent being a group capable of being coupled to a functional group of group L and/or group R⁶ and/or amino acid Xaa⁴ of the further compound. Again, the compound according to formula (I) and the further compound according to formula (I) may be the same or may be different.

In case, the compound of formula (I) is linked via group L to at least one further compound, the moiety L is preferably selected from the group consisting of substituted alkyl, substituted aryl, substituted alkoxy group, substituted heteroaryl, bearing at least one substituent, said substituent being a group capable of being coupled to a functional group of group L and/or of group R⁶ and/or of amino acid Xaa⁴ of the further compound. Again, the compound according to formula (I) and the further compound according to formula (I) may be the same or may be different.

In the following the residues of the further compound according to formula (I) are marked with an "*" to clarify that the further compound (I) may be different from the first compound. Thus the further compound can also be described using formula (I*) as a compound having the structure

cyclo[(R¹*)Xaa¹*-B*-(R²*)Xaa²*-(R³*)Xaa³*-(R⁴*)Xaa⁴*] (I*)

or a pharmaceutically acceptable salt thereof,
wherein Xaa¹* and Xaa³* are independently of each other, and independently of Xaa¹ and Xaa³, a natural or unnatural amino acid,
Xaa²* is, independently of Xaa², a natural or unnatural basic amino acid,
Xaa⁴* is, independently of Xaa⁴, a D-amino acid residue having the structure 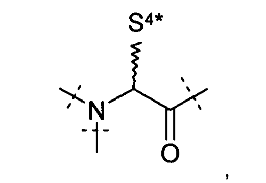 R¹*, R²*, R³* and R⁴* are independently of each other, and independently of R¹, R², R³ and R⁴, H or methyl,
B* is an amino acid group having a structure according to formula II* 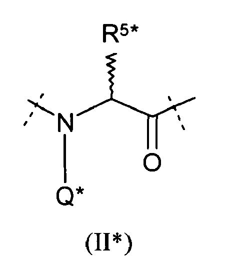 with Q* and R⁵* being as defined above and below.

As regards further definitions of the groups R¹*, Xaa¹*, B*, R²*, Xaa²*, R³*, Xaa³*, R⁴*, Xaa⁴*, R⁵* and Q* and the preferred embodiments of these groups, reference is made to the description of the respective groups R¹, Xaa¹, B, R², Xaa², R³, Xaa³, R⁴, Xaa⁴, R⁵ and Q which equally apply to the groups of the further compounds, with the proviso that the groups R¹*, Xaa¹*, B*, R²*, Xaa²*, R³*, Xaa³*, R⁴*, Xaa⁴*, R⁵* and Q* and the groups R¹, Xaa¹, B, R², Xaa², R³, Xaa³, R⁴, Xaa⁴ , R⁵ and Q are independently selected from each other.

Generally, as to any of the groups of formula (I*), such as groups S⁴*, L*, R⁶*, X*, linkerQ*, and the preferred embodiments of these groups, reference is made to the description of their corresponding groups, such as groups S⁴, L, R⁶, X, linkerQ, and the preferred embodiments of these groups, which equally applies to the groups of the further compounds. Any of said groups are selected independently from each other and may be the same as their counterparts or different therefrom.

According to this aspect, in certain embodiments, it is envisaged that one or more of R⁵ (or L, respectively), R⁵* (or L*, respectively), R⁶ (or X respectively), R⁶* (or X* respectively), S⁴, and S⁴* may or may not be absent, when two or more compounds are linked via any of its respective group. For example, if compounds according to formula (I) are linked via R⁵ and R⁵*, one of R⁵ and R⁵* may be absent. For example, if compounds according to formula (I) are linked via R⁵ and S⁴*, one of R⁵ and S⁴* may be absent.

As described above, the further compound or the compound of formula (I*), respectively, may e.g. be linked via its group R⁵* (or L*) and/or R⁶* (or X*) and/or via its groups S⁴*, to the compound of formula (I). As described above, each of these groups of the further compound may be the same or may be different from the respective group of the first compound, as long as they are encompassed by the respective definitions of these groups as defined above and below. In case the compound of the present invention is linked via its group S⁴ to the further compound, said further compound is preferably linked via its group S⁴*.

The linker group S⁴, and if present the linker group S⁴* of the further compound may, independently of each other, comprise alkyl chains of 1 to 20 carbon atoms, which may be branched or unbranched, substituted or unsubstituted, and which may be interrupted by one or more heteroatoms, cyclic groups and/or heterocyclic groups, and which have at least one functional group suitable for linking both linking groups with each other, or optionally via a further bifunctional or multifunctional linker compound linker_{S4}.

According to one embodiment, the compound has thus, for example, a structure according to the following formula:

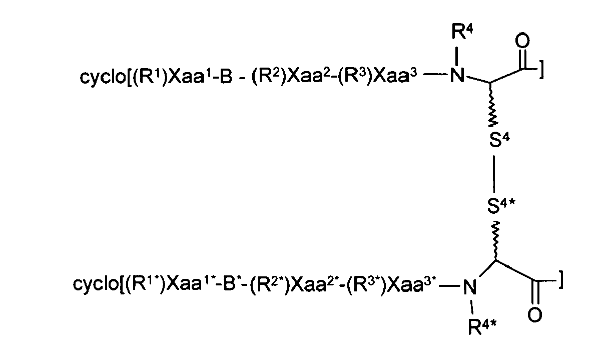

According to another embodiment, the compound has thus, for example, a structure according to the following formula:

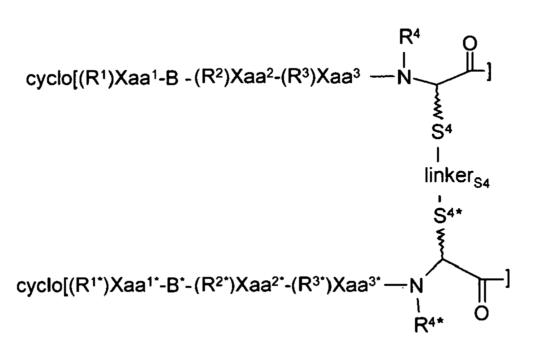

The alkyl chains of S⁴ and S⁴*, if present, may contain from 1 to 14 carbon atoms; for example, from 4 to 10 carbon atoms. The total length of the spacer groups S⁴ and/or S⁴*, taking into account any interrupting heteroatoms and/or cyclic groups, may be equivalent to a linear alkyl chain of approximately 1 to 20 carbons, e.g. 1 to 14 carbons, such as 4 to 10 carbons. As used herein, e.g. in case of the presence of a linker_{S4}, linker groups S⁴ and S⁴* may also be referred to as spacer groups. In certain embodiments, the spacer groups S⁴ and/or S⁴* may comprise an amino acid (or short oligopeptide chain) attached to the side group of one of the amino acids on the cyclic oligopeptide so as to branch off therefrom.

The separation between two or more monomeric compounds of formula (I) (or formula (I*) respectively) (the separation being made up, it will be appreciated, by the bifunctional or multifunctional linker and the spacer groups such as S⁴ and/or S⁴* when present) preferably is long enough to avoid an undesired interference of both compounds. Based on the respective structure of the compounds, the skilled person will readily be able to determine a suitable length.

In principle, any functional group known to those skilled in the art can be used to link S⁴ and/or S⁴*. Suitably functional groups comprised in group S⁴ and/or S⁴* are for example hydroxyl, carboxy, acyl, guanidino, thiol, thio reactive groups, such as maleimide, alkylene, aminooxy and activated derivatives of such groups (e.g. activated esters). The skilled person will readily be able to select suitable functional groups to be linked to each other.

The multifunctional linker comprises at least two functional groups capable of being coupled with the respective functional groups of the linker S⁴ and/or S⁴*. As will be understood by the skilled person, and as suggested above, a variety of functional groups are suitable, including amino, hydroxyl, carboxy, acyl, amido, guanidino, alkynyl, azido, thiol, thio reactive groups, such as maleimide groups, alkylene, aminooxy and activated derivatives of such groups (e.g. activated esters). The skilled person will readily be able to select spacers and a linker with appropriate functional groups for attachment to the functional groups of the chosen cyclic oligopeptides. Approaches to protection and activation of the various groups on the oligopeptides are also within his general knowledge.

In the compounds of the invention, a multifunctional linker may for example comprise or may be derived from a linking agent which comprises, respectively: a group selected from dicarboxylic acids, amino acids, linear oligopeptides, alkynes, dioximes (e.g. formed by the reaction of a dialdehyde-containing linker group and cyclic oligopeptides derivatized (e.g. at a pendant amino group) to contain one or more aminooxy groups, such as aminooxy acetyl), poly(alkylene glycols), carboxylic acid- and/or amino-substituted poly(alkylene glycols), sugars, amino-modified sugars, polyamines and oligo- or poly-aminooxy-functionalised species, such as functionalised peptides or resins, these groups are selected so that they are capable of being reacted with the functional groups of the cyclopeptide.

Regarding S⁴ and/or S⁴*, the multifunctional linker may be referred to as multifunctional linker C or linker S⁴.

In case S⁴ and/or S⁴* comprises an amino group, the multifunctional linker (C) preferably comprises at least one carboxy group or an aldehyde function. In case S⁴ and/or S⁴* comprises a carboxy group, the multifunctional linker preferably comprises at least one amino group. In case S⁴ and/or S⁴* comprises an amino group, the multifunctional linker preferably comprises at least one carboxy group. In case S⁴ and/or S⁴* comprises a thio group, the multifunctional linker preferably comprises at least one thio group or one thio group. According to one aspect of the invention, a bifunctional linker is employed to link the compound of formula (I) with the further compound according to formula (I), i.e. the compound of formula (I) with the compound of formula (I*).

The following structure illustrates a preferred compound of the invention:

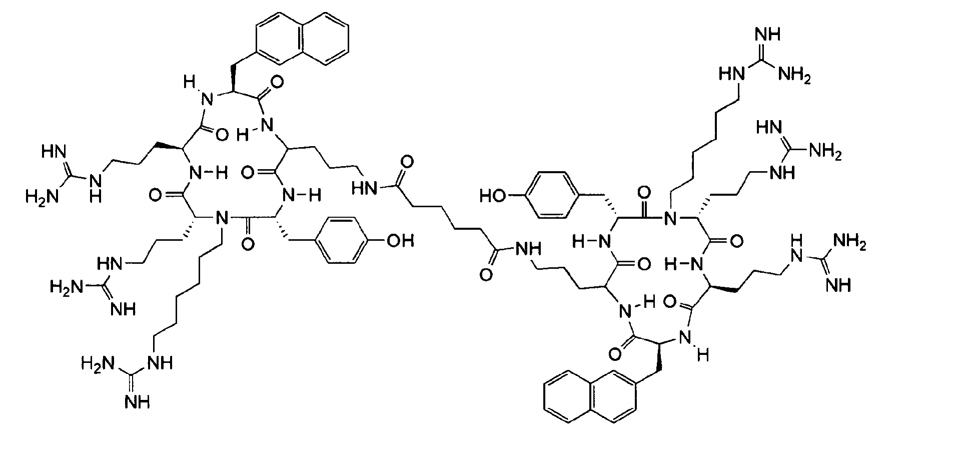

In embodiments of another aspect of the present invention, the multifunctional linker contains at least three functional groups, two of which are suitable for attachment of the cyclic oligopeptide, and one of which is suitable for attachment of a compound D, such as a detectable label or cytotoxic moiety, or of a further linker moiety.

Compounds containing such trifunctional (or more highly functionalized) linkers allow the ready attachment of two oligopeptide moieties of formula (I) plus further groups having specific functions. For example, a third functional group on the multifunctional linker may be employed for the attachment of a cytotoxic compound for targeted chemotherapy of tumors having metastatic potential, and their associated metastases, as a result of the relatively high expression of CXCR4 by such tissues. Preferred cytotoxic moieties may be selected from any of those cytotoxic compounds generally used for chemotherapy of the tumor concerned.

Alternatively, the third functional group may be employed for attachment of a radiolabel (e.g. by means of a chelated radionuclide) for targeted radiotherapy or for diagnostic imaging. Furthermore, the additional functional groups on the multifunctional linker may be employed for the attachment of further cyclic oligopeptide moieties for increasing the affinity and specificity of the compounds. A wide variety of the multifunctional linker may be used and these may contain a large number of functional groups, for example, in the case of peptide-based functional groups. Essentially, therefore, there is no upper limit to the number of cyclic oligopeptides or spacer groups which may be joined to the multifunctional linker.

Moreover, the additional functional groups on a multifunctional linker (such as a peptide) may be used for the coupling of multiple linkers, each bearing two or more cyclic oligopeptides, to each other. In this manner, higher multimers (and dendrimers) may be prepared.

In case the compound of the present invention is linked via its group R⁶ to the further compound, said further compound is preferably linked via the group X* of that further compound to the first compound. Thus, both compounds preferably share the same group R⁶. Alternatively, a bifunctional or multifunctional linker (linker_{R6}) may be used to link R⁶ and R⁶*.

As non limiting example, a preferred structure is presented below.

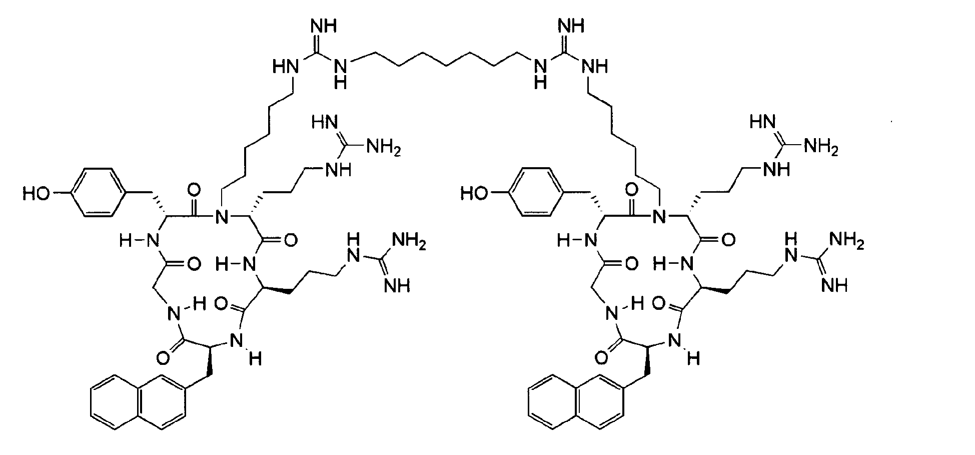

According to one embodiment of the present invention, the group R⁶, of the further compound are, is preferably a branched or unbranched alkyl chain, optionally being interrupted by one or more heteroatoms, more preferably an alkyl group being selected from the group consisting of ethyl, propyl, pentyl, butyl and hexyl and wherein said group R⁶ is being attached to further compound of formula (I), i.e. the further compound (I*), preferably via group X of said further compound of formula (I), with said X more preferably being -NH-C(=O)- or -C(=O)-NH-; or preferably being -NH-C(=NH)- or -C(=NH)-NH-.

Alternatively, the group R⁶ may be a branched or unbranched alkyl chain, optionally being interrupted by one or more heteroatoms, comprising at least one further functional group suitable for attachment of a detectable label or cytotoxic moiety, or a further linker moiety.

In case the compound of the present invention is linked via its group L to the further compound, said further compound is preferably linked via the group L (L*) of that further compound to the first compound.

The linker moiety L and the linker moiety L* have at least one functional group suitable for linking both linking groups with each other, or to another functional group of the respective other compound, or optionally via a further multifunctional, e.g. bifunctional, linker compound E.

In principle any functional groups known to those skilled in the art can be used to link L and/or L*.

The multifunctional linker comprises at least two functional groups capable of being coupled with the respective functional groups of the linker L and/or L*. As would be understood by the skilled person, and as suggested above, a variety of functional groups would be suitable, including amino, hydroxyl, carboxy, acyl, amido, guanidino, alkynyl, azido, thiol, alkylene, aminooxy and activated derivatives of such groups (e.g. activated esters). The skilled person would readily be able to select spacers and a linker with appropriate functional groups for attachment to the functional groups of the chosen cyclic oligopeptides. Approaches to protection and activation of the various groups on the oligopeptides would also be within his general knowledge.

In the compounds of the invention, the multifunctional linker E may for example comprise or may be derived from a linking agent which comprises: a group selected from dicarboxylic acids, amino acids, linear oligopeptides, alkynes, dioximes (e.g. formed by the reaction of a dialdehyde-containing linker group and cyclic oligopeptides derivatized (e.g. at a pendant amino group) to contain one or more aminooxy groups, such as aminooxy acetyl), poly(alkylene glycols), carboxylic acid- and/or amino-substituted poly(alkylene glycols), sugars, amino-modified sugars, polyamines and oligo- or poly-aminooxy-functionalised species, such as functionalised peptides or resins, these groups are selected so that they are capable of being reacted with the functional groups of the cyclopeptide.

In case L and/or L* comprises an amino group, the multifunctional linker preferably comprises at least one carboxy group or an aldehyde function. In case L and/or L* comprises an carboxy group, the multifunctional linker E preferably comprises at least one amino group. In case L and/or L* comprises an amino group, the multifunctional linker preferably comprises at least one carboxy group. In case L and/or L* comprises a thio group, the multifunctional linker preferably comprises at least one thio group or a thio group.

According to one aspect of the invention, a bifunctional linker E is employed to link the compound of formula (I) with a further compound according to formula (I), i.e. the compound of formula (I) with the compound of formula (I*).

In embodiments of another aspect of the present invention, the linker E contains at least three functional groups, two of which are suitable for attachment of the cyclic oligopeptide, and one of which is suitable for attachment of a detectable label or cytotoxic moiety, or a further linker moiety.

Compounds containing such trifunctional (or more highly functionalised) linkers allow the ready attachment of two oligopeptide moieties of formula (I) plus further groups having specific functions. For example, a third functional group on the linker E may be employed for the attachment of a cytotoxic compound for targeted chemotherapy of tumors having metastatic potential, and their associated metastases, as a result of the relatively high expression of CXCR4 by such tissues. Preferred cytotoxic moieties may be selected from any of those cytotoxic compounds generally used for chemotherapy of the tumor concerned.

Alternatively, the third functional group may be employed for attachment of a radiolabel (e.g. by means of a chelated radionuclide) for targeted radiotherapy or for diagnostic imaging. Furthermore, the additional functional groups on the linker E may be employed for the attachment of further cyclic oligopeptide moieties for increasing the affinity and specificity of the compounds. A wide variety of linkers E may be used and these may contain a large number of functional groups, for example, in the case of peptide-based functional groups. Essentially, therefore, there is no upper limit to the number of cyclic oligopeptides or spacer groups which may be joined to the linker E.

Moreover, the additional functional groups on a multifunctional linker (such as a peptide) may be used for the coupling of multiple linkers, each bearing two or more cyclic oligopeptides, to each other, in this manner, higher multimers (and dendrimers) may be prepared.

Thus, according to another preferred embodiment, the present invention for example relates to a compound having the following structure:

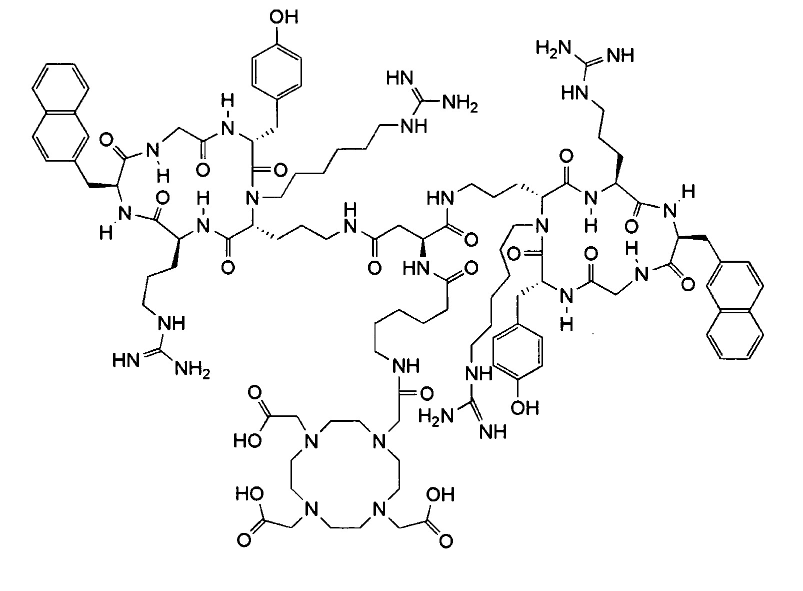

### The compound D:

The "compound D" which may also be referred to herein as "D" or "further compound D" as used herein preferably refers to a substance selected from the group consisting of detectable labels, organic complexation agents, active substances and combinations thereof. Preferably, a compound D is selected from the group consisting of detectable labels, organic complexation agents, cytotoxic agents, and combinations thereof. In some preferred embodiments, the compound D is selected from a detectable label, an organic complexation agent and a combination thereof. In certain preferred embodiments, D comprises, preferably is a detectable label. In certain preferred embodiments, D comprises, preferably is an organic complexation agent. In further preferred embodiments, D comprises, preferably is a combination of an organic complexation agent and a detectable label.

Preferably, the complexation agent is covalently bound to L, R⁵, the C^{alpha} of B, X, R⁶ or S⁴, especially to R⁵.

In certain other embodiments of the invention, D comprises, preferably is a combination of an organic complexation agent and Ga, wherein said Ga is not a radionuclide, such as wherein Ga is a non-radioactive isotope. In certain other embodiments of the invention, D comprises, preferably is a combination of an organic complexation agent and In, wherein said In is not a radionuclide, such as wherein In is a non-radioactive isotope.

In another preferred embodiment, the compound D comprises, preferably is, an active substance. Active substances are well-known to the skilled person. As used herein, active substances are preferably selected from cytotoxic agents, lipids, sugars, sugar conjugates, sugar derivatives, proteins and combinations thereof. In preferred embodiments, an active substance is a cytotoxic agent or a lipid, particularly a cytotoxic agent. "Cytotoxic agents", which may also be referred to herein as "cytotoxic moieties" or "cytotoxic compounds", are well known to the skilled person. Particular examples for cytotoxic moieties, including radionuclides, are disclosed hereinbelow. In other preferred embodiments, the active substance is selected from sugars, sugar conjugates, sugar derivatives, proteins and combinations thereof. Preferably, the protein is an enzyme or an antibody.

In certain preferred embodiments, particularly for endoradiotherapeutic purposes, compounds of the invention comprise a compound D which comprises or is a radioisotope selected from the group consisting of ^{114m}In_{,} ¹⁸⁶Re, ¹⁸⁸Re, ⁷⁷As, ⁹⁰Y, ⁶⁶Ga, ⁶⁷Cu, ¹⁶⁹Er, ¹¹⁷Sn, ¹²¹Sn, ¹²⁷Te, ¹⁴²Pr, ¹⁴³Pr, ^{195m}Pt, ¹⁹⁸Au, ¹⁹⁹Au, ¹⁴⁹Tb, ¹⁶¹Tb, ¹⁰⁹Pd, ¹⁶⁵Dy, ¹⁴⁹Pm, ¹⁵¹Pm, ¹⁵³Sm, ¹⁵⁷Gd, ¹⁵⁹Gd, ¹⁶⁶Ho, ¹⁷²Tm, ¹⁶⁹Yb, ¹⁷⁵Yb, ¹⁷⁷Lu, , ¹⁰⁵Rh, ^{103m}Rh, ¹¹¹Ag, ¹²⁴I, ¹³¹I, and ²¹¹At, ²¹²Bi, ²²⁵Ac; more preferably from the group consisting of ⁹⁰Y, ¹³¹I, and ¹⁷⁷Lu; and most preferably from the group consisting of ⁹⁰Y and ¹⁷⁷Lu.

Thus, the present invention also refers to a compound of formula (I) as described above, wherein S⁴, R⁵, R⁶ or Xaa¹, particularly R⁵, comprises a radionuclide selected from the group consisting of ^{114m}In, ¹⁸⁶Re, ¹⁸⁸Re, ⁷⁷As, ⁹⁰Y, ⁶⁶Ga, ⁶⁷Cu, ¹⁶⁹Er, ^{117m}Sn, ¹²¹Sn,¹²⁷Te, ¹⁴²Pr, ¹⁴³Pr, ^{195m}Pt_{,} ¹⁹⁸Au, ¹⁹⁹Au, ¹⁴⁹Tb, ¹⁶¹Tb, ¹⁰⁹Pd, ¹⁶⁵Dy, ¹⁴⁹Pm, ¹⁵¹Pm, ¹⁵³Sm, ¹⁵⁷Gd, ¹⁵⁹Gd, ¹⁶⁶Ho, ¹⁷²Tm, ¹⁶⁹Yb, ¹⁷⁵Yb, ¹⁷⁷Lu, ¹⁰⁵Rh, ^{103m}Rh, ¹¹¹Ag, ¹²⁴I, ¹³¹I, and ²¹¹At, ²¹²Bi, ²²⁵Ac; more preferably from the group consisting of ⁹⁰Y, ¹³¹I, and ¹⁷⁷Lu; and most preferably from the group consisting of ⁹⁰Y and ¹⁷⁷Lu.

### The term detectable label:

The term "detectable label" as used herein refers to any label which provides directly or indirectly a detectable signal.

For example, the label may be detectable without the addition of further reagents, such as by means of an output of detectable electromagnetic radiation or other nuclear radiation from the label itself, or as a result of its magnetic or paramagnetic properties. The label may also be detectable upon addition of one or more further reagent(s). A person skilled in the art will readily select said further reagent(s) in dependence of the label.

The detectable label is preferably a moiety being suitable for imaging and/or assaying, for example, for identifying, diagnosing, evaluating, detecting and/or quantitating, in vivo or in vitro, in particular for in vivo or in vitro detection via radioscintigraphy, magnetic resonance imaging (MRI), chemiluminescence, near infrared luminescence, gamma imaging, magnetic resonance spectroscopy, fluorescence spectroscopy, SPECT, computed tomography (CT scan), positron emission tomography (PET) or methods for optical tomography.

Suitable detectable labels include, for example, radiolabels, such as radioisotopes, radionuclides, isotopes, enzymes, enzyme substrates or co-factors, enzyme inhibitors, magnetic or paramagnetic moieties or particles, fluorescent groups, biotin (in conjunction with streptavidin complexation), radiolabels in conjugation with organic complexation agents, photoaffinity groups, or enzymes and substrates for bioluminescent imaging, such as firefly luciferase and L-luciferin as the substrate, or combinations thereof.

"Fluorescent labels" or "fluorescent groups" include, but are not limited to NBD (7-nitro-1,2,3-benzoxadiazole), Texas red, phycoerythrin (PE), Cy5, Cy 5.5, cytochrome c, and fluoresceine isothiocyanate (FITC).

"Magnetic or paramagnetic moieties or particles" include, but are not limited to MR contrast agents, e.g. chelates of paramagnetic, ferromagnetic, or diamagnetic metal ions, or magnetic particles. One specific example for a paramagnetic label is gadolinium (Gd) and chelates thereof.

According to preferred embodiments of the present invention, in case the compound of formula (I) comprises a detectable label, said detectable label is preferably a radiolabel or an organic complexation agent or a combination of a radiolabel and an organic complexation agent thereof. In case the compound of formula (I) comprises a radiolabel, said radiolabel is preferably comprised in Xaa⁴, particularly in S⁴; R⁵, particularly in L; Q, particularly in R⁶; or in Xaa¹, particularly in S¹; and is more preferably comprised in R⁵.

In case the detectable label, comprises or is a radiolabel, said radiolabel is preferably a radionuclide selected from the group consisting of ¹¹C, ¹⁸F, ⁴⁷Sc, ⁵¹Cr, ^{52m}Mn, ⁵⁸Co, ⁵²Fe, ⁵⁶Ni, ⁵⁷Ni, ⁶²Cu, ⁶⁴Cu, ⁶⁷Cu, ⁶⁶Ga, ⁶⁸Ga, ⁶⁷Ga, ⁷²As, ⁷⁷As, ⁷⁵Br, ⁷⁶Br, ⁷⁷Br, ⁸²Br, ⁸⁹Zr, ⁹⁰Y, ^{94m}Tc, ^{99m}Tc, ⁹⁷Ru, ¹⁰⁵Rh, ¹⁰⁹Pd, ¹¹¹Ag, ^{110m}In, ¹¹¹In, ^{113m}In, ^{114m}In, ¹²⁰I, ¹²³I, ¹²⁴I, ¹²⁵I, ¹³¹I, ^{117m}Sn, ¹²¹Sn, ¹²⁷Te, ¹⁴²Pr, ¹⁴³Pr, ¹⁴⁹Pm, ¹⁵¹Pm, ¹⁴⁹Tb, ¹⁵³Sm, ¹⁵⁷Gd, ¹⁶¹Tb, ¹⁶⁶Ho, ¹⁶⁵Dy, ¹⁶⁹Er, ¹⁶⁹Yb, ¹⁷⁵Yb, ¹⁷²Tm, ¹⁷⁷Lu, ¹⁸⁶Re, ¹⁸⁸Re, ¹⁹¹Pt, ¹⁹⁷Hg, ¹⁹⁸Au, ¹⁹⁹Au, ²⁰¹Tl, ²⁰³Pb, ²¹¹At, ²¹²Bi and ²²⁵Ac; and more preferably from the group consisting of ¹⁸F, ⁶⁸Ga, ⁸⁹Zr, ⁹⁰Y, ^{99m}Tc, ^{III}In, ¹²³I, ¹²⁴I, ¹³¹I, ¹⁷⁷Lu; and most preferably from the group consisting of ¹⁸F, ⁶⁸Ga, ^{99m}Tc, ¹¹¹In, ¹²³I, and ¹⁷⁷Lu.

Thus, the present invention also refers to a compound of formula (I) as described above, wherein S⁴, R⁵, R⁶ or Xaa¹, particularly R⁵, comprises (cf. compound D) a radionuclide selected from the group consisting of ¹¹C, ¹⁸F, ⁴⁷Sc, ⁵¹Cr, ^{52m}Mn, ⁵⁸Co, ⁵²Fe, ⁵⁶Ni, ⁵⁷Ni, ⁶²Cu, ⁶⁴Cu, ⁶⁷Cu, ⁶⁶Ga, ⁶⁸Ga, ⁶⁷Ga, ⁷²As, ⁷⁷As, ⁷⁵Br, ⁷⁶Br, ⁷⁷Br, ⁸²Br, ⁸⁹Zr, ⁹⁰Y, ^{94m}Tc, ^{99m}Tc, ⁹⁷Ru, ¹⁰⁵Rh, ¹⁰⁹Pd, ¹¹¹Ag, ^{110m}In, ¹¹¹In, ^{113m}In, ^{114m}In, ¹²⁰I, ¹²³I, ¹²⁴I, ¹²⁵I, ¹³¹I, ^{117m}Sn, ¹²¹Sn, ¹²⁷Te, ¹⁴²Pr, ¹⁴³Pr, ¹⁴⁹Pm, ¹⁵¹Pm, ¹⁴⁹Tb, ¹⁵³Sm, ¹⁵⁷Gd, ¹⁶¹Tb, ¹⁶⁶Ho, ¹⁶⁵Dy, ¹⁶⁹Er, ¹⁶⁹Yb, ¹⁷⁵Yb, ¹⁷²Tm, ¹⁷⁷Lu, ¹⁸⁶Re, ¹⁸⁸Re, ¹⁹¹Pt, ¹⁹⁷Hg, ¹⁹⁸Au, ¹⁹⁹Au, ²⁰¹Tl, ²⁰³Pb, ²¹¹At, ²¹²Bi and ²²⁵Ac; and more preferably from the group consisting of ¹⁸F, ⁶⁸Ga, ⁸⁹Zr, ⁹⁰Y, ^{99m}Tc, ¹¹¹In, ¹²³I, ¹²⁴I, ¹³¹I, ¹⁷⁷Lu; and most preferably from the group consisting of ¹⁸F, ⁶⁸Ga, ^{99m}Tc, ¹¹¹In, ¹²³I, and ¹⁷⁷Lu. In some embodiments, D comprises or is a Ga or In radionuclide, especially selected from ⁶⁶Ga, ⁶⁸Ga, ⁶⁷Ga, ^{110m}In, ¹¹¹In, ^{113m}In, and ^{114m}In. In certain embodiments, D comprises or is a Ga radionuclide, such as ⁶⁶Ga, ⁶⁷Ga, or ⁶⁸Ga, especially ⁶⁸Ga.

"Radionuclide" and "radioisotope" are used interchangeably herein. A "radionuclide" as used herein may be (comprised by) a radiolabel or a cytotoxic moiety.

### The organic complexation agent

The term "organic complexation agent" refers to a chelating agent, preferably, capable of complexing at least one radionuclide.

As regards complexation agents suitable for the present invention, reference is made to WO 2009/109332, pages 9 to 14, and the respective metal chelators disclosed therein as well as to WO 97/31657.

According to a preferred embodiment of the present invention, the organic complexation agent is a chelating agent like NODASA, NODAGA, TETA, TRITA, DTPA, EDTA, CDTA, CPTA, EGTA; HBED, TTHA, DTPA, DOTA, NOTA, HP-DOA3, CBTE2a, TE2A, TMT or DPDP, HYNIC, DFO, or HEDTA. Those chelating agents are well known to those skilled in the art for radiopharmaceuticals and radiodiagnosticals.

CBTE2a stands for bis(carboxymethyl)-1,4,8,11-tetraazabicyclo[6.6.2]hexadecane, CDTA stands for cyclohexyl 1,2-diamine tetraacetic acid, CPTA stand for [4-(1,4,8,11-tetraazacyclotetradec-1-yl)-methyl benzoic acid] hydrochloride, DFO stands for N'-[5-[acetyl(hydroxy)amino]pentyl]-N-[5-[[4-[5-aminopentyl(hydroxy) amino]-4-oxobutanoyl]amino]pentyl]-N-hydroxybutanediamide, DO2A stands for 4,1-bis(carboxymethyl)-1,4,8,11-tetraazabicydo[6.6.2]hexadecane. DOTA stands for 1,4,7,10-tetracyclododecane-N,N',N", N"', tetraacetic acid, DPDP stands for (N,N'-dipyridoxylethylenediamine-N,N'-diacetate-5,5'-bis(phosphate), DTPA stands for dietehylenetriaminepentaacetic acid, EDTA stands for ethylenediamine-N,N'-tetraacetic acid, EGTA stands ethyleneglycol-O,O-bis (2-aminoethyl), N, N, N', N' tetraacetic acid, HBED stands for N, N-bis (hydrocybenzyl)-ethylenediamine-N,N'-diacetic acid, HEDTA stands for hydrixyethylediamine triacetic acid, HP-DOA3 stands for 1-(p-nitrobenzyl)-1,4,710-tetraazacyclodecane-4,7,10-triacetate, HYNIC stands for 6-hydrazinyl-N-methylpyridine-3-carboxamide, NODASA stands for 1,4,7-Triazacyclononane-1-succinic acid-4,7-diacetic acid, NODAGA stands for 1-(1-Carboxy-3-carboxypropyl)-4,7-(carbooxy)-1,4,7-triazacyclononane, NOTA stands for 1,4,7-triazacyclononanetriacetic acid, TE2A stands for 4,11-bis(carboxymethyl)-1,4,8,11-tetraazabicyclo[6.6.2]hexadecane, TETA stands for 1,4,8,11-tetraazacyclododecane-1,4,8,11-tetraecetic acid, TMT stands for terpyridine-bis(methylenaminetetraacetic acid, TRITA stands for 1,4,7,10-tetraazacyclotridecane-N,N',N",N"'-tetraacetic acid, TTHA stands for triethylene tetraamine hexaacetic acid.

According to a further embodiment of the present invention, the organic complexation agent is a macrocyclic chelating agent, for example, a porphyrin-like molecule, a pentaazamacrocycle as described by *Zhang et al., 1998,* a phthalocyanine, a crown ether, e.g. a nitrogen crown ether such as the sepulchrates, or crypates.

According to an alternative embodiment, the organic complexation agent is a NₜS₍₄₋ₜ₎ chelating agents, such as the chelating agents described on page 8 to page 9 in WO 97/31657.

Examples of suitable chelators are further described in the international patent application WO 89/07456, such as unsubstituted or substituted 2-imino-thiolanes and 2-iminothiacyclohexanes, in particular 2-imino-4-mercaptomethylthiolane.

According to a preferred embodiment of the present invention, the organic complexation agent is selected from the group consisting of DOTA, NOTA, TRITA, TETA, DTPA, HYNIC and CBTE2a, more preferably DOTA, NOTA; DTPA, and TETA.

More preferably, the organic complexation agent is selected from the group consisting of DOTA and NOTA and DTPA. Thus, according to one embodiment of the present invention, the organic complexation agent is DOTA. According to another preferred embodiment, the organic complexation agent is DTPA and according to a third preferred embodiment, the complexation agent is NOTA.

Thus, the present invention also relates to a compound, as described above, wherein any one of S⁴, R⁵, R⁶ or Xaa¹, particularly wherein R⁵ comprises (cf. compound D) an organic complexation agent, and wherein the organic complexation agent is selected from the group consisting of like NODASA, NODAGA, TETA, TRITA, DTPA, EDTA, CDTA, CPTA, EGTA; HBED, TTHA, DTPA, DOTA, NOTA, HP-DOA3, CBTE2a, TE2A, TMT, DPDP, HYNIC, DFO and HEDTA, in particular wherein the organic complexation agent is selected from the group consisting of DOTA, NOTA, TRITA, TETA, DTPA, HYNIC and CBTE2a, more preferably DOTA, NOTA, DTPA, and TETA, more preferably DOTA, NOTA and DTPA.

In case a compound of the invention comprises a complexation agent, said complexation agent is preferably covalently bound to the respective residues, such as by being covalently bound to L, R⁵, the C^{alpha} of B, X, R⁶ or S⁴, respectively. Preferably, it is covalently bound to R⁵.

In case of complexation agents such as DOTA, for example, 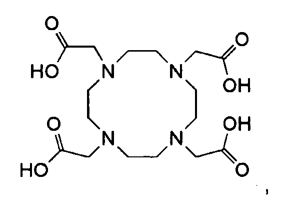 the complexation agent is preferably coupled via one of its carboxy functions.

According to a preferred embodiment, the present invention comprises at least one radiolabel or at least one organic complexation agent or a combination thereof.

In case the compound of formula (I) comprises (cf. compound D) an organic complexation agent or a combination of an organic complexation agent with a radiolabel, said organic complexation agent or said combination of an organic complexation agent with a radiolabel is preferably comprised in R⁵. Thus, the present invention also describes a compound, as described above, wherein R⁵ comprises a radiolabel or an organic complexation agent or a combination thereof. In said case, R⁵ comprises most preferably a combination of a radionuclide and a complexation agent, with the complexation agent being covalently bound, optionally via a linker, to R⁵ or to the backbone C^{alpha} of B and with the radionuclide being complexed by the organic complexation agent. As one example, in case the radionuclide is ⁶⁸Ga, the complexation agent is preferably NOTA or DOTA.

In particularly preferred embodiments, any one of S⁴, R⁵, R⁶ or Xaa¹, particularly R⁵ comprises or consists of (cf. compound D) a combination of an organic complexation agent and a detectable label, particularly a radionuclide. Suitable such combinations are well-known to the skilled person. Certain combinations involve Ga or In. Exemplary combinations are Ga with DOTA, Ga with NODASA, Ga with NODAGA, Ga with DFO or Ga with NOTA. Ga with DOTA is particularly preferred. Specific embodiments include ⁶⁶Ga with DOTA, ⁶⁸Ga with DOTA, ⁶⁷Ga with DOTA, ^{110m}In with DOTA, and ¹¹¹In, DOTA; particularly ⁶⁶Ga with DOTA, ⁶⁸Ga with DOTA, and ⁶⁷Ga with DOTA.

### Pharmaceutically acceptable salt

As described above, the compound of the present invention can be formulated as pharmaceutically acceptable salt. Typical pharmaceutically acceptable salts include those salts prepared by reaction of the compounds of the present invention with a pharmaceutically acceptable mineral or organic acid or an organic or inorganic base. Such salts are known as acid addition and base addition salts. Acids commonly employed to form acid addition salts are inorganic acids such as hydrochloric acid, hydrobromic acid, hydroiodic acid, sulfuric acid, phosphoric acid, and the like, and organic acids such as p-toluenesulfonic acid, methanesulfonic acid, oxalic acid, p-bromophenylsulfonic acid, carbonic acid, succinic acid, citric acid, benzoic acid, acetic acid, and the like. Examples of such pharmaceutically acceptable salts are the sulfate, pyrosulfate, bisulfate, sulfite, bisulfite, phosphate, monohydrogenphosphate, dihydrogenphosphate, metaphosphate, pyrophosphate, bromide, iodide, acetate, propionate, decanoate, caprylate, acrylate, formate, hydrochloride, dihydrochloride, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, hydroxybenzoate, methoxybenzoate, phthalate, xylenesulfonate, phenylacetate, phenylpropionate, phenylbutyrate, citrate, lactate, gamma-hydroxybutyrate, glycolate, tartrate, methanesulfonate, propanesulfonate, naphthalene-1-sulfonate, napththalene-2-sulfonate, mandelate and the like. Preferred pharmaceutically acceptable acid addition salts are those formed with mineral acids such as hydrochloric acid and hydrobromic acid, and those formed with organic acids such as maleic acid and methanesulfonic acid. Salts of amine groups may also comprise quarternary ammonium salts in which the amino nitrogen carries a suitable organic group such as an alkyl, alkenyl, alkynyl, aryl or aralkyl moiety. Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide, calcium carbonate, and the like. The potassium and sodium salt forms are particularly preferred. It should be recognized that the particular counterion forming a part of any salt of this invention is usually not of a critical nature, so long as the salt as a whole is pharmacologically acceptable and as long as the counterion does not contribute undesired qualities to the salt as a whole. The term acceptable salt encompasses also pharmaceutically acceptable solvates of the compounds of the invention, wherein the compound combines with a solvent such as water, methanol, ethanol or acetonitrile to form a pharmaceutically acceptable solvate such as the corresponding hydrate, methanolate, ethanolate or acetonitrilate.

In a further aspect, the present invention relates to a pharmaceutical composition comprising a compound as defined above and at least one pharmaceutically acceptable excipient.

"Pharmaceutically acceptable excipients" are well known in the art as substances other than the active ingredients that may be part of pharmaceutical compositions. Examples for excipients include, but are not limited to one or more carriers, coatings, disintegrants, binders, fillers, diluents, lubricants, stabilizers, surfactants, preservatives, flavouring agents, colouring agents, sorbents, sweeteners, and any combinations thereof.

The pharmaceutical composition may also comprise one or more additional active ingredients known to the skilled person to provide a combination therapy, such as of a disease or disorder described herein.

An exemplary dosage of the compound of the invention may be in the range from about 0.01 to about 1000 mg, such as from about 0.05 to about 500 mg, such as from about 0.1 to about 100 mg, such as from about 0.2 to about 10 mg per kg body weight per day. As used herein, "about" refers to a range around a given value plus/minus 10% thereof. Accordingly, about 10 mg per kg body weight per day refers to 9 to 11 mg per kg body weight per day.

It will be understood that a person skilled in the art can readily determine suitable dosages and administration schemes on the basis of his knowledge, wherein preferred dosages and administration schemes will depend on the condition to be treated.

The pharmaceutical compositions of the invention may be administered by routine methods, for example via oral/peroral, parenteral (preferably intravenous, e.g. by injection), intraperitoneal, intradermal, transdermal, inhalational, topical, nasal, buccal, rectal or vaginal administration routes or via an implanted reservoir. Suitable dosage forms include, but are not limited to capsules, tablets, pellets, aqueous suspensions, aqueous solutions, aerosols, suppositories, creams, gels, ointments and transdermal patches. According to a preferred embodiment, the pharmaceutical composition is administered intravenously. Preferred embodiments involve injection. Other preferred embodiments involve infusion. According to another preferred embodiment, the pharmaceutical composition is administered perorally. Other preferred embodiments involve subcutaneous depots.

The compounds of the invention, which are believed to bind the CXCR4 receptor with high affinity, may be suitable for blocking, disrupting or otherwise interfering with the interaction between the CXCR4 receptor and its natural ligand. Likewise, they may be suitable for targeting cytotoxic moieties or the like to CXCR4 receptors.

Therefore, the compounds and compositions of the invention may be used in methods of treating CXCR4 receptor-related conditions, disorders and diseases.

Accordingly, in a further aspect, the present invention relates to a compound as defined above for use as a medicament.

In a further aspect, the present invention relates to a compound as defined above or composition as defined above for use in a method for the prevention or treatment of a CXCR4 receptor-related disease or disorder.

In addition, the invention relates to the use of a compound as defined above or composition as defined above for the manufacture of a medicament for preventing a CXCR4 receptor-related disease or disorder, as well as to the use of a compound or composition as defined above for the manufacture of a medicament for treating a CXCR4 receptor-related disease or disorder.

In addition, the invention relates to a method of preventing a CXCR4 receptor-related disease or disorder, the method comprising a step of administering a compound as defined above or composition as defined above to a subject in need thereof, as well as to a method of treating a CXCR4 receptor-related disease or disorder, the method comprising a step of administering a compound as defined above or composition as defined above to a subject in need thereof.

"CXCR4" or "CXCR4 receptor" as used herein, refers to a particular receptor, the CXC chemokine receptor 4, which well-known to the skilled person, and which is also called "fusin". It is e.g. expressed on many stem cells, but also in numerous cancers.

Term "CXCR4" or "CXCR4 receptor" as used herein also includes variants thereof. Variants particularly include isoforms encoded by alternative transcriptional splice variants, as well as mutated or truncated forms of said receptor. Preferably the gene or protein sequences of a variant CXCR4 has at least 10%, 20%, 30%, 40%, 50%, 60%, 70%, particularly at least 80% or 90%, more particularly at least 95% or 97%, especially at least 99% sequence identity to a native sequence of CXCR4. As used herein, a gene or protein is said to have "X % sequence identity" to a given sequence if upon an alignment with the best matching sequence of said given sequence the number of matching nucleotides or amino acids, respectively, divided by the number of nucleotides or amino acids of the shorter sequence multiplied by 100 is X. Methods and tools of aligning sequences are well known to the skilled person.

Mutations of a CXCR4 encoding sequence leading to truncated forms of the CXCR4 receptor may e.g. influence the extent of an inflammatory reaction or the metastatic potential of a cancer. Besides, mutations in the CXCR4 gene have been shown to be associated with e.g. WHIM (warts, hypogammaglobulinemia, infections, and myelokathexis) syndrome.

A "CXCR4 receptor-related disease or disorder" as used herein includes any pathological condition, a disease or a disorder, which is directly or indirectly related to the CXCR4 receptor per se or to its function, such as to the interaction of the CXCR4 receptor with its natural ligand CXCL12 (SDF-1). CXCR4 receptor-related disease or disorder particularly includes any disease or disorder that is related directly, indirectly, immediately and/or not immediately to the CXCR4 receptor, the CXCR4 receptor status or CXCR4 receptor signaling. Numerous CXCR4 receptor-related diseases or disorders are known in the art (cf. e.g. *Taniuchi et al., 2005; Kim et al., 2005; Phillips et al., 2003*).

As to the prevention or treatment of a CXCR4 receptor-related disease or disorder, said CXCR4 receptor-related disease or disorder e.g. also includes any disease or disorder involving cells expressing the CXCR4 receptor, such as cancer cells. It may also be a disease or disorder caused or promoted by a cellular pathway such as a signaling pathway involving the CXCR4 receptor. It may also be a disease or disorder caused or promoted by an altered expression such as an overexpression of the CXCR4 receptor and/or by a modification of the CXCR4 receptor.

Particularly as to aspects of the invention that involve imaging or monitoring of CXCR4 receptors, a CXCR4 receptor-related disease or disorder may be any disease or disorder involving any alteration in the status of the CXCR4 receptor, e.g. an altered expression such as an overexpression or decreased expression of the CXCR4 receptor. Likewise, also any CXCR4 receptor-related disease or disorder is envisaged, the therapy or treatment of which is directly or indirectly related to an alteration in the status of the CXCR4 receptor, e.g. an altered expression such as an overexpression or decreased expression of the CXCR4 receptor.

As one non-limiting example in this respect, reference is made to Her2/neu expression in mammacarcinoma, where Her2/neu stabilizes the CXCR4 receptor status, and where an antibody therapy towards Her2/neu leads to a destabilization the CXCR4 receptor status and finally a decreased CXCR4 expression.

As referred to herein, the CXCR4 receptor-related disease or disorder may be a neoplastic condition, an immune disease, an autoimmune disease, a vascular disease, an inflammatory condition and/or a neurological disease.

A "neoplastic condition" as used herein refers to a condition characterized by an increase in mass of a tissue resulting in a neoplasm. Said neoplasm results from a neoplasia, i.e. the proliferation of cells. Preferably, according to the invention, such neoplastic condition relates to an abnormal mass of a tissue, such as a tumor, particularly a malignant tumor. Accordingly, in a preferred embodiment the neoplastic condition is a cancer.

"Immune diseases" are diseases involving a dysfunction of the immune system, such as by an overactive or insufficiently active immune system, which diseases may be congenital or acquired and may affect various components of the immune system.

"Autoimmune diseases" are particular immune diseases, which are known within the art as diseases arising from an overly active immune response of the body against substances and tissues that are normally present in the body. They include, but are not limited to multiple sclerosis (MS), lupus erythematosus, Sjögren's syndrome, ulcerative colitis and rheumatoid arthritis.

"Vascular diseases" are known to the skilled person as diseases primarily affecting the blood vessels. They include e.g. atherosclerosis, hypertonic diseases and thrombosis. Vascular diseases may involve an inadequate ratio of oxygen need to oxygen supply.

"Inflammatory conditions" as used herein comprise diseases or disorders associated with inflammation which include, but are not limited to atherosclerosis, rheumatoid arthritis, vasculitis and asthma. Preferably, the inflammatory condition is a vascular inflammatory condition such as atherosclerosis or a disease related to atherosclerosis such as coronary heart disease (CHD).

"Neurological diseases" include diseases and disorders that can affect the central nervous system, the peripheral nervous system, or the autonomic nervous system. They include but are not limited to multiple sclerosis (MS) and Alzheimer's disease.

In a preferred embodiment of the invention, the CXCR4 receptor-related disease or disorder is any one selected from HIV infection, cancer, rheumatoid arthritis, multiple sclerosis, atherosclerosis and asthma. In one particular embodiment, the CXCR4 receptor-related disease or disorder is atherosclerosis. In another particular embodiment, the CXCR4 receptor-related disease or disorder is a leukaemia, particularly chronic lymphocytic B-cell leukaemia (B-CLL). In another particular embodiment, the CXCR4 receptor-related disease or disorder is pain or involves pain.

In another embodiment of the invention, the CXCR4 receptor-related disease or disorder is selected from any of the diseases and disorders referred to on pages 49 to 58 of WO 2008/08854A, which is specifically incorporated herein by reference.

According to a particular preferred embodiment, the CXCR4 receptor-related disease or disorder is a cancer. The term "cancer" as used herein particularly includes carcinomas, sarcomas, melanomas, blastomas and lymphomas. The term "cancer" may refer to a cancer not including metastases, a cancer including metastases, or to cancer metastases. Hence, it may refer to primary tumors with or without at least one metastasis, or solely refer to cancer metastasis/metastases. Preferably, the cancer and/or cancer metastases expresses the CXCR4 receptor. Accordingly, "cancer metastasis" or "cancer metastases" per se is/are another preferred example for a CXCR4 receptor-related disease or disorder.

In one embodiment of the invention, the cancer is selected from the group consisting of astrocytoma, B-cell lymphoma, breast adenocarcinoma, breast carcinoma, cervical adenocarcinoma, colon adenocarcinoma, colorectal adenocarcinoma, colorectal carcinoma, glioblastoma, hepatocellular carcinoma, hepatoma and pancreatic carcinoma, leukaemia, large cell lung cancer, lung adenocarcinoma, lung carcinoma, lung mesothelioma, lung squamous cell carcinoma, melanoma, neuroblastoma, non small cell and small cell lung cancer (NSCLC and SCLC), ovarian adenocarcinoma, pancreatic adenocarcinoma, pancreatic carcinoma, prostate adenocarcinoma, prostate carcinoma, rectal adenocarcinoma, and renal cell adenocarcinoma.

In another embodiment, the cancer is selected from the group consisting of breast adenocarcinomas, Burkitt's B-cell lymphoma, cervical adenocarcinomas, colon and rectal adenocarcinomas, oesophageal cancer, gliomas, glioblastomas, hepatocellular carcinomas (malignant hepatomas), hepatomas, leukaemia, mamma-carcinomas, melanoma, neuroblastoma, nasopharyngeal adenocarcinoma, non small cell lung cancer (NSCLC), pancreatic adenocarcinomas, prostate cancer, small cell lung cancer (SCLC), T-cell lymphoma, and thyroid cancer.

In a preferred embodiment, the cancer is selected from the group consisting of breast adenocarcinoma, colorectal adenocarcinoma, lymphoma, melanoma carcinoma, prostate carcinoma, prostate adenocarcinoma, small cell lung cancer.

The CXCR4 receptor-related diseases and disorders described herein may be treated by a compound of the invention by administering it to a subject in need thereof. The compound may be administered in form of a pharmaceutical composition as described hereinabove. It may be administered by any known administration route including the ones described hereinabove. In a preferred embodiment, the compound is formulated as pharmaceutically acceptable salt as described hereinabove.

A "subject" as used herein may be an animal or human subject. In preferred embodiments, the subject is a mammalian subject, more preferably a human subject. In one embodiment, the subject is a human subject having a neoplasia such as a cancer or suspected of having a neoplasia such as a cancer, wherein the cancer may or may not involve metastases.

Without the intention of being bound by theory, the present inventors consider that cancer metastasis may be caused by circulating cancer cells expressing CXCR4 that are targeted to sites that attract CXCR4-expressing cells such as stem cells, e.g. to the lungs, liver and bone marrow. CXCR4 overexpression has been shown on numerous tumors. CXCR4 expression on cancer cells may particularly be increased under hypoxic conditions. Accordingly, in a particular embodiment, the invention relates to a compound of the invention for use in reducing, preferably preventing, cancer metastases. In another embodiment, the invention relates to a method of reducing, preferably preventing metastases, the method comprising administering a compound of the present invention to a subject in need thereof, particularly a subject having cancer or suspecting of having cancer.

When used in preventing or treating a CXCR4 receptor-related disease or disorder such as a cancer, the compounds of the invention may or may not include one or more cytotoxic moieties. In one embodiment, the compounds include one or more cytotoxic moieties for targeted chemotherapy of CXCR4-positive tumors, such as CXCR4-expressing cancer.

Examples for "cytotoxic moieties" are well-known within the art and include radionuclides as described herein and chemotherapeutical agents. Chemotherapeutical agents include, but are not limited to bleomycin, carboplatin, cisplatin, cyclophosphamide, chlorambucil, docetaxel, doxorubicin, etoposide, methotrexate, mitoxantrone, paclitaxel, prednisone, teniposide, valrubicin, vinblastine, vincristine, vindesine, vinorelbine. Preferred cytotoxic moieties may be selected from any of those cytotoxic compounds generally used for chemotherapy of the tumor concerned.

In addition, the compounds of the invention, which are thought to bind the CXCR4 receptor with high affinity, may be particularly suitable for use in any type of imaging applications and/or any applications involving the labelling of CXCR4 receptor(s).

Accordingly, in a further aspect, the invention relates to the use of a compound as defined above, wherein the compound comprises a detectable label, for the imaging of CXCR4 receptors, in particular for medical imaging, especially for diagnostic imaging.

The imaging may be any one of *in vivo*-imaging, *ex vivo*-imaging, and *in vitro*-imaging.

Likewise, in another aspect, the invention relates to a method of imaging CXCR4 receptors, in particular of medical imaging, especially of diagnostic imaging, the method comprising administering a compound as defined above or composition as defined above to a sample or a subject, wherein the compound comprises a detectable label.

Said method may be any one of an *in vivo-* method, an *ex vivo-* method, and an *in vitro-*method. Preferably, said method is neither a method for treatment of the human or animal body by surgery or therapy nor a diagnostic method practiced on the human or animal body. Generally, in certain embodiments, a method of the invention does not comprise, preferably is not, a method for treatment of the human or animal body by surgery or therapy or a diagnostic method practiced on the human or animal body.

"Imaging" is well known to the skilled person. Non-limiting suitable imaging techniques and methods are e.g. described in *Weissleder R et al, 2008, Shah K et al, 2005, Weissleder R et al, 2003* and *Kuehl H et al, 2007.*

As used herein, imaging preferably relates to "biological imaging" and/or "molecular imaging", particularly to "medical imaging", and especially to "diagnostic imaging".

"Biological imaging" as used herein generally refers to any imaging used in biology or medicine, particularly to imaging for examining biological material such as a biological sample or a biological subject or part thereof.

"Molecular imaging" is well known in the art (cf. e.g. *Shah K et al, 2005*) and includes imaging any type of molecular and/or cellular processes, e.g. with the aim of monitoring gene expression, simultaneous molecular events, progression or regression of a disease such as cancer.

"Medical imaging", which is generally well-known within the art, concerns imaging for medical purposes. It preferably includes creating images of a sample derived from a subject, or of a subject or part of a subject. Medical imaging may be performed to reveal, diagnose or examine a disease or disorder, preferably a CXCR4 receptor-related disease or disorder such as any of the ones described hereinabove.

"Diagnostic imaging" as used herein refers to imaging for diagnostic purposes, such as for diagnosing a disease or disorder, preferably a CXCR4 receptor-related disease or disorder such as any of the ones described hereinabove.

A method of diagnostic imaging may or may not be a diagnostic method practised on the human or animal body.

A "sample" may be any sample. Non-limiting examples for a sample are cells, tissue section(s), tissue(s), and organ(s).

Preferably, the sample is derived from a subject, particularly from a human subject.

According to the invention, imaging may be carried out on any sample or subject or part of a subject comprising CXCR4 receptors.

According to the invention, imaging may involve any kind of imaging techniques known to the skilled person, wherein said techniques include, but are not limited to positron emission tomography (PET), single photon emission computed tomography (SPECT), magnetic resonance imaging (MRI), tomography such as computed tomography (CT), imaging via gamma cameras, imaging via autoradiography, imaging via phosphor imagers, and any combination(s) thereof.

Preferably, imaging occurs via any of positron emission tomography (PET), single photon emission computed tomography (SPECT), and magnetic resonance imaging (MRI). As will be understood by the skilled person, particularly preferred embodiments for such techniques depend on the respective detectable label used. Generally, when the label is a radionuclide, the detection step may preferably be performed using positron emission tomography (PET) or single photon emission computed tomography (SPECT). Magnetic resonance imaging (MRI) is preferred when magnetic or paramagnetic labels, such as a gadolinium label, are employed. Detectable labels for use with the compounds of the present invention are described hereinabove. In one embodiment, the detectable label is a fluorescent label. In one embodiment, the detectable label allows simultaneous imaging, such as dual PET/CT or PET/MRI. In this case, CT or MRI are preferably employed to analyze the morphology of the sample, subject, or part thereof, which is subjected to imaging.

Imaging may be carried out to determine the distribution or the accumulation of the detectable label, preferably via commonly used methods, such as autoradiography or phosphor imagers. Imaging may e.g. be carried out to obtain relative or quantitative distribution or accumulation data.

In an exemplary embodiment, (a method of) *in vitro-* or *ex vivo*-imaging involves the following steps: A compound of the invention comprising a detectable label is contacted with a sample such as cells, tissue section(s), tissue(s) or organ(s). The compound is preferably dissolved in a suitable buffer and said sample is incubated with this buffer. Incubation may occur for any suitable period of time such as in the range of seconds, minutes or hours. Subsequently, the detectable label is detected. This is effected by a suitable device, the nature of which depends on the imaging method used. Preferably or alternatively, in a further step, one or more images are obtained. This may e.g. be by direct imaging or imaging of slices of the incubated tissues.

In an exemplary embodiment, (a method of) *in vivo*-imaging involves the following steps: A compound of the invention comprising a detectable label is introduced into the living organism e.g. by injection, and, subsequently, the detectable label is detected. This is effected by a suitable device, the nature of which depends on the imaging method used. Preferably or alternatively, in a further step, one or more images are obtained. The acquisition of the imaging data such as the emission data is carried out over a suitable period of time such as for minutes to hours. Using commonly employed equipment and suitable software packages, these data may e.g. result in planar or 3D distribution pattern of the activity distribution in the organism. Depending on the method, the obtained data can be qualitative or quantitative.

In an exemplary embodiment, (a method of) *in vivo*-imaging involves the following steps: a) positioning a subject in an imaging device, b) delivering a compound of the invention to the subject, c) acquiring at least one image; or a) delivering a compound of the invention to the subject, b) positioning a subject in an imaging device, c) acquiring at least one image.

In preferred embodiments, a method of imaging does not involve a step of treatment of the human or animal body by surgery or therapy. Yet, certain aspects of the invention explicitly concern a compound or composition of the invention for use in a method of imaging CXCR4 receptors, in particular of medical imaging, especially of diagnostic imaging, wherein said method involves at least one step of treatment of the human or animal body by surgery or therapy.

Preferably, any of the (methods of) imaging referred to herein is employed for the imaging of CXCR4 receptors on stem cells, tumor stem cells, hematopoietic stem cells and other progenitor cells and tissues under remodeling and repair by stem and progenitor cell adhesion.

"Tissues under remodeling and repair by stem and progenitor cell adhesion" as used herein relates to, without being limited thereto, cells or tissues such as cells or tissues engaged in a neoangiogenic process, cells or tissues affected by vascular injury and cells or tissues affected by myocardial infarction.

Preferably, *in vivo*-imaging of CXCR4 expression using compounds of the present invention that are radiolabeled with appropriate radionuclides is effected via PET or SPECT. In *in vivo*-imaging, images of the subject may be taken after a short time after administration, by which stage any tissues having a relatively high expression of CXCR4 will show a relative concentration of the compound of the invention.

In case of *in vivo*-methods for imaging, the compound preferably comprises a radiolabel and the imaging is preferably performed using PET or SPECT. Preferred embodiments concern *in vivo*-methods for the diagnostic imaging of a neoplastic condition.

In case of *in vitro*-methods for imaging, the compound preferably comprises a radiolabel or fluorescent label and the imaging is preferably performed using autoradiography or fluorescence. Preferred embodiments concern *in vitro*-methods for the diagnostic imaging of a neoplastic condition.

In preferred embodiments, the invention relates to the imaging of tumors such as cancer. As described above, cancer may refer to a cancer not including metastases, to a cancer including metastases, or to cancer metastases.

In one embodiment, the cancer is selected from the group consisting of astrocytoma, B-cell lymphoma, breast adenocarcinoma, breast carcinoma, cervical adenocarcinoma, colon adenocarcinoma, colorectal adenocarcinoma, colorectal carcinoma, glioblastoma, hepatocellular carcinoma, hepatoma and pancreatic carcinoma, leukaemia, large cell lung cancer, lung adenocarcinoma, lung carcinoma, lung mesothelioma, lung squamous cell carcinoma, melanoma, neuroblastoma, non small cell and small cell lung cancer (NSCLC and SCLC), ovarian adenocarcinoma, pancreatic adenocarcinoma, pancreatic carcinoma, prostate adenocarcinoma, prostate carcinoma, rectal adenocarcinoma, and renal cell adenocarcinoma.

In another embodiment, the cancer is selected from the group consisting of breast adenocarcinomas, Burkitt's B-cell lymphoma, cervical adenocarcinomas, colon and rectal adenocarcinomas, oesophageal cancer, gliomas, glioblastomas, hepatocellular carcinomas (malignant hepatomas), hepatomas, leukaemia, mamma-carcinomas, melanoma, neuroblastoma, nasopharyngeal adenocarcinoma, non small cell lung cancer (NSCLC), pancreatic adenocarcinomas, prostate cancer, small cell lung cancer (SCLC), T-cell lymphoma, and thyroid cancer.

In a preferred embodiment, the cancer is selected from the group consisting of breast adenocarcinoma, colorectal adenocarcinoma, lymphoma, melanoma carcinoma, prostate carcinoma, prostate adenocarcinoma, small cell lung cancer.

Preferably, the imaging allows a clear delineation of CXCR4 positive tumors (including or not including any metastases), e.g. *in vivo.* Imaging according to the invention may provide tools for the diagnosis of cancer, the detection of tumors and/or tumor metastases, the investigation of tumors and/or tumor metastases, the removal of tumors and/or tumor metastases via surgery, and the like.

In one embodiment of the invention, the imaging is employed for diagnosing or investigating any CXCR4 receptor-related disease or disorder, such as the ones referred to herein.

In one particular embodiment, said disease or disorder is selected from any of the diseases and disorders referred to on pages 49 to 58 of WO2008/08854A, which is specifically incorporated herein by reference.

In one preferred embodiment, imaging is employed for diagnosing or investigating an immune disease, an autoimmune disease, an inflammatory condition and/or a neurological disease, preferred examples thereof being as described above.

In other preferred embodiments, (a method of) imaging is employed for monitoring any CXCR4 receptor-related disease or disorder, such as the ones referred to herein. In particular embodiments, (a method of) imaging is employed for monitoring for investigating inflammatory processes or angiogenic processes.

Accordingly, in a further aspect, the invention relates to a method for monitoring a CXCR4 receptor-related disease or disorder. The invention also relates to a compound or composition of the invention for use in monitoring a CXCR4 receptor-related disease or disorder. The invention also relates to a compound or composition of the invention for use in a method of monitoring a CXCR4 receptor-related disease or disorder.

Using a compound of the invention in imaging as described herein, or employing a method of imaging as described herein, is preferably done to determine the localization, the extent and/or the kinetics of a disease or disorder. In the case of analyzing kinetics, the extent of the disease is preferably analyzed early during therapy in order to be able to quickly detect a response to therapy.

The compounds of the invention are expected to allow early response monitoring as well as the selection of patients that may especially benefit from a planned therapy. A selection of patients means that patients are selected before commencing any therapy, for which disease it is know that the density of CXCR4 receptors correlates with the reaction to therapy or with the expected response. The therapy must not necessarily be a therapy directed to CXCR4, such as by employing a compound of the invention as an antagonist, but can be directed to any target structure which correlates with CXCR4 receptor density.

One exemplary embodiment relates to the qualitative or quantitative imaging of the CXCR4 receptor status *in vivo* using compounds of the invention comprising a detectable label, e.g. by means of PET using the aforementioned compounds, for planning of individualized therapies that directly address (are mediated by) the CXCR4 receptor status or indirectly affect or modulate the CXCR4 receptor status.

Non-limiting examples in this respect relate to the therapy of mammacarcinomas by means of anti-Her2/neu antibodies (e.g. with trastuzumab, trade name Herceptin®) and the anti-angiogenetic antibody therapy of colon carconmas using anti-VEGF-A antibody bevacizumab (trade name Avastin®)

In another embodiment, a compound of the invention is employed to follow or monitor the efficiency of such therapies by therapy response evaluation, preferably early therapy response evaluation. For this purpose the compounds of interest may be injected prior to or early after beginning of such therapies to evaluate response to therapy via comparison of the signal, such as the CXCR4-PET signal, prior to (baseline scan) as well as early after or during therapy.

Other preferred embodiments relate to the use of a compound of the invention for imaging inflammatory processes, preferably *in vivo,* particularly via PET imaging, and to a corresponding method.

Other preferred embodiments relates to the use of a compound of the invention for imaging angiogenetic processes / angiogenesis processes as well as to a corresponding method.

A further aspect of the invention relates to the use of a CXCR4 receptor ligand, preferably a compound as defined hereinabove, to monitor the extent of stem cell depletion, such as during an endoradiotherapeutic approach. Preferably, the use alternatively or further includes monitoring an increasing pool of stem cells in the bone marrow, such as after stem cell transplantation.

One embodiment concerns a method of monitoring the extent of stem cell depletion during an endoradiotherapeutic approach, the method comprising administering a CXCR4 receptor ligand, preferably a compound as defined hereinabove, to a sample or a subject. Preferably, the method includes a subsequent step of monitoring the increasing pool of stem cells in the bone marrow after stem cell transplantation.

Said CXCR4 receptor ligand may be any known molecule that specifically binds to the CXCR4 receptor. Examples include but are not limited to FC 131 referred to above and its derivatives, particularly i) the ligands disclosed in WO 2007/096662 A2, ii) the ligands disclosed in WO 2009/027706 A2. Preferably, the CXCR4 receptor ligand is any of the compounds described hereinabove. Preferably, said ligand comprises a detectable label, particularly the ones described herein.

In one embodiment, the invention relates to the use of a compound of the invention for the diagnostic imaging of changes of the status of CXCR4 expressing cells, particularly stem cells, in the bone marrow.

In a further aspect, the present invention relates to a compound as defined above or composition as defined above for use in a diagnostic method practiced on the human or animal body for the diagnosis of a CXCR4 receptor-related disease or disorder.

Such diagnostic method includes technical steps that are constitutive for making the diagnosis, wherein specific interactions with the human or animal body occur when carrying out these steps, as well as the diagnosis for curative purposes. Said diagnosis *stricto sensu* represents the deductive medical decision phase as a purely intellectual exercise.

In one embodiment, such diagnostic method comprises the steps of administering a compound of the invention to a subject, collecting imaging data from the subject, comparing the imaging data with standard values, finding a symptom of a CXCR4 receptor-related disease or disorder during the comparison, attributing the symptom to a particular clinical picture to establish the diagnosis.

Moreover, the invention relates to a method of diagnosing a CXCR4 receptor-related disease or disorder, wherein the method comprises a step of administering a compound as defined above or composition as defined above to a sample derived from a subject or to a subject, and a subsequent step of imaging CXCR4 receptors.

Preferably, the compound comprises a detectable label, and the method comprises a step of administering a compound as defined above to a sample derived from a subject or to a subject, and particularly comprises a subsequent step of detecting the detectable label and/or a subsequent step of obtaining one or more images.

Due to their features, the compounds of the invention may be suitably used for various other applications directly or indirectly related to the CXCR4 receptor. Accordingly, in even further aspects, the present invention relates to any of the following embodiments:
Use of a compound of the invention, particularly wherein the compound comprises a detectable label, in the visualization of CXCR4 receptor and CXCR4 receptor containing tissue.

Use of a compound of the invention for affinity purification of CXCR4 receptors or cells containing one or more CXCR4 receptors.

Use of a compound of the invention for the diagnostic imaging of changes of CXCR4 receptor expression of cells, preferably stem cells, particularly cells in the bone marrow.

A method of affinity purification of CXCR4 receptors or cells containing one or more CXCR4 receptors, comprising a step of contacting a compound of the invention with a sample containing a CXCR4 receptor or cells containing one or more CXCR4 receptors, particularly wherein the method further comprises one or more steps of removing other constituents from the sample to increase purity of the CXCR4 receptor or cells containing one or more CXCR4 receptors.

The CXCR4 ligands of the present invention may be modified with additional (functional) moieties and/or moieties that immobilize the CXCR4 ligands.

A method of determining the metastatic potential of cells of a neoplasia, the method comprising exposing said cells to a compound of the invention, so as to allow the compound to bind to CXCR4 receptors on the surface of the cells, and determining the presence and/or amount of compound bound to the cells. In this method, an increased number of CXCR4 receptors correlates to an increased metastatic potential of the primary tumor. Accordingly, conclusions as to the metastatic potential of cells of a neoplasia may be drawn from the presence and/or amount of a compound described above bound to the cells via CXCR4 receptors. According to the current knowledge of the inventors, the compounds of the invention may allow for imaging of the metastatic potential of primary tumors of e.g. a cancer selected from the group consisting breast adenocarcinomas, Burkitt's B-cell lymphoma, cervical adenocarcinomas, colon and rectal adenocarcinomas, oesophageal cancer, gliomas, glioblastomas, hepatocellular carcinomas (malignant hepatomas), hepatomas, leukaemia, mamma-carcinomas, melanoma, neuroblastoma, nasopharyngeal adenocarcinoma, non small cell lung cancer (NSCLC), pancreatic adenocarcinomas, prostate cancer, small cell lung cancer (SCLC), T-cell lymphoma, and thyroid cancer. Said method of determining the metastatic potential of cells may be carried out *in vivo* or *in vitro* (i.e. using a sample of cells or tissue removed from a patient). When the compound of the invention comprises a radionuclide, the imaging, or the determination of the presence and/or amount of bound compound, may in particular be performed using PET or SPECT. When magnetic or paramagnetic labels are employed, magnetic resonance imaging is preferred.

The compounds described hereinabove may also be suitably employed in the field of stem cell mobilization and/or transplantation. As it is known in the art (cf. *Levesque et al., 2008),* hematopoietic stem cells (HSCs), which normally reside in the bone marrow, can be forced into the blood by mobilization, which is used clinically to harvest large numbers of HSCs for transplantation. One example for a suitable compound is plerixafor described above. The mobilization of hematopoietic stem cells from the bone marrow to the bloodstream makes use of the interaction between the chemokine SDF-1/CXCL12 and its receptor CXCR4, which serves to retain HSCs within the bone marrow. For mobilization, this interaction may be disturbed by molecules acting as CXCR4 ligands, which is why the compounds of the present invention, e.g. as direct antagonists of the interaction between SDF-1 and CXCR4, may be suitable for inducing mobilization of stem cells. Therefore, it is envisaged that the compounds of the present invention may function as such mobilizing agents and may be used for improving the stem-cell harvest from bone-marrow donors and shorten the collection time as compared to originally employed methods.

Accordingly, in a further aspect, the invention relates to the use of a compound described hereinabove for mobilizing and/or harvesting stem cells. The invention further relates to a compound as defined hereinabove for use in a method of mobilizing and/or harvesting stem cells.

The invention also relates to a method of mobilizing stem cells in a subject, the method comprising a step of administering a compound of the present invention to a sample containing stem cells or to a subject. Also envisaged is a method of harvesting stem cells, the method comprising a step of administering a compound of the present invention to a sample containing stem cells or to a subject and a subsequent step of collecting stem cells from said sample or subject. Preferably, the latter methods are no methods for the treatment of the human or animal body by surgery or therapy.

One exemplary application for stem cell mobilization is its use during cancer therapy, such as by radioimmunotherapeutic treatment, e.g. of lymphomas by means of anti-CD20 radiolabeled antibodies. To overcome the side effects of such therapies, i.e. complete stem cell (bone marrow) depletion, stem cell transplantations are carried out. Prior to the radiotherapeutic approach, stem cells are mobilized from their niches in the bone marrow, are collected from the blood, stored and re-injected after the therapy. Since CXCR4 ligands have been described to mobilize stem cell from the bone marrow niches, the compounds of the invention may be valuable compounds for this therapeutic approach.

Thus, in a further aspect, the invention relates to a compound as defined above or composition as defined above for use in a method of stem cell transplantation comprising the following steps
i) administering a compound of the present invention to a subject,
ii) collecting stem cells from said subject,
iii) optionally storing the collected stem cells, and
iv) re-introducing the collected stem cells into the subject.

Further uses and methods in which the compounds of the invention may be employed will be readily apparent to a person skilled in the art based on the disclosures herein.

### Examples:

The following abbreviations are used:
DCM = dichloromethane, RP-HPLC = reversed phase high pressure liquid chromatography, TFA = trifluoroacetic acid, NMR = nuclear magnetic resonance, THF = tetrahydrofurane, EtOAc = ethyl acetate, RT = room temperature, Boc = tert-butyloxycarbonyl, DIEA = diisopropylethylamine, MeOH = methanol, NMP = *N*-methyl-pyrollidone, DPPA = diphenylphosphoryl azide, DMF = *N*,*N*-dimethylformamide. Ac = acetate, Fmoc = fluorenylmethyloxycarbonyl, Alloc = allyloxycarbonyl, Xaa = undefined amino acid, Dde = 1-(4,4-dimethyl-2,6-dioxocyclohexylidene)ethyl, SPPS = solid phase peptide synthesis, Nal = L-3-(2-naphthyl)alanine, R(Pbf) = arginine with Pbf protected side chain, Tyr(tBu) = tyrosine with tBu protected side chain, Fmoc-Tyr(tBu) = tyrosine with tBu protected side chain and Fmoc protected *N*^{α}

### 1. General description of synthetic pathways:

All commercially available chemical reagents were used without further purification. Technical solvents were distilled before use.

Trityl resins were purchased from *PepChem* and amino acid derivatives from *Iris Biotech GmbH, NovaBiochem, Merck, Bachem, Neosystem, Aldrich,* while all other chemicals were bought from *Aldrich, Fluka* und *Merck* if not stated otherwise.

NMP was obtained from *BASF* and used without further destillation. Dry solvents were purchased from *Aldrich, Fluka* and *Merck.* Dry dichloromethane was distilled from calciumhydride under argon and kept over 4 Å molecular sieve. Water for RP-HPLC was filtered through a 0,22 µm filter (Millipore, Millipak40).

RP-HPLC analyses were performed using an Omnicrom YMC column (4.6 mm × 250 mm, 5 µm C₁₈, 1 mL/min). The eluent was a linear gradient from water (0.1% TFA) to acetonitrile (0.1 % TFA) over 30 minutes and detection at 220 nm and 254 nm. The retention time (*Rₜ*) of the analytical RP-HPLC is given in minutes with the gradient in percentage of acetonitrile. Purities were determined at 220 nm with the Unicorn software package and are given relative to their starting compound.

Semi-preparative RP-HPLC was done on a *Beckman* System Gold equipped with high pressure module 125, UV-detector 166, and using an Omnicrom ODS-A C18 (120 Å, 5 µm, 250 mm × 20 mm) column in combination with the same solvents as stated above.

NMR spectra were recorded on a Bruker Avance 250 or Bruker DMX 500 at 298K. The chemical shifts are reported in ppm on the δ scale relative to the solvent signal. ¹³C-NMR-spectra were recorded using ¹H-broad band decoupling. Pulse programs were taken from the Bruker library or written by members of our group. Samples were prepared in tubes with a diameter of 5 mm using 0.5 ml of deuterated solvent with a final concentration of approximately 20-50 mM. The resulting spectra were processed on a PC workstation using Bruker TOPSPIN 1.3 and MestRe Nova software.

ESI mass spectra were recorded on a Finnigan LCQ in combination with an Agilent/HP 1100 RP-HPLC system using a *Omnicrom YMC* ODS-A C18 column (120 Å, 3 µm, 125 mm × 2 mm) with a flow rate of 0.2 mL/min. The eluent was a linear gradient from water to acetonitrile with 0.1 % formic acid over 20 min with detection at 220 nm.

**Building blocks for solid phase peptide synthesis (SPPS).** *N*^{α}-*o*-nitrobenzoesulfonyl- D-alanine (*N*^{α}-Ns-D-Ala) was synthesized starting from *N*^{α}-H-D-Ala by Ns protection. 1,4,7-tris(*tert*-butoxycarbonylmethyl)-1,4,7,10-tetraazacyclododecane-10-acetic acid (tris(t-Bu)DOTA) was synthesized according to *Mizukami et al., 2008.*

Aminoalcohols were Boc protected prior to alkylation and 7-aminoheptan-1-ol was synthesized according to procedures described in the following literature: *Forbes et al., 2006; Nilsen et al., 2007; Chong et al., 2000.*

For on-resin modification of the *N*-alkyl residue Dde-6-aminoalcohol was synthesized according to *Demmer et al., 2008.*

**Peptide synthesis.** Standard Fmoc strategy with acid labile side chain protecting groups (t-Bu for Tyr and 2,2,4,6,7- pentamethyldihydrobenzofurane-5-sulfonyl (Pbf) for Arg) was employed to construct peptides on tritylchloridpolystyrene (TCP) Resin. Standard peptide bonds were built by coupling with O-(benzotriazol-1-yl)-*N*,*N*,*N*;*N*'-tetramethyluronium tetrafluoroborate (TBTU) and addition of *N*-hydroxybenzotriazole (HOBt) to suppress racemization. *N*-alkylated amines were acylated using 2-(7-aza-1H-benzotriazole-1-yl)-1,1,3,3-tetramethyluronium hexafluorophosphate (HATU) with 1-hydroxy-7-azabenzotriazole (HOAt) as racemization suppressant. HATU was also used to acylate anilines and to attach tris(t-Bu)DOTA. Gly was chosen as C-terminal residue to avoid racemization in the cyclization step and at the same time raise its yields by turn preformation of the N-terminal D-amino acid.

*N*-alkylation was achieved via the Fukuyama-Mitsunobu reaction by treating Ns-protected amines with alcohols under typical Mitsunobu conditions (diisopropylazodicarboxylate (DIAD) and triphenylphosphine) *(Demmer et al., 2008).* Ns was cleaved by treatment with 1,8-diazabicyclo[5.4.0]undec-7-ene (DBU) and 2-mercaptoethanol to yield the secondary amine.

*N*^{α}-alkylated peptides were built from resin-bound *N*^{α}-Ns-D-Ala-Arg(Pbf)-Nal-Gly by *N-*alkylation. For peptides with acyl modifications in the peptoid side-chain Dde was deprotected, and acylated. From there on both classes of peptoids were treated in the same way with Ns deprotection and attachment of Fmoc-D-Tyr(t-Bu) with HATU. In contrast peptoids having an additional alkylation on the peptoid side chain, were Ns deprotected after the initial alkylation to the backbone, Fmoc-Tyr was attached, and Dde was cleaved off the peptoid side chain orthogonal to Fmoc (*Diaz-Mochon et al., 2004).* The amine on the side chain was subsequently reprotected with Ns followed by the second alkylation, and final Fmoc deprotection.

The peptoids were cleaved from the resin with 20% 1,1,1,3,3,3-hexafluoroisopropanol (HFIP) in DCM and cyclized with DPPA and NaHCO₃ in DMF. Final deprotection of acid labile groups was done in 95% trifluoroacetic acid (TFA) containing 2.5% H₂O and 2.5% triisopropylsilane (TIPS) before RP-HPLC purification. For peptides with alkylated peptoid side chains an additional Ns deprotection step was included prior to the final acidic deprotection. ESI-MS was used to identify the peptides and the purity determined by analytical RP-HPLC was better than 95%. Indium and Gallium ions were chelated by DOTA bearing peptides in an additional step in aqueous NH₄Ac solution and again purified by RP-HPLC. The dimeric peptides were synthesized by coupling two monomeric peptoids with a diacid.
**1. Loading of Tritylchloridpolystyrene (TCP) Resin.** Peptide synthesis was carried out using TCP-resin (0.9 mmol/g) following standard Fmoc-strategy. Fmoc-Xaa-OH (1.2 eq.) were attached to the TCP resin with DIEA (2.5 eq.) in anhydrous DCM (0.8 mL/g resin) at room temperature for 1 h. The remaining trityl chloride groups were capped by addition of 1mL/g(resin) of a solution of MeOH, DIEA (5:1; v:v) for 15 min. The resin was filtered and washed 5 times with DCM and 3 times with MeOH. The loading capacity was determined by weight after drying the resin under vacuum and ranged from 0.4-0.9 mmol/g.
**2. On-Resin Fmoc Deprotection.** The resin-bound Fmoc peptide was treated with 20% piperidine in NMP (v:v) for 10 minutes and a second time for 5 minutes. The resin was washed 5 times with NMP.
**3. TBTU/HOBt Coupling.** A solution of Fmoc-Xaa-OH (2 eq.), TBTU (2 eq.), HOBt (2 eq.), DIEA (5.2 eq.) in NMP (1 mL/g resin) was added to the resin-bound free amine peptide and shaken for 60 min at room temperature and washed 5 times with NMP.
**4. Ns Protection.** A solution of Ns-Cl (5 eq.) and collidine (10 eq.) in NMP (1 mL/g resin) was added to the resin-bound free amine peptide and shaken for 15 min at room temperature. The resin was washed 3 times with NMP and 3 times with dry THF.
**5. *N*-alkylation under Mitsunobu Conditions.** A solution of triphenylphosphine (5 eq.), DIAD (5eq.) and ROH (10 eq.) in dry THF (1 mL/g resin) was added to the resin-bound Ns protected peptides and shaken at room temperature for 30 minutes. The reaction was repeated with fresh reaction solution until no more starting material or conversion could be observed. The resin was filtered off, and washed 3 times with dry THF and 3 times with NMP.
**6. Dde Deprotection.** For Dde-group deprotection, the resin-bound peptides were stirred in a solution of 2% hydrazine in DMF (1 mL/g resin) for 10 minutes and a second time for 5 minutes. The resin was washed 5 times with DMF.
**7. On-Resin Ns Deprotection.** For Ns deprotection, the resin-bound Ns-peptides were stirred in a solution of mercaptoethanol (10 eq.) and DBU (5 eq.) in NMP (1 mL/g resin) for 5 minutes. The deprotection procedure was repeated one more time and the resin was washed 5 times with NMP.
**8. HATU/HOAt Coupling.** A solution of Fmoc-Xaa-OH or tris(t-Bu)DOTA (2 eq.), HA-TU (2 eq.), HOAt (2 eq.), DIEA (4 eq.) in NMP (1 mL/g resin) was added to the resin-bound peptides and shaken for 3 hours at room temperature and washed 5 times with NMP.
**9. Peptide Cleavage.** For complete cleavage from the resin the peptides were treated three times with a solution of DCM and HFIP (4:1; v:v) at room temperature for half an hour and the solvent evaporated under reduced pressure.
**10. Cyclization.** To a solution of peptide in DMF (1 mM peptide concentration) and Na-HCO₃ (5 eq.) DPPA (3 eq.) was added at RT and stirred over night or until no linear peptide could be observed by ESI-MS. The solvent was evaporated to a small volume under reduced pressure and the peptides precipitated in saturated NaCl solution and washed two times in HPLC grade water.
**11. Removal of Acid Labile Side Chain Protecting Groups.** Cyclized peptides were stirred in a solution of TFA, water and TIPS (95:2.5:2.5; v:v:v) at room temperature for one hour or until no more protected peptide could be observed by ESI-MS and precipitated in diethylether and washed two more times.
**12. Chelation of In with DOTA Ligands.** DOTA ligands were dissolved in 5 M aqueous ammonium acetate (0.5 ml; pH 4.5) and treated with InCl₃ (5 eq.) dissolved in 5 M ammonium acetate (0.05 ml). After 15 min of stirring at RT the solution was subjected to HPLC purification.
**13. Chelation of Ga with DOTA Ligands.** DOTA ligands were dissolved in 0.01 M aqueous ammonium acetate (0.5 ml; pH 4.5) and treated with Ga(NO₃)₃ (10 eq.) dissolved in 0.01 M ammonium acetate (0.05 ml) resulting in a final pH of 3. After 2-4 h of stirring at RT the solution was subjected to HPLC purification.
**14. Guanylation of amines.** A 10 mM solution of Peptides in DMF was treated with pyrrazolcarboxamidine-HCl (10 eq.) and DIEA (20 eq.) and stirred at RT for 2h or until no more starting material could be observed by ESI-MS. The solution was directly purified by RP-HPLC.
**15. Dde Deprotection orthogonal to Fmoc.** The deprotection solution was freshly prepared as described in the literature by dissolving 1.25 g (1.80 mmol) of NH₂OH·HCl and 0.918 g (1.35 mmol) of imidazole in 5mL of NMP by sonication (*Diaz-Mochon et al., 2004).* The solution was diluted with DCM (1:1; v:v) and the resin-bound peptide treated for 3 h with 1 mL of solution per g resin.
**16. Ns Deprotection in Solution.** For Ns deprotection cyclized peptides were treated with a solution of mercaptoethanol (10 eq.) and DBU (5 eq.) in 2.5 ml DMF for 30 minutes and precipitated in saturated NaCl solution and washed two times in HPLC grade water.
**17. Acylation in Solution.** Fully deprotected peptides were stirred with HATU (1.1 eq.) and DIEA (2.2 eq.) and the corresponding acid (1 eq.) or corresponding diacid (0.5 eq.) in DMF (10 mM peptide concentration) for 30 minutes at RT. The solution was directly purified by HPLC separation.
**18. Synthesis of *N*^{α}-Ns-D-alanine:**

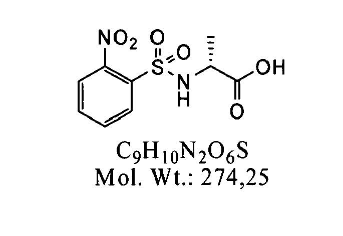

**To** a solution of D-alanine (0.60 g, 6.7 mmol) and Na₂CO₃ (1.78 g, 16.8 mmol) dissolved in 33 mL H₂O a solution of *o*-nitrobenzenesulfonylchloride (1.49 g, 6.7 mmol) in 33 mL THF was added under stirring at room temperature. After stirring for 2 h the THF was evaporated and the aqueous phase acidified with conc. HCl to a pH of 1. The precipitate was filtered off, dissolved in ethyl acetate, dried with Na₂SO₄, and evaporated to dryness to give a colorless, solid as sufficiently pure product (1.01 g, 55%). ¹H NMR (250 MHz, CDCl₃, 10% CD₃OD): δ 8.02 (m, 1H), 7.79 (m, 1H), 7.65 (m, 2H), 4.02 (m, 1H), 1.37 (d, 3H). ¹³C NMR (63 MHz, CDCl₃, 10% CD₃OD): 177.4, 137.6, 136.8, 134.4, 129.3, 56.0, 23.3. Rₜ (10-100%): 13.9 min. *m*/*z* calculated for C₉H₁₀N₂O₆S: 274.03; found 587.1 [2M + K⁺].

### Example 1: ya(Hex)RNalG

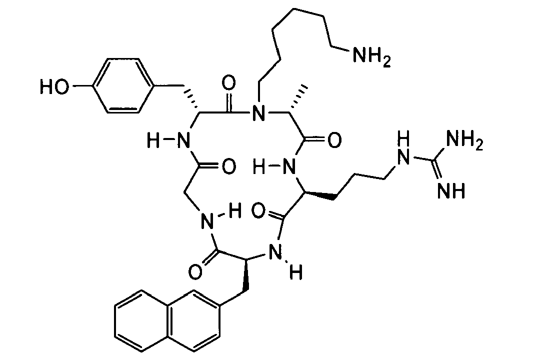

**ya(Hex)RNalG**
*cyclo*(-D-Tyr-(α-6-aminohexyl)-D-Ala-R-Nal-G)
C₃₉H₅₃N₉O₆
Exact Mass: 743,41
Mol. Wt.: 743,89

The linear peptide *N*^{α}-Fmoc-D-Ala-R(Pbf)-Nal-G was synthesized with standard Fmoc peptide chemistry following general protocols for resin-attachment **(1.)** of Gly and repeated Fmoc deprotection **(2.)** and attachment of the following (protected) amino acid **(3.).** Subsequently the Ala *N*^{α} was deprotected **(2.),** Ns reprotected **(4.),** and was alkylated with *N-*Boc-6-aminohexanol **(5.)** multiple times until no further conversion of the starting material could be observed. Subsequently the Ala *N*^{α}-Ns group was cleaved **(7.),** Fmoc-Tyr(tBu) attached **(8.)** in multiple couplings until no further conversion of the starting material could be observed. After cleavage of the Fmoc group **(2.)** the peptoid was cleaved from the resin **(9.),** cyclized **(10.),** deprotected **(11.)** and purified by RP-HPLC.

### Example 2: yA(Hex)RNalG

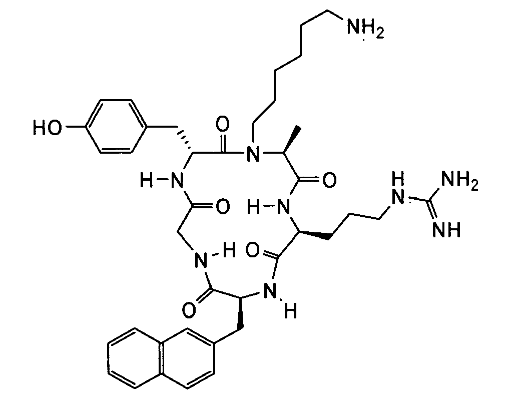

**yA(Hex)RNalG**
*cyclo*(-D-Tyr-(α-6-aminohexyl)-L-Ala-R-Nal-G)
C₃₉H₅₃N₉O6
Exact Mass: 743,41
Mol. Wt.: 743,89

The linear peptide *N*^{α}-Fmoc-L-Ala-R(Pbf)-Nal-G was synthesized with standard Fmoc peptide chemistry following general protocols for resin-attachment **(1.)** of Gly and repeated Fmoc deprotection **(2.)** and attachment of the following (protected) amino acid **(3.).** Subsequently the Ala *N*^{α} was deprotected **(2.),** Ns reprotected **(4.),** and was alkylated with *N-*Boc-6-aminohexanol **(5.)** multiple times until no further conversion of the starting material could be observed. Subsequently the Ala *N*^{α}-Ns group was cleaved **(7.),** Fmoc-Tyr(tBu) attached **(8.)** in multiple couplings until no further conversion of the starting material could be observed. After cleavage of the Fmoc group **(2.)** the peptoid was cleaved from the resin **(9.),** cyclized **(10.),** deprotected **(11.)** and purified by RP-HPLC.

### Example 3: yG(Hex)RNalG

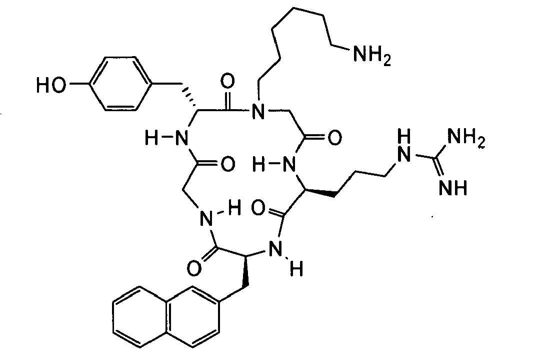

**yG(Hex)RNalG**
*cyclo*(-D-Tyr-(α-6-aminohexyl)-G-R-Nal-G)
C₃₈H₅₁N₉O₆
Exact Mass: 729,4
Mol. Wt.: 729,87

The linear peptide *N*^{α}-Fmoc-G-R(Pbf)-Nal-G was synthesized with standard Fmoc peptide chemistry following general protocols for resin-attachment **(1.)** of Gly and repeated Fmoc deprotection **(2.)** and attachment of the following (protected) amino acid **(3.).** Subsequently the Gly *N*^{α} was deprotected **(2.),** Ns reprotected **(4.),** and was alkylated with *N*-Boc-6-aminohexanol **(5.)** multiple times until no further conversion of the starting material could be observed. Subsequently the Gly *N*^{α}-Ns group was cleaved **(7.),** Fmoc-Tyr(tBu) attached **(8.)** in multiple couplings until no further conversion of the starting material could be observed. After cleavage of the Fmoc group **(2.)** the peptoid was cleaved from the resin **(9.)**, cyclized **(10.),** deprotected **(11.)** and purified by RP-HPLC.

### Example 4: ya(But)RNalG

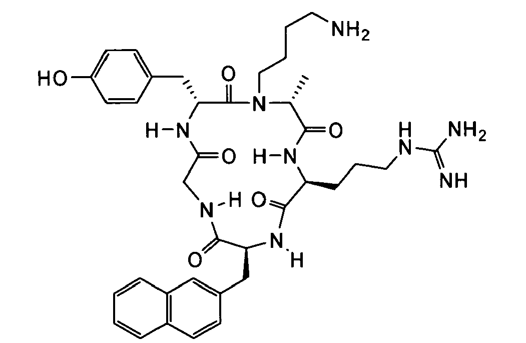

**ya(But)RNalG**
*cyclo*(-D-Tyr-(α-4-aminobutyl)-D-Ala-R-Nal-G)
C₃₇H₄₉N₉O₆
Exact Mass: 715,38
Mol. Wt.: 715,84

The linear peptide *N*^{α}-Ns-D-Ala-R(Pbf)-Nal-G was synthesized with standard Fmoc peptide chemistry following general protocols for resin-attachment **(1.)** of Gly and repeated Fmoc deprotection **(2.)** and attachment of the following (protected) amino acid **(3.).** Subsequently the Ala *N*^{α} was alkylated with *N*-Boc-6-aminobutanol **(5.)** multiple times until no further conversion of the starting material could be observed. Subsequently the Ala *N*^{α}-Ns group was cleaved **(7.),** Fmoc-Tyr(tBu) attached **(8.)** in multiple couplings until no further conversion of the starting material could be observed. After cleavage of the Fmoc group **(2.)** the peptoid was cleaved from the resin **(9.),** cyclized **(10.),** deprotected **(11.)** and purified by RP-HPLC.

### Example 5: ya(Pent)RNaIG

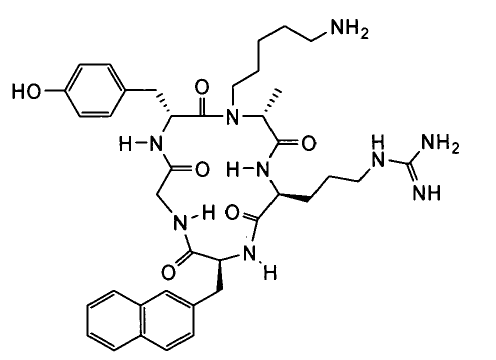

**ya(Pent)RNaIG**
*cyclo*(-D-Tyr-(α-5-aminopentyl)-D-Ala-R-Nal-G)
C₃₈H₅₁N₉O₆
Exact Mass: 729,4
Mol. Wt.: 729,87

The linear peptide *N*^{α}-Ns-D-Ala-R(Pbf)-Nal-G was synthesized with standard Fmoc peptide chemistry following general protocols for resin-attachment **(1.)** of Gly and repeated Fmoc deprotection **(2.)** and attachment of the following (protected) amino acid **(3.).** Subsequently the Ala *N*^{α} was alkylated with *N*-Boc-6-aminopentanol **(5.)** multiple times until no further conversion of the starting material could be observed. Subsequently the Ala *N*^{α}-Ns group was cleaved **(7.),** Fmoc-Tyr(tBu) attached **(8.)** in multiple couplings until no further conversion of the starting material could be observed. After cleavage of the Fmoc group **(2.)** the peptoid was cleaved from the resin **(9.),** cyclized **(10.),** deprotected **(11.)** and purified by RP-HPLC.

### Example 6: ya(Hept)RNalG

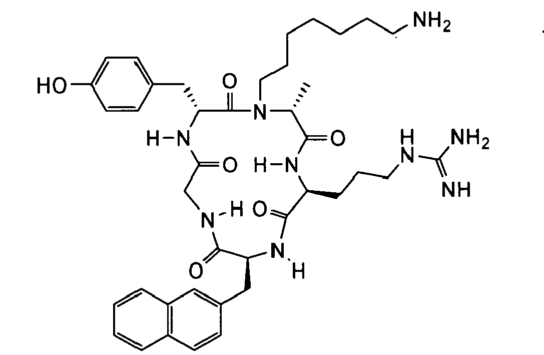

**ya(Hept)RNalG**
*cyclo*(-D-Tyr-(α-7-aminoheptyl)-D-Ala-R-Nal-G)
C₄₀H₅₅N₉O₆
Exact Mass: 757,43
Mol. Wt.: 757,92

The linear peptide *N*^{α}-Ns-D-Ala-R(Pbf)-Nal-G was synthesized with standard Fmoc peptide chemistry following general protocols for resin-attachment **(1.)** of Gly and repeated Fmoc deprotection **(2.)** and attachment of the following (protected) amino acid **(3.).** Subsequently the Ala *N*^{α} was alkylated with *N*-Boc-6-aminoheptanol **(5.)** multiple times until no further conversion of the starting material could be observed. Subsequently the Ala *N*^{α}-Ns group was cleaved **(7.),** Fmoc-Tyr(tBu) attached **(8.)** in multiple couplings until no further conversion of the starting material could be observed. After cleavage of the Fmoc group **(2.)** the peptoid was cleaved from the resin **(9.),** cyclized **(10.),** deprotected **(11.)** and purified by RP-HPLC.

### Example 7: ya(Hex, Ac)RNalG

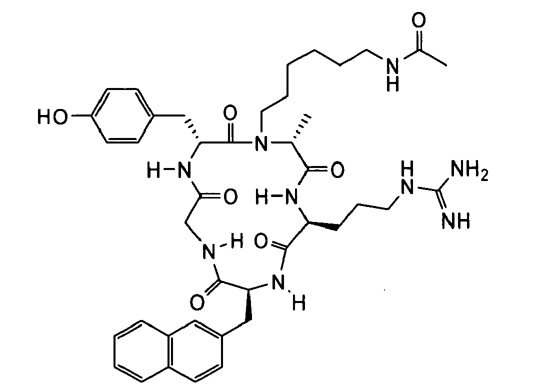

**ya(Hex, Ac)RNalG**
*cyclo*(-D-Tyr-(α-6-aminohexyl, Ac)-D-Ala-R-Nal-G)
C₄₁H₅₅N₉O₇
Exact Mass: 785,42
Mol. Wt.: 785,93

The linear peptide *N*^{α}-Ns-D-Ala-R(Pbf)-Nal-G was synthesized with standard Fmoc peptide chemistry following general protocols for resin-attachment **(1.)** of Gly and repeated Fmoc deprotection **(2.)** and attachment of the following (protected) amino acid **(3.).** Subsequently the Ala *N*^{α} was alkylated with *N*-Dde-6-aminohexanol **(5.)** multiple times until no further conversion of the starting material could be observed. The peptoid side chain was modified by Dde cleavage **(6.)** and acylaction with acetic acid **(3.).** Subsequently the Ala *N*^{α}-Ns group was cleaved **(7.),** Fmoc-Tyr(tBu) attached **(8.)** in multiple couplings until no further conversion of the starting material could be observed. After cleavage of the Fmoc group **(2.)** the peptoid was cleaved from the resin **(9.),** cyclized **(10.),** deprotected **(11.)** and purified by RP-HPLC.

### Example 8: ya(Hex, FBz)RNalG

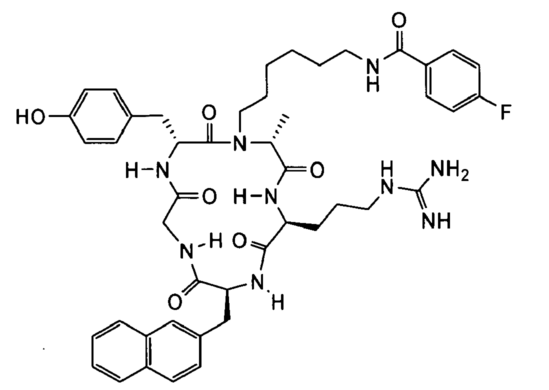

**ya(Hex, FBz)RNalG**
*cyclo*(-D-Tyr-(α-6-aminohexyl, 4-fluorobenzoic acid)-D-Ala-R-Nal-G)
C₄₆H₅₆FN₉O₇
Exact Mass: 865,43
Mol. Wt.: 865,99

The linear peptide *N*^{α}-Ns-D-Ala-R(Pbf)-Nal-G was synthesized with standard Fmoc peptide chemistry following general protocols for resin-attachment **(1.)** of Gly and repeated Fmoc deprotection **(2.)** and attachment of the following (protected) amino acid **(3.).** Subsequently the Ala *N*^{α} was alkylated with *N*-Dde-6-aminohexanol **(5.)** multiple times until no further conversion of the starting material could be observed. The peptoid side chain was modified by Dde cleavage **(6.)** and acylaction with 4-fluorobenzoic acid **(3.).** Subsequently the Ala *N*^{α}-Ns group was cleaved **(7.),** Fmoc-Tyr(tBu) attached **(8.)** in multiple couplings until no further conversion of the starting material could be observed. After cleavage of the Fmoc group **(2.)** the peptoid was cleaved from the resin **(9.),** cyclized **(10.),** deprotected **(11.)** and purified by RP-HPLC.

### Example 9: ya(Hex, DOTA)RNalG

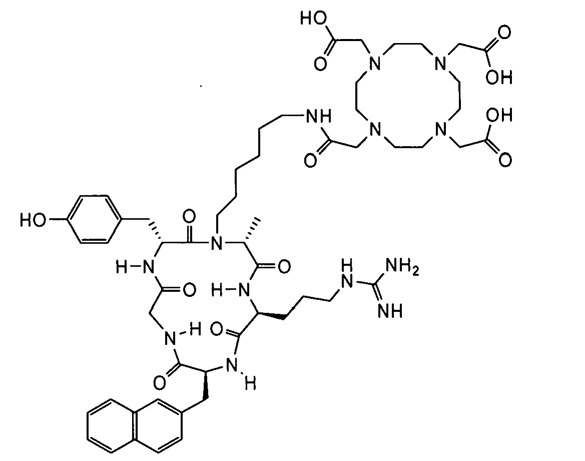

**ya(Hex, DOTA)RNalG**
*cyclo*(-D-Tyr-(α-6-aminohexyl, DOTA)-D-Ala-R-Nal-G)
C₅₅H₇₉N₁₃O₁₃
Exact Mass: 1129,59
Mol. Wt.: 1130,3

The linear peptide *N*^{α}-Ns-D-Ala-R(Pbf)-Nal-G was synthesized with standard Fmoc peptide chemistry following general protocols for resin-attachment **(1.)** of Gly and repeated Fmoc deprotection **(2.)** and attachment of the following (protected) amino acid **(3.)**. Subsequently the Ala *N*^{α} was alkylated with *N-*Dde-6-aminohexanol **(5.)** multiple times until no further conversion of the starting material could be observed. The peptoid side chain was modified by Dde cleavage **(6.)** and acylaction with tris(t-Bu)DOTA **(8.).** Subsequently the Ala *N*^{α}-Ns group was cleaved **(7.),** Fmoc-Tyr(tBu) attached **(8.)** in multiple couplings until no further conversion of the starting material could be observed. After cleavage of the Fmoc group **(2.)** the peptoid was cleaved from the resin **(9.),** cyclized **(10.),** deprotected **(11.)** and purified by RP-HPLC. The metal chelates of the pure peptides were obtained by labeling with Indium **(12.)** or Gallium **(13.)** with subsequent RP-HPLC purification.

### Example 10: ya(But, FBz)RNalG

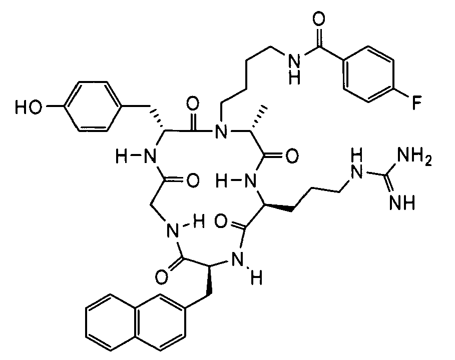

**ya(But, FBz)RNalG**
*cyclo*(-D-Tyr-(α-4-aminobutyl, 4-fluorobenzoic acid)-D-Ala-R-Nal-G)
C₄₄H₅₂FN₉O₇
Exact Mass: 837,4
Mol. Wt.: 837,94

The amine of purified peptide ya(But)RNalG was acylated with 4-fluorobenzoic acid **(17.)** and purified by RP-HPLC.

### Example 11: ya(Pent, FBz)RNaIG

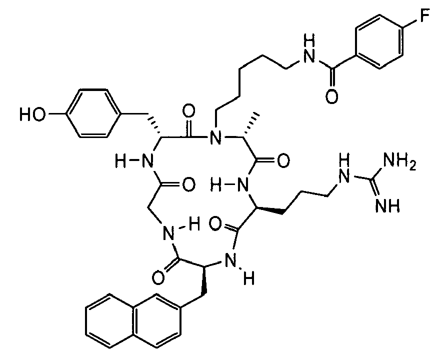

**ya(Pent, FBz)RNalG**
*cyclo*(-D-Tyr-(α-5-aminopentyl, 4-fluorobenzoic acid)-D-Ala-R-Nal-G)
C₄₅H₅₄FN₉O₇
Exact Mass: 851,41
Mol. Wt.: 851,96

The amine of purified peptide ya(Pent)RNalG was acylated with 4-fluorobenzoic acid **(17.)** and purified by RP-HPLC.

### Example 12: ya(Hept, FBz)RNalG

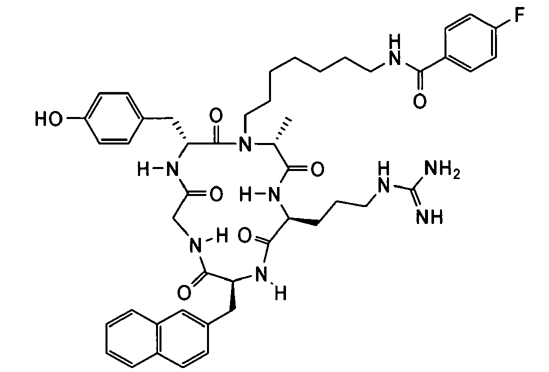

**ya(Hept, FBz)RNalG**
*cyclo*(-D-Tyr-(α-7-aminoheptyl, 4-fluorobenzoic acid)-D-Ala-R-Nal-G)
C₄₇H₅₈FN₉O₇
Exact Mass: 879,44
Mol. Wt.: 880,02

The amine of purified peptide ya(Hept)RNalG was acylated with 4-fluorobenzoic acid **(17.)** and purified by RP-HPLC.

### Example 13: ya(Hex, Gua)RNalG

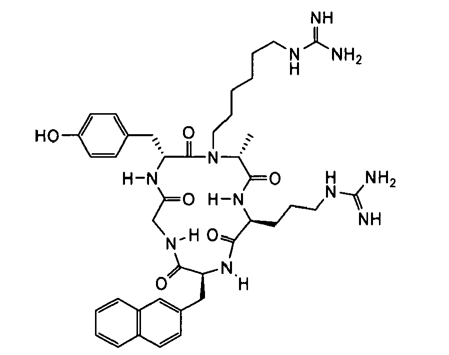

**ya(Hex, Gua)RNalG**
cyclo(-D-Tyr-(α-6-guanidinohexyl)-D-Ala-R-Nal-G)
C₄₀H₅₅N₁₁O₆
Exact Mass: 785,43
Mol. Wt.: 785.93

The amine of purified peptide ya(Hex)RNalG was guanylated **(14.)** and purified by RP-HPLC.

### Example 14: yA(Hex, Gua)RNalG

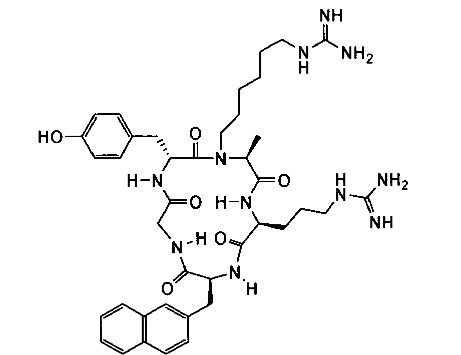

**yA(Hex, Gua)RNalG**
*cyclo*(-D-Tyr-(α-6-guanidinohexyl)-L-Ala-R-Nal-G)
C₄₀H₅₅N₁₁O₆
Exact Mass: 785,43
Mol. Wt.: 785,93

The amine of purified peptide yA(Hex)RNalG was guanylated **(14.)** and purified by RP-HPLC.

### Example 15: ya(Hex, Bz)RNaIG

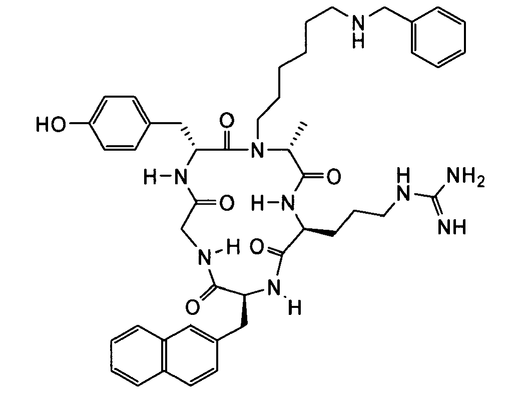

**ya(Hex, Bz)RNalG**
*cyclo*(-D-Tyr-(α-6-aminohexyl, benzyl)-D-Ala-R-Nal-G)
C₄₆H₅₉N₉O₆
Exact Mass: 833,46
Mol. Wt.: 834,02

The linear peptide *N*^{α}-Ns-D-Ala-R(Pbf)-Nal-G was synthesized with standard Fmoc peptide chemistry following general protocols for resin-attachment **(1.)** of Gly and repeated Fmoc deprotection **(2.)** and attachment of the following (protected) amino acid **(3.).** Subsequently the Ala *N*^{α} was alkylated with ***N*-Dde-6-aminohexanol** (5.) multiple times until no further conversion of the starting material could be observed. Subsequently the Ala *N*^{α}-Ns group was cleaved **(7.),** Fmoc-Tyr(tBu) attached **(8.)** in multiple couplings until no further conversion of the starting material could be observed. The peptoid side chain was modified by Dde cleavage orthogonal to Fmoc **(15.),** Ns reprotection **(4.),** and alkylation with benzylic alcohol **(5.)** (one time for 15 minutes). After cleavage of the Fmoc group (2.) the peptoid was cleaved from the resin (9.), cyclized **(10.),** Ns deprotected in solution **(16.)**, followed by deprotection of the acid labile groups **(11.)** and purified by RP-HPLC.

### Example 16: ya(Hex, Nap)RNalG

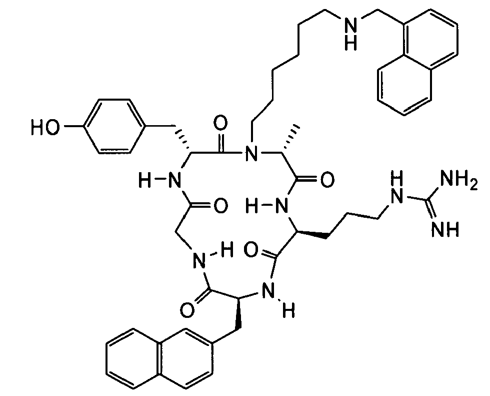

**ya(Hex, Nap)RNalG**
***cyclo*(-D-Tyr-(α-6-aminohexyl,**1-naphthylmethyl)-D-Ala-R-Nal-G)
C₅₀H₆)N₉O₆
Exact Mass: 883,47
Mol. Wt.: 884,08

The linear peptide *N*^{α}-Ns-D-Ala-R(Pbf)-Nal-G was synthesized with standard Fmoc peptide chemistry following general protocols for resin-attachment **(1.)** of Gly and repeated Fmoc deprotection **(2.)** and attachment of the following (protected) amino acid **(3.).** Subsequently the Ala *N^{α}* was alkylated with *N*-Dde-6-aminohexanol **(5.)** multiple times until no further conversion of the starting material could be observed. Subsequently the Ala *N^{α}*-Ns group was cleaved **(7.),** Fmoc-Tyr(tBu) attached **(8.)** in multiple couplings until no further conversion of the starting material could be observed. The peptoid side chain was modified by Dde cleavage orthogonal to Fmoc **(15.),** Ns reprotection **(4.),** and alkylation with 1-naphthylmethanol **(5.)** (one time for 15 minutes). After cleavage of the Fmoc group **(2.)** the peptoid was cleaved from the resin **(9.),** cyclized **(10.),** Ns deprotected in solution **(16.),** followed by deprotection of the acid labile groups **(11.)** and purified by RP-HPLC.

### Example 17: ya(Hex)RNaIG Dimer 4

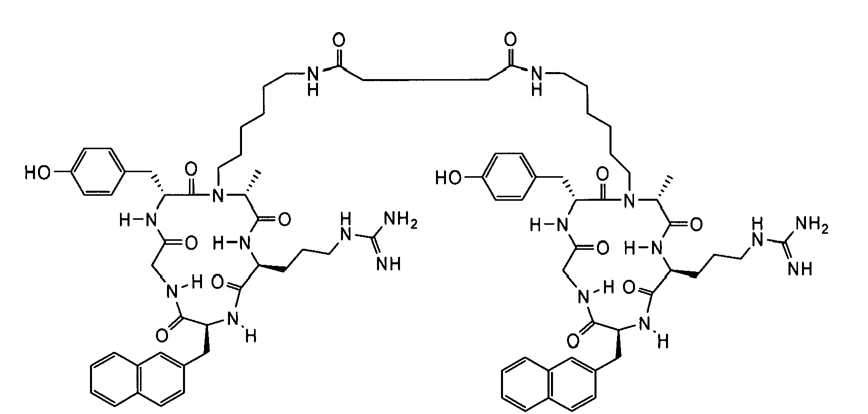

**ya(Hex)RNalG Dimer 4**
ya(Hex)RNalG butan-1,4-diacid dimer
C₈₂H₁₀₈N₁₈O₁₄
Exact Mass: 1568,83
Mol. Wt.: 1569,85

Fully deprotected ya(Hex)RNalG was dimerized with succinic acid (17.) and purified by RP-HPLC.

### Example 18: ya(Hex)RNalG Dimer 6

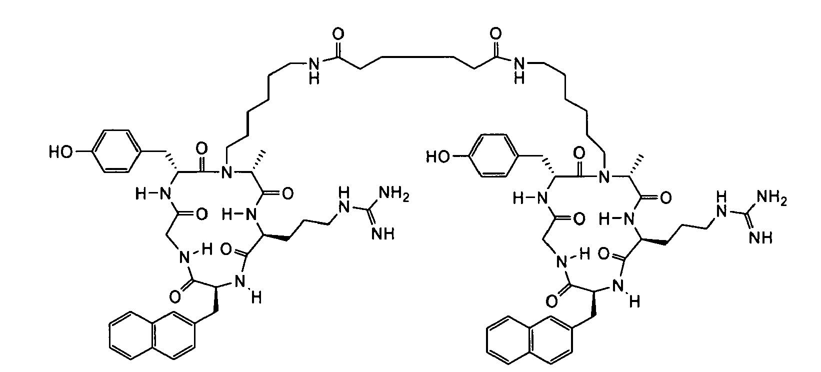

**ya(Hex)RNalG Dimer 6**
ya(Hex)RNalG hexan-1,6-diacid dimer
C₈₄H₁₁₂N₁₈O₁₄
Exact Mass: 1596.86
Mol. Wt.: 1597,9

Fully deprotected ya(Hex)RNalG was dimerized with adipic acid (17.) and purified by RP-HPLC.

### Example 19: ya(Hex)RNaIG Dimer 8

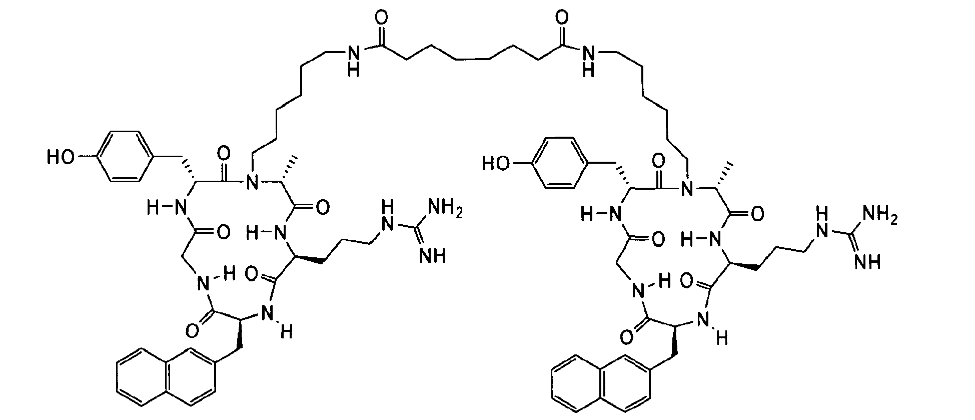

**ya(Hex)RNaIG Dimer 8**
ya(Hex)RNalG octan-1,8-diacid dimer
C₈₆H₁₁₆N₁₈O₁₄
Exact Mass: 1624,89
Mol. Wt.: 1625,95

Fully deprotected ya(Hex)RNalG was dimerized with suberic acid **(17.)** and purified by RP-HPLC.

### Analytical Data:

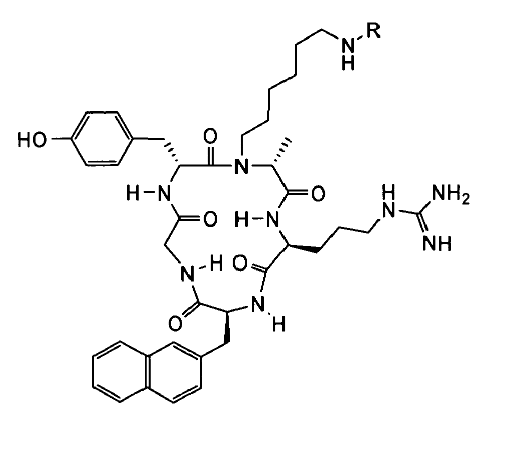

| Nr | Name | R | IC₅₀ | n Repeats | exact mass | HPLC-MS | |
|---|---|---|---|---|---|---|---|
| | | | | | | m+H⁺ | R_{T} |
| | ya(Hex)RNalG | H | 0.3 ± 0.2 | 4 | 743.41 | 744.4 | 7.54 |
| | ya(Hex,DOTA) RNalG | 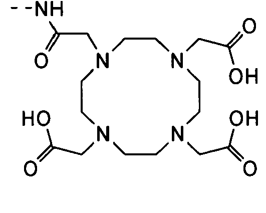 | 509.7 ± 0.2 | 2(3) | 1129.59 | 1130.7 | 7.21 |
| | ya(Hex, FBz) RNalG | 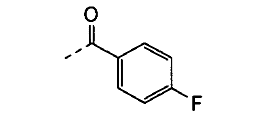 | 21.4 ± 5.8 | 3 | 865.43 | 866.6 | 9.35 |
| | ya(Hex, DOTA, In) RNalG | 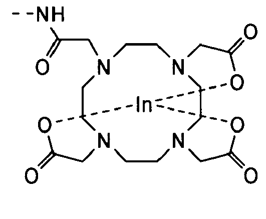 | 301.6 ± 42.7 | 3 | 1241.47 | 7.35 | 1242.5 |
| | ya(Hex,Ac) RNalG | 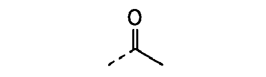 | 44.1 ± 6.2 | 3 | 785.42 | 8.06 | 786.6 |
| | ya(Hex,1 Nap) RNalG | 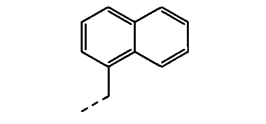 | 5.1 ± 1.3 | 3 | 883.47 | 9.52 | 884.5 |
| | ya(Hex, Bz) RNalG | 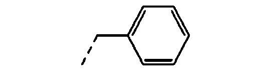 | 4.2 ± 1.4 | 5 | 833.46 | 8.16 | 834.5 |
| | ya(Hex, Gua) RNalG | 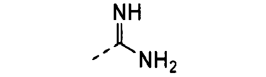 | 0.04 ± 0.02 | 5 | 785.43 | 6.96 | 786.5 |
| | | | | | | | |

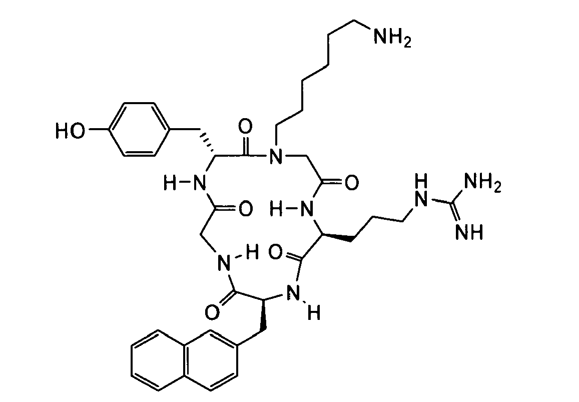

| Nr | Name | IC₅₀ | n Repeats | exact mass | HPLC-MS | |
|---|---|---|---|---|---|---|
| | | | | | m+H⁺ | R_{T} |
| | yG(Hex)RNalG | 103,8 ± 3,5 | 3 | 729.4 | 730.4 | 6.77 |

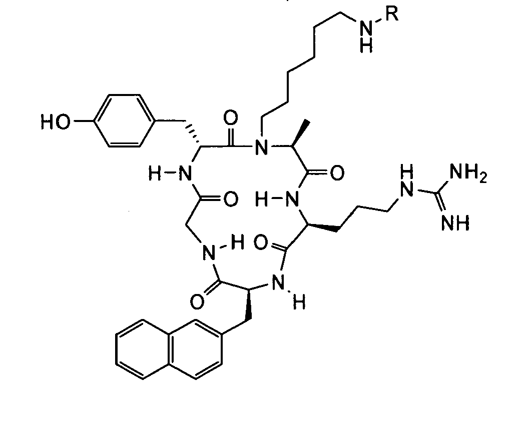

| Nr | Name | R | IC₅₀ | n Repeats | exact mass | HPLC-MS | |
|---|---|---|---|---|---|---|---|
| | | | | | | m+ H⁺ | R_{T} |
| | yA(Hex)RNalG | H | 94.2 ± 38.0 | 3 | 743.41 | 744.5 | 7.07 |
| | yA(Hex,Gua)RNa IG | 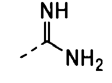 | 4.4 ± 0.3 | 3 | 785.43 | 786.5 | 7.2 |

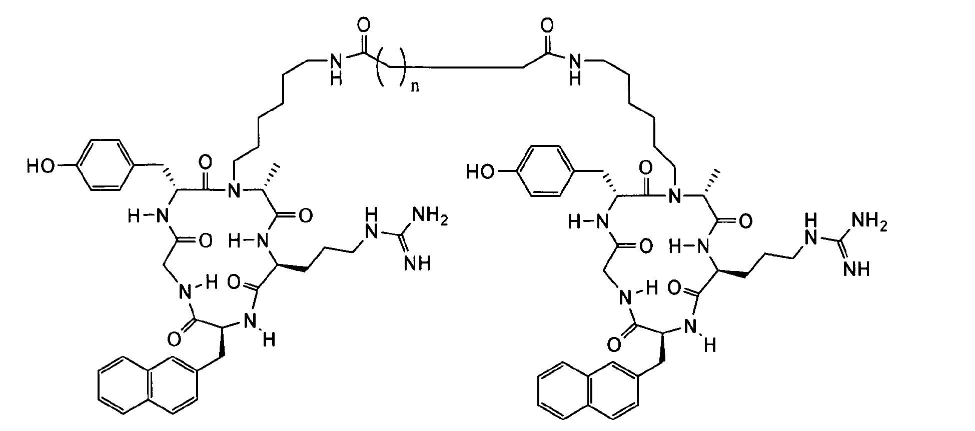

| Nr | Name | n | IC₅₀ | n Repeats | exact mass | HPLC-MS | |
|---|---|---|---|---|---|---|---|
| | | | | | | m+H⁺ | R_{T} |
| | Dimer 4 | 1 | 5.5 ± 1.4 | 3 | 1568.83 | 1569.8 | 8.47 |
| | Dimer 6 | 3 | 7.8 ± 3.1 | 3 | 1596.86 | 1597.8 | 8.57 |
| | Dimer 8 | 5 | 15.9 ± 10.6 | 3 | 1624.89 | 1625.8 | 8.76 |
| | | | | | | | |

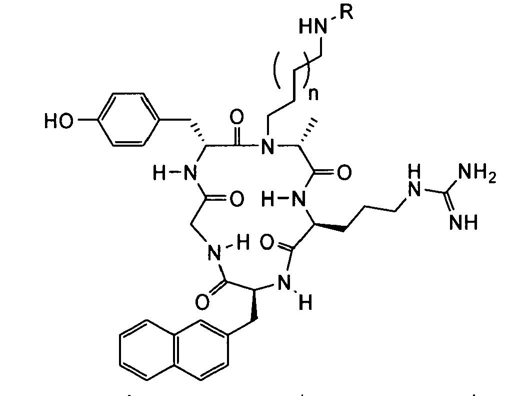

| Nr | Name | n | R | IC₅₀ | n Repeats | Exact mass | HPLC-MS | |
|---|---|---|---|---|---|---|---|---|
| | | | | | | | m+ H⁺ | R_{T} |
| | ya(But)RNalG | 1 | H | 0.9 ± 0.1 | 3 | 715.38 | 716.5 | 6.82 |
| | ya(But,FBz)RNalG | 1 | 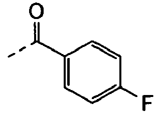 | 26.0 ± 5.7 | 3 | 837.4 | 838.5 | 8.74 |
| | ya(Pent)RNalG | 2 | H | 1.6 ± 0.3 | 3 | 729.4 | 730.5 | 6.92 |
| | ya(Pent, FBz)RNalG | 2 | 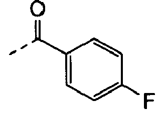 | 34.2 ± 9.3 | 3 | 851.41 | 852.5 | 9.27 |
| | ya(Hept)RNalG | 4 | H | 2.4 ± 0.8 | 3 | 757.43 | 758.5 | 7.20 |
| | ya(Hept,FBz)RNalG | 4 | 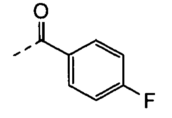 | 16.5 ± 3.1 | 3 | 865.43 | 866.6 | 9.35 |

### Example 20: Receptor Binding Assays.

For receptor binding assays cells were resuspended in PBS/0.2 % BSA. A total of 200 µl of the suspension containing 400,000 Jurkat cells were incubated with 25 µl of the tracer solution (containing 100000cpm / 25µl, approx. 0.1 nM) and 25 µl of the diluent or the competitor at different concentrations. For determination of IC₅₀ values, cyclo(-D-Tyr¹ [¹²⁵I]-Arg²-Arg³-Nal⁴-Gly⁵) was used as a tracer. Nonspecific binding was determined in the presence of 1 µM cold cyclo(-D-Tyr¹[¹²⁷I]-Arg²-Arg³-Nal⁴-Gly⁵). After shaking for 2 h at room temperature, the incubation was terminated by centrifugation at 2000 rpm for 5 min. Cell pellets were washed once with 400µl cold PBS followed by a second centrifugation step. Cell bound radioactivity was determined by using a gamma counter. Experiments were repeated 2-3 times in triplicates. IC₅₀ values of the compounds were calculated by nonlinear regression using GraphPad Prism (GraphPad Prism 4.0 Software, Inc., San Diego, CA, USA). Each data point is the average of three determinations.

For *in vivo*-studies of receptor binding of the compounds according to the invention, animal experiments are envisaged. In an exemplary method, parental CMS5 cells (tumorigenic fibrosarcoma cell line) and transduced CMS5/CXCR4 cells are injected subcutaneously e.g. in female Swiss nu/nu mice (Charles River, France). For each mouse, e.g. 1.5×10⁶ CMS5 cells and 2×10⁶ CMS5/CXCR4 cells are resuspended in 75 µl PBS and mixed with the same volume Matrigel-Matrix HC (BD Biosciences, Heidelberg, Germany) according to the manufacturer's protocol. Subsequently cell suspension are inoculated at each shoulder. E.g. after 14-16 days of tumor growth, mice may be used for imaging and biodistribution purposes. Methods for imaging and for biodistribution studies are well known within the art. Imaging may e.g. be effected by means of scintigraphic images obtained by a gamma camera. For an exemplary envisaged biodistribution study, 10 µCi of ¹²⁵I-labeled compound are injected intravenously into the tail vain of tumor-bearing mice. The animals are sacrificed and dissected at suitable times, such as 30, 60 and 120 min after tracer injection. Organs of interest are removed and the radioactivity is measured in weighted tissue samples by e.g. using a gamma counter.

### References

Burger-Kentischer A et al, 2002, Expression of macrophage migration inhibitory factor in different stages of human atherosclerosis, Circulation 105, 1561-1566.
Chong, J. M.; Heuft, M. A.; Rabbat, P. Solvent effects on the monobromination of alpha,omega-diols: A convenient preparation of omega-bromoalkanols. Journal of Organic Chemistry 2000, 65, 5837-5838
Demmer, O.; Dijkgraaf, I.; Schottelius, M.; Wester, H. J.; Kessler, H. Introduction of functional groups into peptides via N-alkylation. Organic Letters 2008, 10, 2015-2018 Diaz-Mochon, J. J.; Bialy, L.; Bradley, M. Full orthogonality between Dde and Fmoc: The direct synthesis of PNA-peptide conjugates. Organic Letters 2004, 6, 1127-1129. [ Forbes, C. C.; DiVittorio, K. M.; Smith, B. D. Bolaamphiphiles promote phospholipid translocation across vesicle membranes. Journal of the American Chemical Society 2006, 128,9211-9218
N. Fujii, S. Oishi, K. Hiramatsu, T. Araki, S. Ueda, H. Tamamura, A. Otaka, S. Kusano, S. Terakubo, H. Nakashima, J. A. Broach, J. O. Trent, Z. X. Wang, S. C. Peiper, Angewandte Chemie-International Edition 2003, 42, 3251
Kim J, Takeuchi H, Lam ST, Turner RR, Wang HJ, Kuo C, Foshag L, Bilchik AJ, Hoon DS (2005) Chemokine receptor CXCR4 expression in colorectal cancer patients increases the risk for recurrence and for poor survival. J Clin Oncol.; 23(12):2744-53.
Kuehl H, Veit P, Rosenbaum SJ, Bockisch A, Antoch G (2007) Can PET/CT replace separate diagnostic CT for cancer imaging? Optimizing CT protocols for imaging cancers of the chest and abdomen. J Nucl Med.; 48 Suppl 1:45S-57S.
J. P. Levesque and I.G. Winkler, Curr Opin Organ Transplant. 2008 Feb;13(1):53-8) Libby P. (2002) Inflammation in atherosclerosis. Nature 420, 868-874.
Hansson, GK, 2005, Inflammation, atherosclerosis, and coronary artery disease, N. Engl. J. Med. 351, 1985-1695.
Mizukami, S.; Takikawa, R.; Sugihara, F.; Hori, Y.; Tochio, H. et al. Paramagnetic relaxation-based F-19 MRI probe to detect protease activity. Journal of the American Chemical Society 2008, 130, 794-795
Nilsen, A.; England, P. M. A subtype-selective, use-dependent inhibitor of native AMPA receptors. J Am Chem Soc 2007, 129, 4902-4903
Phillips RJ, Burdick MD, Lutz M, Belperio JA, Keane MP, Strieter RM (2003) The stromal derived factor-1/CXCL12-CXC chemokine receptor 4 biological axis in non-small cell lung cancer metastases. Am J Respir Crit Care Med.; 167(12):1676-86.
Ross R (1993), The pathogenesis of atherosclerosis: a perspective for the 1990s. Nature 362:801-80.
Schober A. Bernhagen J, Weber C. Chemokine-like functions of MIF in atherosclerosis, J. Mol. Med (2008), 86, 761-770.
Shah K, Weissleder R. (2005) Molecular optical imaging: applications leading to the development of present day therapeutics. NeuroRx.;2(2):215-25.
Taniuchi S, Masuda M, Fujii Y, Izawa K, Kanegane H, Kobayashi Y (2005) The role of a mutation of the CXCR4 gene in WHIM syndrome. Haematologica; 90(9):1271-2. Weissleder R, Ntziachristos V (2003) Shedding light onto live molecular targets. Nat Med.; 9(1):123-8.
Weissleder R, Pittet MJ (2008) Imaging in the era of molecular oncology, Nature ; 452(7187):580-9.
Zhang et al., Inorg. Chem. 37(5), 1998, 956-963
WO 89/07456
WO 97/31657
WO 2008/08854
WO 2007/096662
WO 2009/027706
WO 2009/109332

## Claims

1. A compound having a structure according to formula I
cyclo[(R¹)Xaa¹-B-(R²)Xaa²-(R³)Xaa³-(R⁴)Xaa⁴] (I)
or a pharmaceutically acceptable salt thereof,
wherein Xaa¹ and Xaa³ are independently of each other, a natural or unnatural amino acid, preferably with Xaa¹ and Xaa³ being, independently of each other, an amino acid comprising an aromatic moiety in its side chain,
Xaa² is a natural or unnatural basic amino acid,
Xaa⁴ is glycine or a D-amino acid,
R¹, R², R³ and R⁴ are independently of each other H or alkyl,
B is an amino acid group having a structure according to formula II 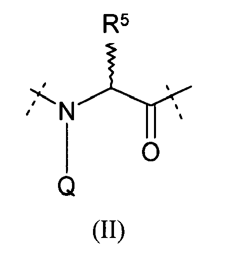 wherein R⁵ is selected from the group consisting of H, optionally substituted alkyl, optionally substituted aryl and optionally substituted heteroaryl, and a moiety L,
and wherein Q is a group being sterically more demanding than a methyl group.

2. The compound of claim 1, wherein Q is a linker moiety comprising the group -X-R⁶ with X being selected from the group consisting of =N-, -N=, -Y-, -C(=Y)-NH-, -NH-C(=Y)-, -NH-C(=Y)-Y'-, -Y'-C(=Y)-NH-, -Y'-C(=Y)-, -C(=Y)-Y'-, -C(=Y)-, alkynylene, azide, and 1,2,3-triazole, with Y and Y' being independently of each other selected from the group consisting of NH, O and S, and with R⁶ being selected from the group consisting of H, optionally substituted alkyl, optionally substituted aryl, optionally substituted alkoxy group, optionally substituted heteroaryl, a compound D, and wherein in case R⁶ is substituted, the substituent preferably comprises a compound D, particularly wherein the compound D is selected from the group consisting of detectable labels, organic complexation agents, active substances and combinations thereof, particularly wherein the active substance is selected from cytotoxic agents and lipids.

3. The compound of claim 1 or 2, wherein R⁵, particularly group L; and/or ii) group Q, particularly group X or R⁶; and/or iii) amino acid Xaa⁴, particularly group S⁴; are optionally attached to at least one further compound of formula (I)

4. The compound of any of claims 1 to 3, wherein the linker moiety L is or is optionally substituted with a compound D, particularly selected from the group consisting of detectable labels, organic complexation agents, active substances and combinations thereof, particularly wherein the active substance is selected from cytotoxic agents and lipids;
and/or
wherein Xaa¹ and Xaa³ are, independently of each other, selected from the group phenylalanine, D-phenylalanine, tyrosine, D-tyrosine, tryptophan, D-tryptophan, D-phenylglycine, phenylglycine, naphthylalanine (Nal) and D-naphthylalanine (D-Nal).

5. The compound of any of claims 1 to 4, wherein Xaa¹ is D-tyrosine and wherein Xaa³ is tryptophan or naphthylalanine, preferably wherein Xaa³ is naphthylalanine;
and/or
wherein Xaa² is arginine and wherein Xaa⁴ is glycine.

6. The compound of any of claims 1 to 5, wherein Q is -[C(R⁷R⁸)]ₙ-X-R⁶, with R⁷ and R⁸ being independently of each other selected from the group consisting of H and optionally substituted alkyl, preferably with both R⁷ and R⁸ being H, and with integer n being in the range of from 1 to 25, preferably 1, 2, 3, 4, 5 or 6; and/or
wherein R⁵ is selected from the group consisting of H, methyl, aminomethyl, 1-aminoethyl, 1-aminopropyl, 1-aminopentyl, 1-aminobutyl and 1-aminohexyl, preferably wherein R⁵ is methyl.

7. The compound of any of claims 1 to 6, wherein the amino acid group having the structure according to formula (II) is present in D-configuration.

8. The compound of any of claims 2 to 7, wherein X is selected from the group consisting of -Y-, -C(=Y)-Y'-, -Y'-C(=Y)-, -NH-C(=Y)-Y'-, -Y'-C(=Y)-NH- with Y and Y' being NH, and preferably wherein Q is -[C(R⁷R⁸)]ₙ-X-R⁶ with both R⁷ and R⁸ being H.

9. The compound of any of claims 1 to 8, having the formula
cyclo[D-Tyr-B-Arg-Nal -Gly];
particularly wherein B is an amino acid group having a structure according to formula III, IV or V 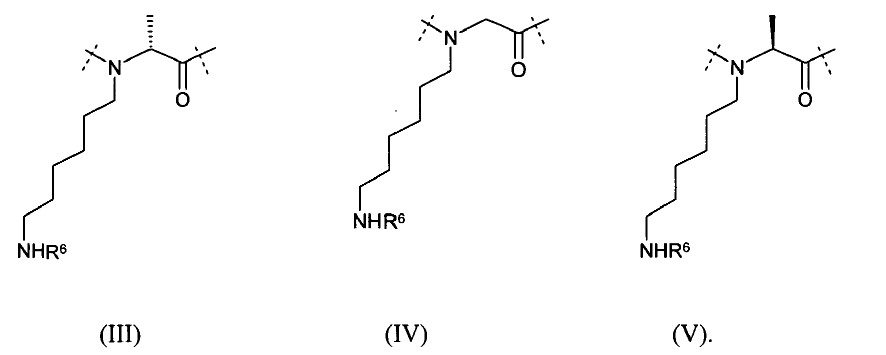 especially wherein R⁶ is H or 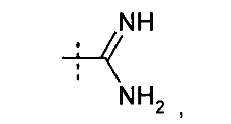 in particular wherein R⁶ is

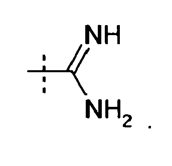

10. The compound of any of claims 2 to 9, wherein R⁶ is an alkyl group being selected from the group consisting of ethyl, propyl, pentyl, butyl and hexyl and wherein said group R⁶ is being attached to a further compound of formula (I), preferably via group X of said further compound of formula (I);
in particular wherein said compound has the formula

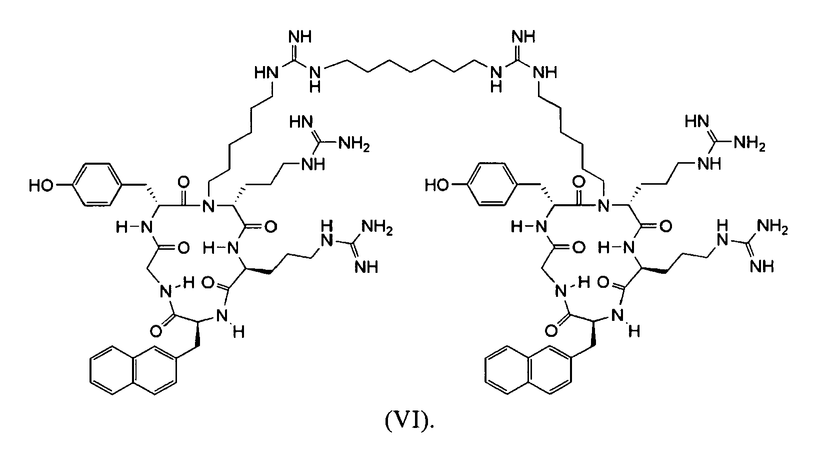

11. The compound of any of claims 1 to 10, wherein R⁵, R⁶ and/or S⁴ comprises a detectable label, an organic complexation agent or a combination thereof; particularly wherein R⁶ comprises or is a radiolabel, an organic complexation agent or a combination thereof; especially wherein R⁵ or L comprises or is a radiolabel, an organic complexation agent or a combination thereof.

12. The compound of claim 11, wherein any one of R⁵, R⁶ and/or S⁴ comprises, particularly wherein R⁵ comprises:
i) an organic complexation agent, and wherein the organic complexation agent is selected from the group consisting of NODASA, NODAGA, TETA, TRITA, DTPA, EDTA, CDTA, CPTA, EGTA; HBED, TTHA, DTPA, DOTA, NOTA, HP-DOA3, CBTE2a, TE2A, TMT, DPDP, HYNIC, DFO, and HEDTA, particularly selected from DOTA, NOTA, DTPA, and TETA; and/or
ii) a radionuclide selected from the group consisting of ¹¹C, ¹⁸F, ⁴⁷Sc, ⁵¹Cr, ^{52m}Mn , ⁵⁸Co, ⁵²Fe , ⁵⁶Ni, ⁵⁷Ni, ⁶²Cu, ⁶⁴Cu, ⁶⁷Cu, ⁶⁶Ga, ⁶⁸Ga, ⁶⁷Ga, ⁷²AS, ⁷⁷As, ⁷⁵Br, ⁷⁶Br, ⁷⁷Br, ⁸²Br, ⁸⁹Zr, ⁹⁰Y, ^{94m}Tc, ^{99m}Tc, ⁹⁷Ru, ¹⁰⁵Rh, ¹⁰⁹Pd, ¹¹¹Ag, ^{110m}In ¹¹¹In, ^{113m}In ^{114m}In, ¹²⁰I,¹²³I, ¹²⁴I, ¹²⁵I, ¹³¹I, ^{117m}Sn, ¹²¹Sn, ¹²⁷Te, ¹⁴²Pr, ¹⁴³Pr, ¹⁴⁹Pm, ¹⁵¹Pm, ¹⁴⁹Tb, ¹⁵³Sm, ¹⁵⁷Gd, ¹⁶¹Tb, ¹⁶⁶Ho, ¹⁶⁵Dy, ¹⁶⁹Er, ¹⁶⁹Yb, ¹⁷⁵Yb, ¹⁷²Tm, ¹⁷⁷Lu, ¹⁸⁶Re, ¹⁸⁸Re, ¹⁹¹Pt, ¹⁹⁷Hg, ¹⁹⁸Au, ¹⁹⁹Au, ²⁰¹Tl, ²⁰³Pb, ²¹¹At, ²¹²Bi and ²²⁵Ac; particularly from the group consisting of ¹⁸F ⁶⁸Ga, ⁸⁹Zr, ⁹⁰Y, ^{99m}Tc, ¹¹¹In ¹²³I, ¹²⁴I, ¹³¹I, ¹⁷⁷Lu; especially from the group consisting of ¹⁸ F, ⁶⁸Ga,^{99m}Tc, ¹¹¹In, ¹²³I, and ¹⁷⁷Lu; and/or
iii) a combination of a radionuclide and a complexation agent, with the complexation agent being covalently bound to L, R⁵, the C^{alpha} of B, X, R⁶ or S⁴, especially to R⁵, particularly a combination selected from Ga with DOTA, Ga with NODASA, Ga with NODAGA, Ga with DFO or Ga with NOTA.

13. A pharmaceutical composition comprising a compound as defined in any of claims 1 to 12 and at least one pharmaceutically acceptable excipient.

14. A compound as defined in any of claims 1 to 12 or a composition as defined in claim 13
i) for use as a medicament; or
ii) for use in a method for the prevention or treatment of a CXCR4 receptor-related disease or disorder; or
iii) for use in a diagnostic method practiced on the human or animal body for the diagnosis of a CXCR4 receptor-related disease or disorder; or
iv) for use in a method of imaging CXCR4 receptors, in particular of medical imaging, especially of diagnostic imaging, said method involving at least one step of treatment of the human or animal body by surgery or therapy.

15. Use of a compound as defined in any of claims 1 to 12 or of a composition as defined in claim 13, wherein the compound comprises a detectable label, for the imaging of CXCR4 receptors, in particular for medical imaging, especially for diagnostic imaging.

16. A method of imaging CXCR4 receptors, in particular of medical imaging, especially of diagnostic imaging,
the method comprising administering a compound as defined in any of claims 1 to 12 to a sample or a subject, wherein the compound comprises a detectable label.
